(19) Europäisches Patentamt
European Patent Office
Office européen des brevets

(11) EP 1 372 659 B1

(12) EUROPÄISCHE PATENTSCHRIFT

(45) Veröffentlichungstag und Bekanntmachung des Hinweises auf die Patenterteilung:
**28.07.2004 Patentblatt 2004/31**

(21) Anmeldenummer: **02742877.0**

(22) Anmeldetag: **06.04.2002**

(51) Int Cl.[7]: **A61K 31/506**, A61P 17/00, A61P 17/06, A61P 31/12, A61P 35/00, A61P 33/00, A61K 31/295

(86) Internationale Anmeldenummer:
**PCT/EP2002/003835**

(87) Internationale Veröffentlichungsnummer:
**WO 2002/080923 (17.10.2002 Gazette 2002/42)**

(54) **SUBSTANZEN ZUR THERAPIE VON ERKRANKUNGEN, DIE DURCH HOCHPROLIFERIERENDE ZELLEN VERURSACHT WERDEN**

SUBSTANCES FOR USE IN THERAPY OF DISEASES THAT ARE CAUSED BY HIGHLY PROLIFERATIVE CELLS

SUBSTANCES POUR TRAITER DES MALADIES PROVOQUEES PAR DES CELLULES A HAUTE PROLIFERATION

(84) Benannte Vertragsstaaten:
**AT BE CH CY DE DK ES FI FR GB GR IE IT LI LU MC NL PT SE TR**

(30) Priorität: **07.04.2001 DE 10117479**
**29.05.2001 EP 01113051**

(43) Veröffentlichungstag der Anmeldung:
**02.01.2004 Patentblatt 2004/01**

(73) Patentinhaber:
- **Schmalz, Hans-Günther**
  **50939 Köln (DE)**
- **Universitätsklinikum Charité Medizinische Fakultät der Humboldt-Universität zu Berlin**
  **13125 Berlin (DE)**

(72) Erfinder:
- **SCHMALZ, Hans-Günther**
  **50939 Köln (DE)**
- **PROKOP, Aram**
  **13437 Berlin (DE)**
- **WIEDER, Thomas**
  **72144 Dusslingen (DE)**
- **DANIEL, Peter**
  **13187 Berlin (DE)**

(74) Vertreter:
**Meyers, Hans-Wilhelm, Dr.Dipl.-Chem. et al**
**Patentanwälte**
**von Kreisler-Selting-Werner**
**Postfach 10 22 41**
**50462 Köln (DE)**

(56) Entgegenhaltungen:
- **HOSSAIN, N. ET AL: "Synthesis of 2'- and 3'-spiro-isoxazolidine derivatives of thymidine and their conversions to 2',3'-dideoxy-2',3'-didehydro-3'-C- substituted nucleosides by radical promoted fragmentation" TETRAHEDRON (1993), 49(44), 10133-56, 1993, XP001041896**
- **HARAGUCHI, KAZUHIRO ET AL: "Uracil and adenine nucleosides having a 2',3'-bromovinyl structure: highly versatile synthons for the synthesis of 2'-C- and 3'-C-branched 2',3'-unsaturated derivatives" TETRAHEDRON (1993), 49(7), 1371-90, XP001041725**
- **HARAGUCHI, KAZUHIRO ET AL: "Preparation and reactions of 2'- and 3'-vinyl bromides of uracil nucleosides: versatile synthons for anti-HIV agents" TETRAHEDRON LETT. (1991), 32(28), 3391-4, XP001041740**
- **DATABASE WPI Section Ch, Week 199217 Derwent Publications Ltd., London, GB; Class B03, AN 1992-136754 XP002185460 & JP 04 077485 A (YAMASA SHOYU KK), 11. März 1992 (1992-03-11)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 372 659 B1

## Beschreibung

Die vorliegende Erfindung betrifft Substanzen und Arzneimittel zur Behandlung von Erkrankungen, die durch hoch-proliferierende Zellen verursacht werden, sowie Verfahren zur Herstellung solcher Substanzen und Arzneimittel.

Die Entstehung von Tumoren oder anderer gutartiger hyperproliferativer Erkrankungen, wie z. B. der Schuppenflechte oder des Keloids, ist auf eine gestörte Balance zwischen der Gewebeneubildung und dem regulierten Absterben von Zellen aus dem Gewebeverband zurückzuführen. In der klinischen Praxis wird durch den Einsatz zytotoxischer Behandlungsmethoden, wie der Chemotherapie, der Strahlentherapie und der Hyperthermie, versucht, diese gestörte Balance wieder ins Gleichgewicht zu bringen und die überschüssigen Tumorzellen gleichzeitig abzutöten. Es ist allgemein anerkannt, dass die meisten derzeit in der klinischen Praxis eingesetzten Chemotherapeutika ihre Wirkung durch Einleitung der Apoptose (programmierter Zelltod) entfalten (Hannun, 1997). Allerdings entwickelt ein Teil der an malignen Tumoren erkrankten Patienten frühzeitig eine Chemotherapie- und Strahlenresistenz oder ist primär therapierefraktär (Hickman, 1996). Weiterhin ist bekannt, dass Primärtumor und Metastasen auf zytotoxische Therapien oft ganz different ansprechen. Aufgrund neuerer Untersuchungen ist wahrscheinlich, dass die Ursache für Resistenzen in unterschiedlichen Störungen der Apoptosesignalkaskade liegt (Raisova et al., 2000).

Besonders die Therapie eines Rezidivs der kindlichen akuten lymphoblastischen Leukämie (ALL), der häufigsten malignen Erkrankung im Kindesalter, ist noch immer nicht befriedigend gelöst. So versterben z. B. trotz aggressiver Therapie ca. 70% der erkrankten Kinder im Rezidiv der ALL. Ähnliches trifft auch auf andere teilweise schwer therapierbare Tumorerkrankungen, wie das Schilddrüsenkarzinom, das sehr häufige Mammakarzinom, das schwer zugängliche Medulloblastom oder auf Gliome zu. Eine gutartige Hauterkrankung, die auch mit solchen Therapien behandelt wird, ist die Psoriasis (Schuppenflechte). Sie stellt eine der häufigsten Erkrankungen der Haut dar, an der zwei bis vier Prozent der Menschen leiden. Auch die Therapie dieser Erkrankung ist noch stark verbesserungsbedürftig.

Es besteht also ein starkes Bedürfnis, Substanzen und Arzneimittel zu entwickeln, mit denen die Heilungserfolge und die Überlebenschancen von Patienten verbessert werden, die an den oben aufgeführten Erkrankungen leiden. Insbesondere bei Tumorerkrankungen und Leukämien ist es von Bedeutung, neue Arzneimittel zu bereitzustellen, die hochselektiv gegen unnatürlich proliferierende Zellen wirken und dabei gesunde Zellen möglichst wenig angreifen. Darüber hinaus ist es von besonderer Wichtigkeit, Therapiestoffe gegen solche Tumoren bereitzustellen, die sich gegenüber bereits bekannten Substanzen als resistent erweisen.

Überraschenderweise wird diese Aufgabe durch Arzneimittel der folgenden Strukturformeln (**1a** oder **1b**) gelöst:

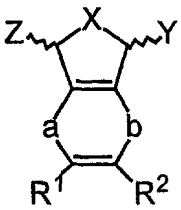

**1a**

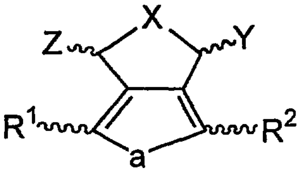

**1b**

mit

X = O, S, $CH_2$, NR, keine Bindung,

mit der Maßgabe, dass wenn X = O ist, die Verbindung der Formel 1a mit einem Metall-Ligandenkomplex assoziiert

ist gemäß Formel 1'a;

Y = Adenin, Cytosin, Guanin, Uracil, Thymin, Bromuracil, Purine oder Pyrimidine sowie deren Derivate, Nucleobasen, Heterozyklen, Aminoalkyl, Aminoaryl oder sonstige zur Ausbildung von H-Brücken befähigte Reste,

Z = Alkyl, Fluoralkyl, Aryl, Fluoraryl, $-(CH_2)_nOR'$, mit R' = H, $SiR_3$, Alkyl, Fluoralkyl, Aryl, Fluoraryl, Acyl und n = 0 bis 5, wobei vorzugsweise n = 1 ist;

a = keine Bindung, Einfachbindung oder $CH_2$, bei Formel **1b** auch $CH_2CH_2$ ;

b = keine Bindung, Einfachbindung oder $CH_2$; wobei a und b so ausgewählt werden, dass die a und b umfassende Struktureinheit ein 1,3-Dien beinhaltet,

$R^1$ = -H, F, Alkyl, Fluoralkyl, Aryl, Fluoraryl, OR, $-CO_2R$, $-SO_2OR$, $-CONR_2$,

$R^2$ = -H, F, Alkyl, Fluoralkyl, Aryl, Fluoraryl, OR, $-CO_2R$, $-CONR_2$, $-SO_2OR$,

R= H, verzweigte und geradkettige Alkyl, insbesondere Methyl, Ethyl, Propyl, Thexyl, tert-Butyl

und/oder deren Salze.

Wenn a, b oder X als "keine Bindung" angegeben wird, bedeutet dies, dass die jeweils zwei Kohlenstoffatome, die in der Strukturformel mit a, b oder X verbunden sind, nicht verknüpft sind. Da die a und b umfassende Struktureinheit ein 1,3-Dien beinhalten soll, können jedoch nicht sowohl a als auch b "keine Bindung" sein.

Die a und b enthaltende Struktureinheit kann zum Beispiel als Cyclobutadienring, Cyclopentadienring oder als offenes 1,3-Butadien vorliegen, wobei im letzteren Fall $R^1$ oder $R^2$ mit dem endständigen Kohlenstoffatom verbunden sein kann.

Bei der $SiR_3$-Gruppe sind die R vorzugsweise Methyl-, Ethyl-, Propyl-, tert-Butyl oder Thexylreste.

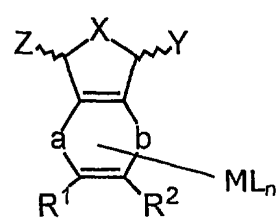

**1´a**

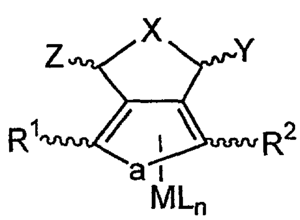

**1´b**

Weiterhin sind Arzneimittel der Strukturformeln **1'a** und **1'b** geeignet, bei denen das 1,3-Dien an ein Metallfragment $ML_n$ gebunden ist, wobei

n eine ganze Zahl von 2 bis 4 ist

M = Mn, Fe, Co, Ru und

L = CO, CN-R, CN, $CR_2$, COR, Hal, Cyclopentadienyl (Cp) oder ein substituiertes Cp-Derivat ist, und R ausgewählt wird wie für Strukturformel **1a** und **1b** angegeben.
und/oder deren Salze.

An ein M können dabei gleiche oder verschiedene L gebunden sein.

Das Metallfragment ist vorzugsweise ein $Fe(CO)_3$ - Komplex.

Besonders geeignet sind Arzneimittel mit einer Strukturformel **2, 3, 4a, 4b** oder **4c**:

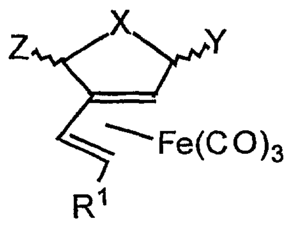

**2**

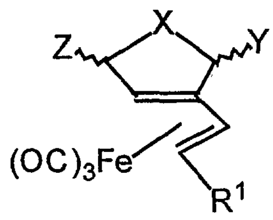

**3**

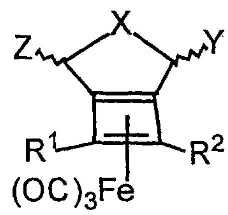

**4a**

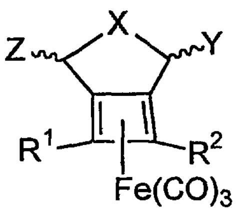

**4b**

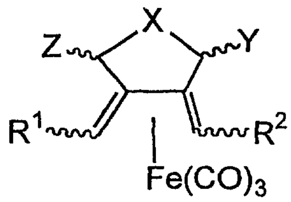

**4c**

wobei die $Fe(CO)_3$-Einheit $\eta^4$-gebunden,

X = O, $CH_2$ oder keine Bindung ist und
Y, Z, $R^1$ und $R^2$ wie für Strukturformel **1a** und **1b** ausgewählt werden. Bei den Strukturformeln **4a** und **4b** handelt es sich um Resonanzformeln. **4c** ist aus **4b** durch eine formale Ringöffnung erhältlich.

Insbesondere geeignet sind Arzneimittel mit einer Strukturformel **5**:

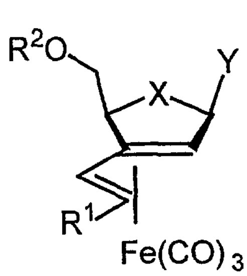

**5**

mit

X = O, $CH_2$,
Y = Adenin, Cytosin, Guanin, Uracil, Thymin, Bromuracil, Purine oder Pyrimidine sowie deren Derivate, Nucleo-

basen, Heterozyklen,
$R^1$ = -H, -$CO_2R$,
und
$R^2$ = H, $SiR_3$, Alkyl, Fluoralkyl, Aryl, Fluoraryl, Acyl.

Gegenstand der Erfindung sind auch Substanzen und Verfahren zu deren Herstellung der Strukturformel **5,** bei denen

$R^2$ = $SiR_3$, X = O,

Y und $R^1$ wie für Strukturformel **5** angegeben ausgewählt werden und R wie bei Strukturformel **1a** und **1b** angegeben ausgewählt ist. Diese Verbindungen und Verfahren zu deren Herstellung sind jedoch nicht Gegenstand der Erfindung, wenn Y Bromuracil, Uracil oder Methyluracil ist und $R^2$ gleichzeitig Thexyl$(CH_3)_2$Si ist. Gegenstand der Erfindung sind in diesen Fällen die entsprechenden Arzneimittel.

Gegenstand der Erfindung sind insbesondere die Verbindungen der folgenden Strukturformeln:

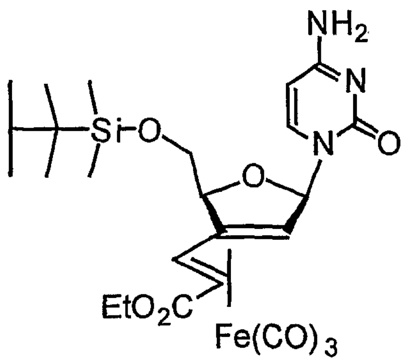

**6**

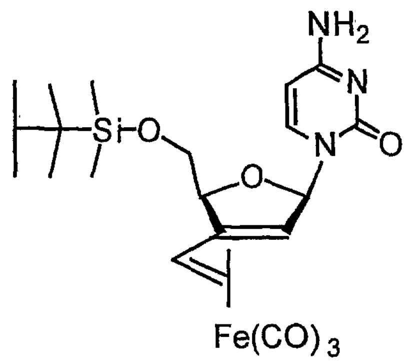

**7**

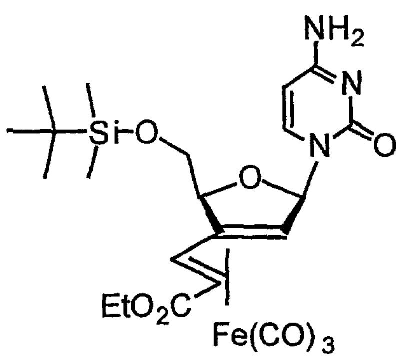

**8**

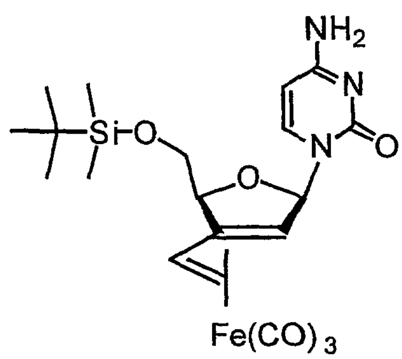

**9**

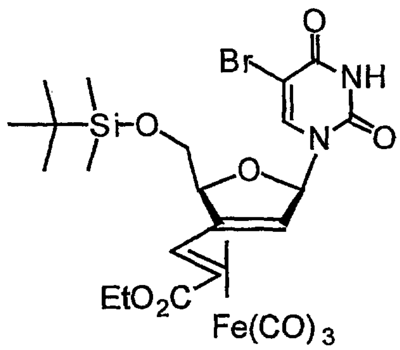

10

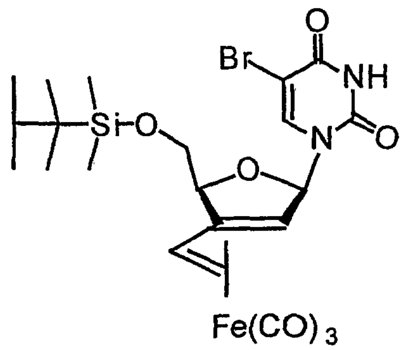

11

Gegenstand der Erfindung ist auch eine Verbindung der Strukturformel **35**:

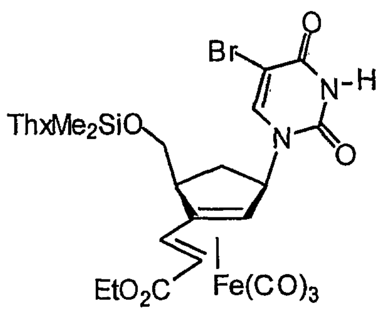

**35**

Gegenstand der Erfindung sind die Racemate der erfindungsgemäßen Verbindungen und die Enantiomere.

Die erfindungsgemäßen Verbindungen und die Verbindungen der Strukturformel **1a**

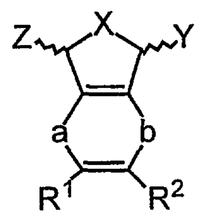

**1a**

wobei

X = O, S, $CH_2$, NR oder keine Bindung ist und

Y, Z, a, b, $R^1$ und $R^2$ wie in Anspruch 1 ausgewählt werden,

werden erfindungsgemäß verwendet zur Herstellung eines Arzneimittels zur Behandlung maligner Erkrankungen des Knochenmarks oder anderer blutbildender Organe, solider Tumoren, epithelialer Tumoren, gutartiger oder semimaligner schnell proliferierender Tumore oder Hauterkrankungen, insbesondere Psoriasis vulgaris, Keloide und Basaliome, Lymphome, insbesonder Hodgkin- und Non-Hodgkin-Lymphome, entzündlicher, chronisch entzündlicher, bakterieller und autoimmuner Erkrankungen, sowie zur antibakteriellen, antimykotischen, anti-Protozoen, anti-Plasmodien anti-helminthischen oder immunsuppressiven Therapie.

Die erfindungsgemäßen Arzneimittel und Verbindungen eignen sich unerwarteterweise zur Therapie von pathologisch schnell proliferierendem Gewebe, besonders von Knochenmark, aber auch von soliden Tumoren, wie epithelialen Tumoren oder insbesondere Hirntumoren. Weiterhin erstreckt sich die Anwendbarkeit der beschriebenen Substanzen auch auf die Behandlung gutartiger, hyperproliferativer Erkrankungen der Haut, wie z. B. die Psoriasis oder das Keloid. Die Arzneimittel und Substanzen der Erfindung zeichnen sich dadurch aus, dass sie in besonderem Maße geeignet sind, selektiv das Wachstum von hochproliferierenden Zellen zu inhibieren. Dadurch leiten sie die Apoptose von hochproliferierenden Zellen ein und bewirken so deren Zerstörung, wobei gesunde Zellen sehr wenig beeinträchtigt werden.

Die Substanzen sind im besonderen Maße membrangängig, was zu einer hohen intrazellulären Wirkstoffkonzentration führt. Die hohe Wirksamkeit wird daher wahrscheinlich durch die ausgeprägte Lipophilie der Substanzen erzielt. Die Substanzen unterscheiden sich grundlegend von bereits bekannten zur Therapie verwendeten Nukleosidanaloga, wie Cytarabin und Fludarabin-5'dihydrogenphosphat. Sie sind in der Lage, vorhandene Zytostatikaresistenzen zu brechen.

Die Arzneimittel und Verbindungen der Erfindung sind insbesondere geeignet zur Behandlung von Tumorerkrankungen und Leukämie. Sie leiten den apoptotischen Zelltod nicht nur in aus Tumorzellen entstandenen permanenten Zellinien (BJAB-Zellen), sondern auch in primären Zellen von Patienten mit einer akuten lymphoblastischen Leukämie (ALL) ein. Dadurch können die erfindungsgemäßen Substanzen gegen Tumorerkrankungen des Knochenmarks, aber auch gegen Tumore einer anderen Provenienz, wie z. B. epitheliale Tumore, Sarkome oder maligne Erkrankungen der Haut usw. eingesetzt werden. Besonders soll darauf hingewiesen werden, dass mit den entwickelten Substanzen aufgrund ihrer Lipophilie eine Überschreitung der Blut-Hirnschranke möglich ist, und diese daher auch für maligne Hirntumore, wie z. B. das Medulloblastom oder Gliome, eingesetzt werden können. Insgesamt können die erfindungsgemäßen Substanzen zur Behandlung maligner Erkrankungen des Knochenmarks oder anderer blutbildender Organe, solider Tumoren, epithelialer Tumoren, gutartiger oder semimaligner schnell proliferierender Hauterkrankungen, insbesondere Psoriasis vulgaris, Keloide und Basaliome, sowie entzündlicher und chronisch entzündlicher Erkrankungen verwendet werden. Sie sind auch geeignet zur antiviralen, antibakteriellen, antimykotischen, anti-Protozoen, anti-helminthischen oder immunsuppressiven Therapie.

In der Fig. 1A ist gezeigt, dass die Substanzen N76 (Verbindung der Strukturformel **5** mit $R^1 = CO_2Et$, $R^2$ = Thexyldimethylsilyl, X = O, Y = Bromuracil) und N69 (Verbindung der Strukturformel **6**) bei einer Konzentration im Zellkulturmedium von 25 μmol/l in 60 - 70 % der BJAB-Zellen Apoptose auslösen. Die Messung der Apoptose basiert auf einer Methode, die die für die Apoptose typische Fragmentierung der DNA auf Einzelzellniveau nachweist, die diese Zelltodform von der Nekrose unterscheidet. Dazu wurden BJAB-Zellen 24 h mit unterschiedlichen Konzentrationen N76 oder N69 behandelt. Kontrollen enthielten entsprechende Mengen des Lösungsvermittlers Ethanol. Nach der Behandlung wurde die Fragmentierung der DNA durch Färbung mit Propidiumiodid und anschließender Quantifizierung mittels Durchflußzytometrie, wie von Eßmann et al. (2000) beschrieben, gemessen. Die Werte sind gegeben als % apoptotische Zellen der Gesamtpopulation ± SD (n=3).

Die Substanzen wirken jedoch nicht nur auf permanente Zellinien, sondern auch auf Lymphoblasten, die direkt von Kindern mit einer ALL gewonnen wurden, proapoptotisch (Fig. 1B). Im Unterschied zum bereits bekannten und sehr verbreitet eingesetzten Chemotherapeutikum Epirubicin (abgekürzt mit E) leiten die Substanzen N76 und N69 auch in therapieresistenten Patientenzellen *in vitro* die Apoptose ein (Fig. 1B ). In diesem Versuch wurden Lymphoblasten von Kindern mit einer akuten lymphoblastischen Leukämie (ALL) isoliert und nach Verdünnung mit Zellkulturmedium für 36 h mit 25 μM N76, 25 μM N69 oder als Positivkontrolle mit 9 μM Epirubicin (E) behandelt. Kontrollen enthielten entsprechende Mengen des Lösungsvermittlers Ethanol. Nach der Behandlung wurde die Fragmentierung der DNA durch Färbung mit Propidiumiodid und anschließender Quantifizierung mittels Durchflußzytometrie, wie von Eßmann et al. (2000) beschrieben, gemessen. Die Werte sind gegeben als % apoptotische Zellen der Gesamtpopulation ± SD (n=3).

In der Abfolge der Apoptose kommt es zur Aktivierung einer Familie von Cysteinproteasen, den sogenannten Caspasen, die die Zelle während des ablaufenden Todesprogramms von innen heraus auflösen (Cohen, 1997). Um die Spezifität der erfindungsgemäßen Substanzen näher zu untersuchen, wurde in einem Western Blot die Prozessierung und Aktivierung der Caspase-3 nachgewiesen (Figur 2). Dazu wurden BJAB-Zellen 24 h mit unterschiedlichen Konzentrationen von N76 behandelt. Kontrollen enthielten entsprechende Mengen des Lösungsvermittlers Ethanol (Ke). Nach der Behandlung wurde die Prozessierung der Procaspase-3 mittels spezifischer Immundetektion im Western Blot, wie von Eßmann et al. (2000) beschrieben, bestimmt. Die Positionen der Procaspase-3 und der prozessierten Untereinheit im SDS-Polyacrylamidgel sind durch Querstriche am linken Rand von Fig.2 gekennzeichnet. Die Zugabe von 25 oder 50 μmol/l der Substanz N76 zum Medium von BJAB-Zellen löst in diesen Zellen eine Prozessierung der Procaspase-3 aus. Zu sehen ist der spezifische, immunchemische Nachweis der aktiven Untereinheit der Caspase-3 in behandelten Zellen im Unterschied zu den entsprechenden Kontrollzellen. Das Ergebnis macht deutlich, dass die erfindungsgemäßen Substanzen spezifisch eine apoptotische Kaskade induzieren.

Die Verwendbarkeit erfindungsgemäßer Substanzen für die Therapie verschiedener bösartiger Erkrankungen des blutbildenden Systems wurde an Zellen von Patienten mit unterschiedlichen Erkrankungen erprobt. Dazu wurden Zellen von Patienten mit verschiedenen Leukämien isoliert und nach Verdünnung in Zellkulturmedium für 36 h mit 25 μM N69 behandelt. Kontrollen enthielten entsprechende Mengen des Lösungsvermittlers Ethanol. Nach der Behandlung wurde die Fragmentierung der DNA durch Färbung mit Propidiumiodid und anschließender Quantifizierung mittels Durchflußzytometrie, wie von Eßmann et al. (2000) beschrieben, gemessen. Das Ergebnis ist Figur 3 zu entnehmen. Die gemessenen Werte sind gegeben als % apoptotische Zellen der Gesamtpopulation und stellen den Mittelwert aus zwei unabhängigen Experimenten dar. Figur 3 zeigt, dass 25 μmol/l N69 sowohl in Zellen von Patienten mit einem Rezidiv der akuten lymphoblastischen Leukämie (ALL-Rez) als auch in Zellen von Patienten mit einer Erstmanifestation dieser Erkrankung (ALL), in Zellen von Patienten mit einer akuten myeloblastischen Leukämie (AML) und in Zellen von Patienten mit einer chronischen myeloblastischen Leukämie (CML) den apoptotischen Zelltod auslösen.

Damit werden mit N76 und N69 zwei Substanzen der allgemeinen Strukturformel 2 als wirksame Mittel gegen bestimmte Tumorzellen, besonders die der kindlichen ALL, aber auch gegen andere maligne Erkrankungen unterschiedlicher Herkunft, bereitgestellt. Zusammenfassend ist in Fig. 4 gezeigt, dass insbesondere die Substanz N69 *in vitro* eine signifikant bessere proapoptotische Wirkung gegenüber primären ALL-Zellen aufweist als die etablierten Zytostatika Doxorubicin, Cytarabin und Fludarabin-5'dihydrogenphosphat. In diesen Versuchen wurden die Zellen von 11 ALL-Patienten isoliert und nach Verdünnung in Zellkulturmedium für 36 h mit 25 μM N69, 25 μM N76 oder entsprechenden Mengen Cytarabin (AraC), Fludarabin oder Doxorubicin behandelt. Kontrollen enthielten entsprechende Mengen des Lösungsvermittlers Ethanol. Nach der Behandlung wurde die Fragmentierung der DNA durch Färbung mit Propidiumiodid und anschließender Quantifizierung mittels Durchflußzytometrie, wie von Eßmann et al. (2000) beschrieben, gemessen. P-Werte wurden mittels eines gepaarten t-Tests berechnet und sind direkt in Fig. 4 für die einzelnen Vergleichspaare angegeben. P-Werte $< 0.05$ gelten als statistisch signifikant.

Um die Rolle der verschiedenen Komponenten der neu beschriebenen Substanzen näher zu charakterisieren, wurden die eisenkomplexierte Verbindung N69 und die Verbindung, die durch Abspalten der $Fe(CO)_3$-Gruppe aus N69 erhältlich ist, hergestellt und in einem kombinierten Proliferationsund Zelltodassay eingesetzt. Dazu wurden BJAB-Zellen 24 h und 72 h mit unterschiedlichen Konzentrationen von N69 oder der entsprechenden Verbindung ohne Eisenkomplex behandelt. Kontrollen enthielten entsprechende Mengen des Lösungsvermittlers Ethanol. Nach der Behandlung wurde die Zellzahl in der Neubauer-Zählkammer und die Anzahl toter Zellen durch Trypan-Blaufärbung, wie von Wieder et al. (1995) beschrieben, bestimmt. In Fig. 5A ist die Gesamtzahl lebender Zellen mal $10^{-5} \pm$ SD (n=3) nach Inkubation mit Kontrollmedium (offene Kreise), 10 μM N69 (geschlossene Kreise) oder 25 μM N69 (offene Dreiecke) nach 24 h und 72 h gezeigt. In Fig. 5B ist der zu diesen Zeitpunkten bei der entsprechenden Konzentration bestimmte Anteil toter Zellen in % ± SD (n=3) gegeben. Die linke Spalte zeigt das Ergebnis nach Behandlung mit der dekomplexierten Substanz, die rechte Spalte das Ergebnis für das komplexierte N69. Wie unschwer zu erkennen ist, spielt das komplexierte Eisen eine wichtige Rolle für den Zelltod-einleitenden Effekt der Substanzen. Dabei verschiebt das komplexierte Eisen die Dosis/Wirkungskurve des Zelltod-induzierenden Effekts von N69 zu niedrigeren Konzentrationen. Während 25 μM der eisenfreien Verbindung nach 72 h nur in ca. 20 % der Zellen Zelltod induziert, sterben in Anwesenheit von 25 μM eisenhaltigem N69 bereits nach 24 h ca. 90 % der behandelten Zellen (Figur 5B). Hingegen ist für die antiproliferativen Eigenschaften von N69 eher der Nukleosidanteil verantwortlich, da beide getesteten Substanzen bei 10 μM die Proliferation signifikant erniedrigen und bei 25 μM vollständig blockieren (siehe Figur 5A). Das heißt, es werden in der vorliegenden Schrift neue Substanzklassen beschrieben, die zwei für die Wirkung essentielle Strukturmerkmale besitzen. Es soll jedoch betont werden, dass auch solche Substanzen der Erfindung, die nicht als Eisen-Komplex appliziert werden, eine hohe therapeutische Wirksamkeit aufweisen.

In einem weiteren Ausführungsbeispiel wird eine proapoptotische Wirkung für das neu synthetisierte, carbozyklische Nukleosidanalogon JV-206-1 (Strukturformel **35**) nachweisen. In Fig. 6A ist gezeigt, dass die 48 stündige Inkubation von BJAB-Zellen mit JV-206-1 (**35**) den Zelltod auslöst. Weitere Untersuchungen ergaben, dass die BJAB Zellen durch JV-206-1 in den mitochondrialen Apoptosesignalweg getrieben werden. Dies wurde mittels Färbung der Zellen mit dem Mitochondrien-spezifischen Farbstoff JC-1, wie von Wieder *et al*. (2001) beschrieben, nachgewiesen. Die Inkubation der Zellen mit JV-206-1 führte hierbei zu einer konzentrationsabhängigen Erhöhung des Anteils von Zellen mit einem erniedrigten mitochondrialen Membranpotential ($\Delta\Psi_m$), was auf eine starke Aktivierung der Mitochondrien während des apoptotischen Prozesses hinweist (Fig. 6B). Als spezifisches Apoptosemerkmal wurde außerdem die Fragmentierung der DNA, wie von Eßmann *et al*. (2000) beschrieben, gemessen. Dabei stellte sich heraus, daß der überwiegende Teil der Zellen bei JV-206-1 Konzentrationen von 20 μM durch Apoptose stirbt (Fig. 6C). Der Anteil der apoptotischen Zellen lag in diesen Versuchen je nach Konzentration und Inkubationszeit zwischen 20% und 48% der Gesamtpopulation.

Die erfindungsgemäßen Arzneimittel können topisch oder intravenös appliziert werden. Bei intravenöser Applikation werden die Substanzen im Konzentrationsbereich zwischen 0,1 bis 100 μg/ml, bezogen auf das Blutvolumen des Patienten, verabreicht. Die Substanzen werden in einer Konzentration von 0,1 bis 5 Gew.-%, bezogen auf das fertige Präparat, in die erkrankte Haut eingerieben.

Die vorliegende Erfindung betrifft auch Verfahren zur Synthese der erfindungsgemäßen Verbindungen, die in den Schemata 1 bis 5 zusammengefaßt sind. In einer Ausführungsform der Erfindung umfaßt das Verfahren die folgenden Schritte:

Schritt (a): Überführung eines Glycosides (bevorzugt ein Glucosederivat) **12** durch Umsetzung mit einem Trialkylsilyl-Reagenz in eine geschützte Verbindung **13**;

Schritt (b): Umwandlung von **13** in ein oder mehrere Epoxide vom Typ **14** unter Mitsunobu-Bedingungen (Azodicarbonsäurediester/Trialkylphosphan);

Schritt (c): Ringverengung von **14** (auch als Gemisch) zu Aldehyden vom Typ **15** durch Erhitzen mit LiBr in Toluol in Gegenwart von Donor-Kosolventien wie Tetraalkylharnstoffe.

Schritt (d): Olefinierung des Aldehydes **15** zu Dienen vom Typ **16**, vorzugsweise durch Wittig-Reaktion;

Schritt (e): Komplexierung von **16** zu **17** unter Verwendung von $Fe_2(CO)_9$ oder anderer zum Transfer der $Fe(CO)_3$-Gruppe befähigten Reagenzien;

Schritt (f): Einführung einer Nucleobase oder einer Gruppe Y (nach Formel **5**) durch diastereoselektive, eisenunterstützte nucleophile Substitution, bevorzugt unter Verwendung silylierter Nucleobasen in Gegenwart einer Lewissäure (Heßler 1994).

Der allgemeine Syntheseweg (Schema 1) wurde im Rahmen der Dissertation von Erik Heßler (Universität Frankfurt am Main 1993) und der Diplomarbeit von Andre Majdalani (Universität Frankfurt am Main 1994) entwickelt und damit publiziert. Die Schritte (a), (b) und (c) (Herstellung der Aldehyd-Zwischenstufen vom Typ **15**, mit $R_3Si$ = Thexyldimethylsilyl) basieren auf einem literaturbekannten Verfahren (Rehnberg 1990). Die Abtrennung von diastereomeren Nebenprodukten erfolgt durch Chromatographie und/oder Kristallisation auf der Stufe von **17, 18** oder **19**.

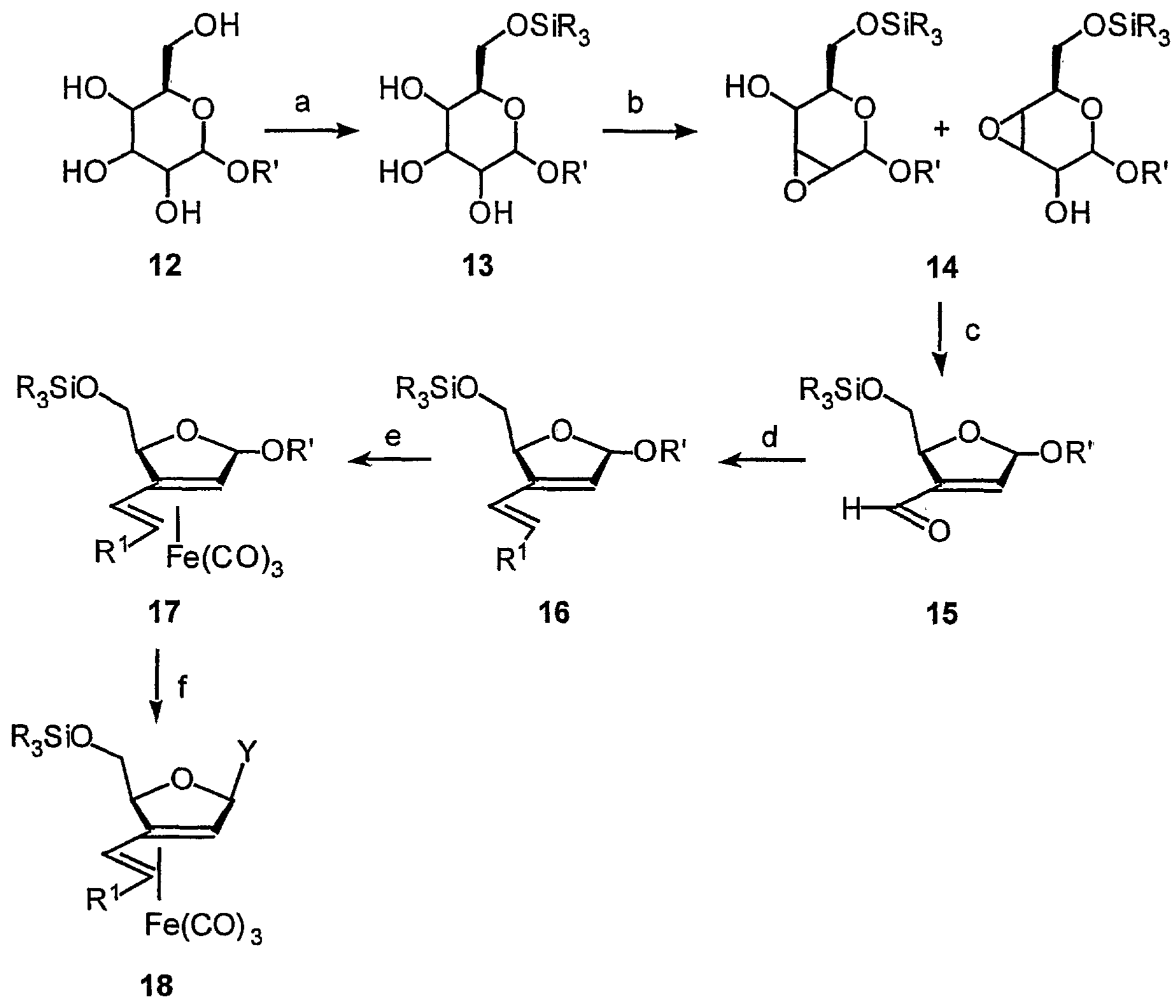


12 13 14 15 16 17 18

(Schema 1)

Zur Variation der Gruppe $R^2$ bei der Herstellung von Verbindungen des Typs **5** (Schema 2) wird

(g) die Silylgruppe von **17** mit Fluorid abgespalten;

(h) die freie OH-Funktion erneut verethert oder verestert und

(f) die Gruppe Y wiederum durch diastereoselektive, Eisen-unterstützte nucleophile Substitution, bevorzugt unter Verwendung silylierter Nucleobasen in Gegenwart einer Lewissäure, eingeführt.

17

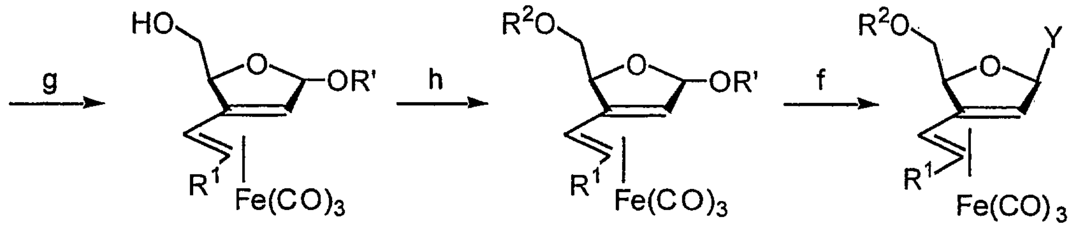


19 20 5

(Schema 2)

Beispielsweise kann der Schritt (f) die folgende Reaktion umfassen:

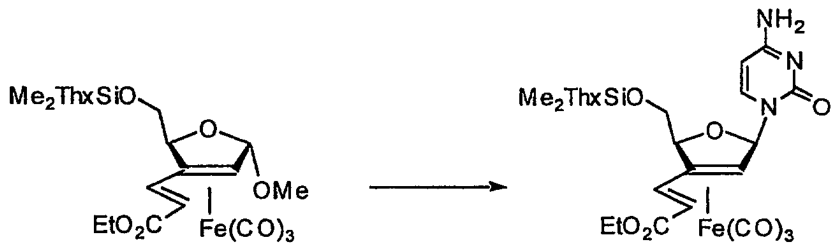

28 6

Die vorliegende Erfindung betrifft auch ein Verfahren zur Synthese von Substanzen vom Typ **44** (Typ **2** mit X = $CH_2$, z.B. Verbindung **35**), die als Arzneimittel verwendet werden (Schema 3). Es umfaßt die folgenden Schritte:

Schritte (a, b, c): Überführung von Propargylalkohol **36** in das Acetal **37** durch C-Silylierung, Oxidation des Alkohols zum Aldehyd und säurekatalysierte Acetalisierung mit Allylalkohol;
Schritt (d): Cylisierung von **37** durch eine Pauson-Khand- oder Pauson-Khandartige Reaktion;
Schritt (e): Diastereoselective Reduktion von **38**, vorzugsweise mit Natriumborhydrid in Gegenwart von Cer(III) chlorid;
Schritte (f, g): Desilylierung und Acetylierung zu **40**;
Schritt (h): Diastereoselektive Pd-katalysierte Einführung einer Nucleobase oder einer anderen (nucleophilen) Gruppe Y (nach Formel **5**);
Schritt (i, j): säurekatalysierte Acetalhydrolyse zum Hydroxy-Aldehyd und nachfolgende Silylierung, Veretherung oder Veresterung der OH-Funktion (variable Einführung von $R^2$);
Schritt (k): Olefinierung der Aldehyde **42** zu Dienen vom Typ **43,** vorzugsweise durch Wittig-Reaktion (variable Festlegung von $R^1$);
Schritt (l): Diastereoselektive Komplexierung von **43** zu **44** unter Verwendung von $Fe_2(CO)_9$ oder anderer zum Transfer der $Fe(CO)_3$-Gruppe befähigten Reagenzien;

Der allgemeine Syntheseweg bis zu den Zwischenstufen vom Typ **42** (Schema 3) wurde bereits publiziert (J. Velcicky, J. Lex, H.-G. Schmalz, *Org. Lett.* **2002**, *4*, 565-568). Die Schritte (a) bis (d) (Herstellung des racemischen Pauson-Khand-Produktes **38** wurden in Analogie zu einem Literaturverfahren (N. Jeong, B.Y. Lee, S.M. Lee, Y.K. Chung, S.-G. Lee, *Tetrahedron Lett.* **1993**, *34*, 4023) durchgeführt.

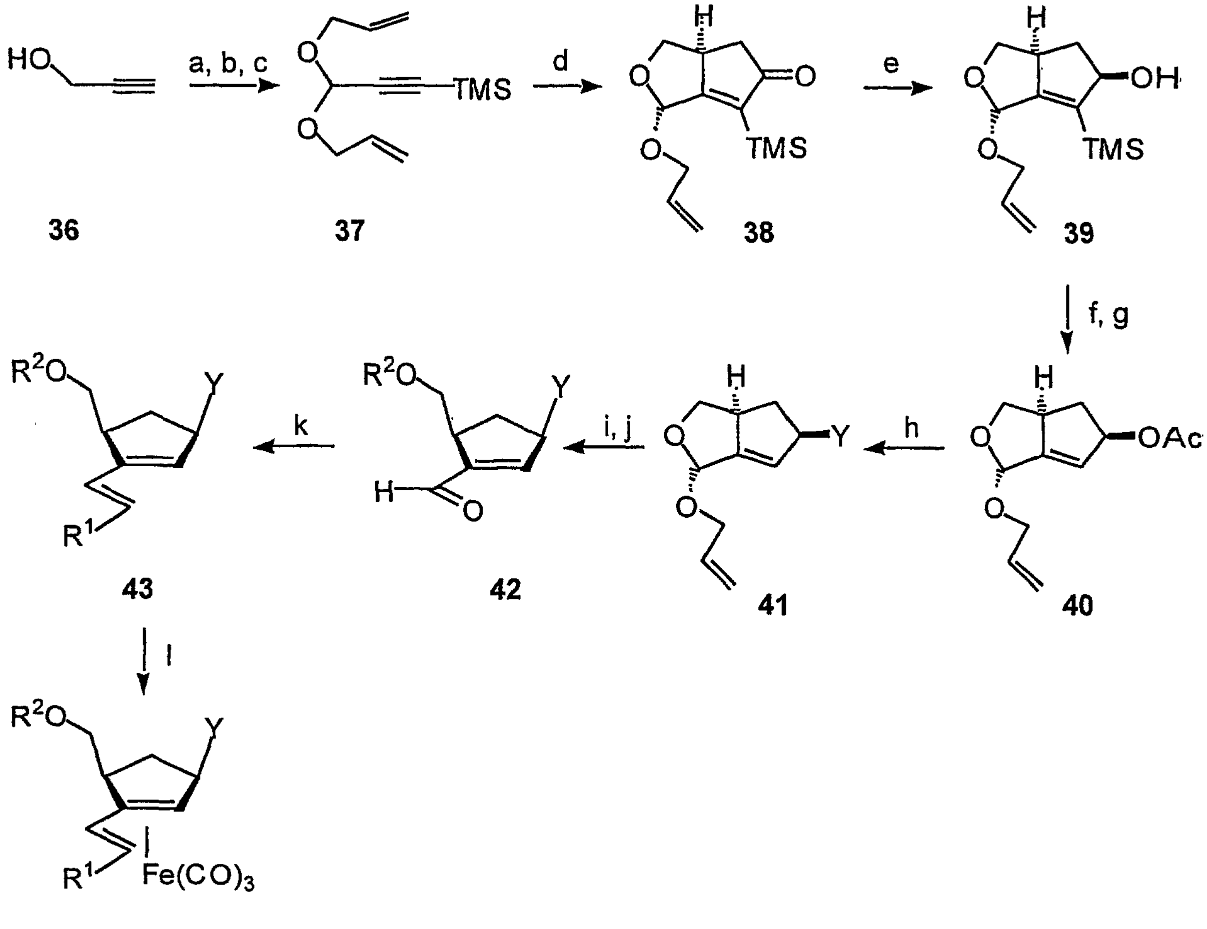


36 37 38 39 40 41 42 43 44

(Schema 3)

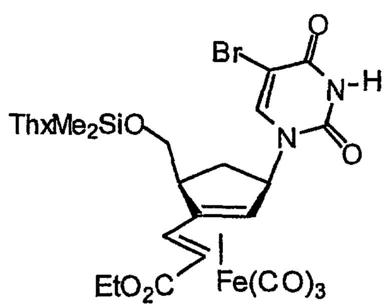

35

Zur Kontrolle der absoluten Konfiguration des Pauson-Khand-Produktes **38** kann der chirogene Schritt (d) entweder enantioselektiv durchgeführt (in Gegenwart chiraler Rhodium oder Iridium-Komplexe) werden oder das Produkt durch kinetische Racematspaltung (z.B. wie in Schema 4 dargestellt durch Oxazaborolidin-katalysierte Boranreduktion) aufgetrennt werden.

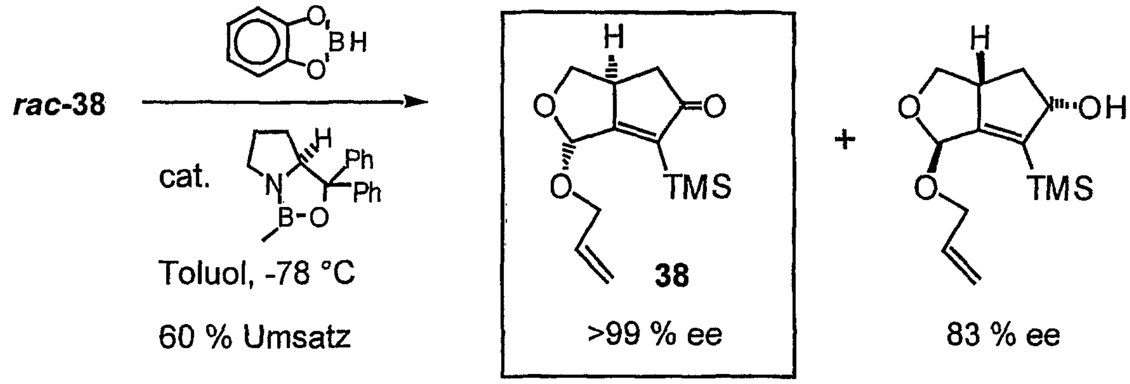


38

34 % 49 %

(Schema 4)

Die vorliegende Erfindung betrifft auch ein Verfahren zur Synthese von Substanzen vom Typ **52** (Typ **3** mit X = O, Z = $CH_2OR^2$, z.B. Verbindung **53**), die als Arzneimittel verwendet werden (Schema 5). Es umfaßt die folgenden Schritte:

Schritte (a, b): Überführung von Ribonolacton **45** in das tritylgeschütze Enoltriflat **46** durch Tritylierung der primären OH-Funktion und Umsetzung mit Trifluormethansulfonsäureanhydrid in Pyridin;
Schritt (c): Pd-katalysierte Kupplung von **46** mit einem Vinylstannan oder Vinylboran zu **47;**
Schritt (d): Diastereoselektive Komplexierung von **47** zu **48** unter Verwendung von $Fe_2(CO)_9$ oder anderer zum Transfer der $Fe(CO)_3$-Gruppe befähigten Reagenzien;
Schritt (e): Reduktion des Lactons zum Lactol **49** mit Diisobutylaluminiumhydrid;
Schritt (f): Säurekatalysierte Tritylspaltung und paralelle Substitution der OHgegen eine OMe-Gruppe (Methanol als Lösungsmittel);
Schritt (g): Silylierung, Veretherung oder Veresterung der OH-Funktion unter basischen Bedingungen (variable Einführung von $R^2$);
Schritt (h): Einführung einer Nucleobase oder einer Gruppe Y (nach Formel 5) durch diastereoselektive, eisen-unterstützte nucleophile Substitution, bevorzugt unter Verwendung silylierter Nucleobasen in Gegenwart einer Lewissäure.

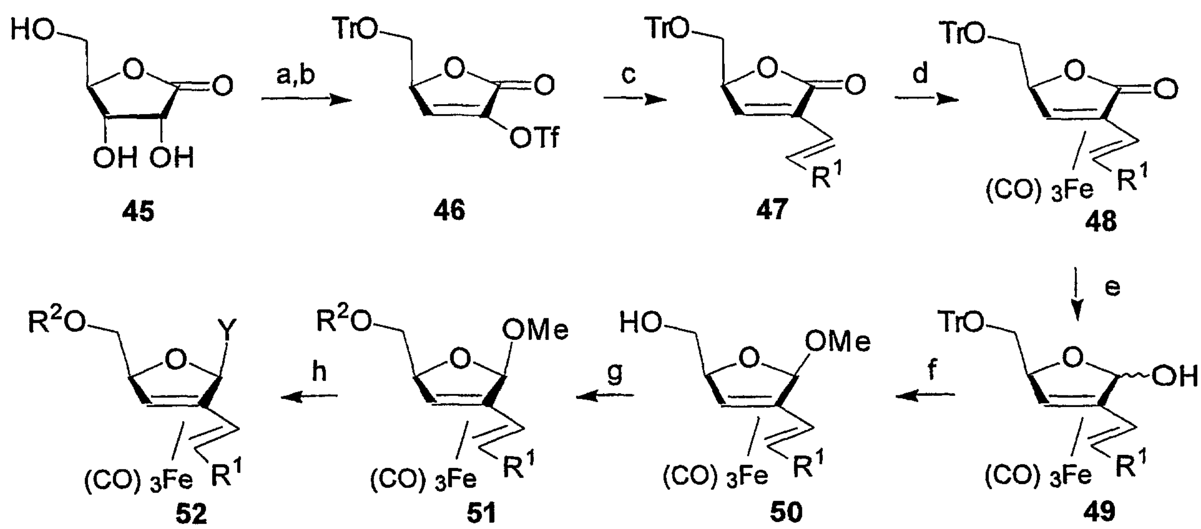


45 46 47 48

52 51 50 49

(Schema 5)

**Synthesevorschriften:**

**1. Herstellung von (+)-Methyl-6-O-(dimethyl-thexyl-silyl)-α-*D*-glucopyranosid (21)**

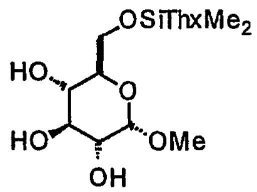

**21**

In einem 100 mL Schlenkkolben wurden in einer Argonatmosphäre 4.70 g (24.20 mmol) (+)-Methyl-α-*D*-glucopyranosid in 30 mL abs. Pyridin gelöst und anschließend im Eisbad auf 0°C gekühlt. Unter magnetischem Rühren und Argon-Gegenstrom injizierte man mit einer Spritze 5.0 mL (24.60 mmol) Thexyldimethylsilylchlorid, ließ noch 1 h bei 0°C rühren und enfernte dann das Kühlbad. Nach weiteren 22.5 h Rühren bei Raumtemp. wurde zweimal mit je 50 mL MeOH versetzt und jeweils im Vak. am Rotationsverdampfer wieder eingeengt. Anschließend überführte man das Reaktionsgemisch mit 200 mL Essigester in einen Scheidetrichter, wusch die organische Phase mit 2 mal 100 mL einer Lösung aus 190 mL $H_2O$, 20 mL konz. HCl und 15 g $NH_4Cl$, sowie mit je 100 mL ges. wäßr. $NaHCO_3$-Lösung und ges. wäßr. NaCl-Lösung. Die wäßrigen Phasen extrahierte man mit 2 x 100mL Essigester und trocknete die vereinigten organischen Phasen mit $Na_2SO_4$. Nach Filtration wurde im Vak. am Rotationsverdampfer eingeengt und der Rückstand durch Flash-Chromatographie an 190 g Kieselgel (Essigester) gereinigt. Man erhielt nach ausgiebigem Trocknen im Ölpumpenvakuum 6.27g (77 %) des analysenreinen Silylethers **21** als farblosen Feststoff. **Schmp.:** 88 - 89°C (Essigester), farbloser Feststoff. - **DC:** Essigester, $R_f$ = 0.34. - **FT-IR (KBr):** 3375 (s und br., O-H), 2958, 2902, 2876 (s,s,s, ges. C-H), 1466, 1379, 1251, 1152, 1118, 1081 (m,m,m,s,s,s), 1052 (s, C-O),831, 778 (s,m). - **$^1$H-NMR:** (400 MHz, $CD_3OD$) : δ = 0.12 (ψs, 6H, $(CH_3)_2Si$), 0.87 (ψs, 6H, $(CH_3)_2C$), 0.91 (d, J = 6.9 Hz, 6H, $(\underline{CH_3})_2CH$), 1.65 (sept, J = 6.9 Hz, 1H, $(CH_3)_2C\underline{H}$), 3.28 (ψt, J = 8.9 Hz, 1H, 4-H), 3.35 (dd, $^3J_{2,1}$ = 3.7 Hz, $^3J_{2,3}$ = 9.7 Hz, 1H, 2-H), 3.39 (s, 3H, OMe), 3.51 (ddd, $^3J_{5,6b}$ = 2.1 Hz, $^3J_{5,6a}$ = 5.5 Hz, $^3J_{5,4}$ = 9.9 Hz, 1H, 5-H), 3.60 (ψt, J = 9.5 Hz, 1H, 3-H),3.75 (dd, $^3J_{6a,5}$ = 5.5 Hz, $^2J_{6a,6b}$ = 11.2 Hz, 1H, 6-Ha), 3.89 (dd, $^3J_{6b,5}$ = 2.1Hz, $^2J_{6b,6a}$ = 11.2 Hz, 1H, 6-Hb), 4.63 (d, J = 3.7 Hz, 1H, 1-H). - **Analyse:** $C_{15}H_{32}O_6Si$ (336.50) ber.: C:53.54 %, H: 9.58 %; gef.: C: 53.28 %, H: 9.88 %.

**2. Herstellung von Epoxiden des Typs 22 und 23 als Gemisch**

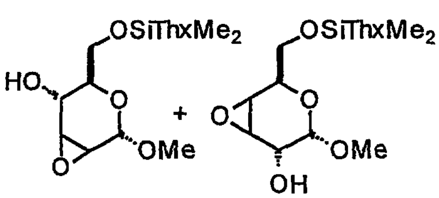

**22** **23**

In einem 500 mL Dreihalskolben, mit Rückflußkühler und aufgesetztem Hg-Bubbler wurden unter Schutzgas 10.35g (30.76 mmol) des Silylethers **21** und 9.70 g (36.98 mmol) Triphenylphosphan in 250 mL abs. Benzol vorgelegt und durch kräftiges magnetisches Rühren weitgehend gelöst. Zu dieser Lösung gab man (unter Argon-Gegenstrom) mit einer Spritze 5.3 mL (33.66 mmol) DEAD und ließ 1 h bei Raumtemp. reagieren (wobei die Lösung nach 30 min trüb wurde) bevor man für 4 h zum Sieden erhitzte. Nach Abkühlen auf Raumtemp. wurde die Reaktionslösung in einen Scheidetrichter überführt und jeweils mit 250 mL 1N HCl-Lösung und ges. wäßr. NaCl-Lösung gewaschen. Die wäßrigen Phasen extrahierte man mit 2 x 300 mL und 1 x 150 mL Ether und trocknete die vereinigten organischen Phasen mit $Na_2SO_4$. Nach Filtration wurde im Vak. am Rotationsverdampfer eingeengt, der Rückstand mit $CH_2Cl_2$ auf Kieselgel aufgezogen und anschließend (in zwei Portionen) durch Flash-Chromatographie an 190 g Kieselgel (*n*-Hexan

/ Essigester = 2+1, dann 5+3) gereinigt. Man erhielt nach Trocknen im Ölpumpenvakuum 8.71 g (89 %) eines farblosen Feststoffs, bei dem es sich laut $^1$H-NMR (wahrscheinlich) um ein Gemisch von vier isomeren Epoxiden handelte. Das Hauptprodukt konnte durch präparative HPLC (Bed. s.u.) als farbloser Feststoff in analytisch reiner Form isoliert werden.

**Rohgemisch: - DC** : *n*-Hexan / Essigester = 1 + 1, $R_f$= 0.34 und 0.41. - **analyt. HPLC** : *n*-Hexan / Essigester = 10 + 6.67; MN Nucleosil 50-10; 2 mL/min;Refraktom.; Retentionszeiten: 3.21 min, 3.43 min, 4.20 min, 5.13 min. - **präp. HPLC** : *n*-Hexan / Essigester = 10+6.67; 0.1 L/min; RI 20.-**Analyse** : $C_{15}H_{30}O_5Si$ (318.48); ber.: C:56.57 %, H: 9.49 %; gef.: C: 56.80 %, H: 9.39 %.

**Hauptprodukt: - DC :** *n*-Hexan / Essigester = 1 + 1, $R_f$ = 0.34. - **analyt. HPLC :** *n*-Hexan / Essigester = 10 + 6.67; MN Nucleosil 50-10; 2 mL/min; Refraktom.; Retentionszeit : 4.20 min. - **präp. HPLC** : *n*-Hexan / Essigester = 10+6.67; 0.1 L/min; RI 20. - **FT-IR (KBr) :** $\tilde{\nu}$ = 3441 (s und br., O-H), 2959, 2878 (s,m, ges. C-H), 1463, 1408 (m,m), 1257, 1149, 1130, 1105 (jeweils s), 1059 (s, C-O), 1001, 983, 906, 822, 780, 763 (jeweils s). - **$^1$H-NMR :** (250 MHz, $CDCl_3$) : $\delta$ = 0.13 ($\psi$s, 6H, $(CH_3)_2Si$), 0.85 ($\psi$s, 6H, $(CH_3)_2C$), 0.88 (d, J = 6.9 Hz, 6H, $(C\underline{H}_3)_2CH$), 1.62 (sept, J = 6.9 Hz, 1H, $(CH_3)_2C\underline{H}$), 2.52 (breites s, 1H, $D_2O$ austauschbar, 4-OH), 3.45 (s, 3H, OMe), 3.46 (dd, $^3J_{3,4}$ = 1.8 Hz, $^3J_{3,2}$ = 4.2 Hz, 1H, 3-H), 3.54 (dd, $^3J_{2,1}$ = 3.1 Hz, $^3J_{3,2}$ = 4.2 Hz, 1H, 2-H), 3.67 ($\psi$dt, $^3J_{5,6}$ = 4.9 Hz, $^3J$ = 9.0 Hz, 1H, 5-H), 3.74-3.85 (m, u.a. mit J = 5.3 Hz, J = 10.5 Hz, 2H, 6-H), 3.93 (breites d, J = 8.7 Hz, 1H, 4-H), 4.88 (dd, $^4J_{1,5}$ = 0.5 Hz, $^3J_{1,2}$ = 3.1 Hz, 1H, 1-H). - **Analyse :** $C_{15}H_{30}O_5Si$ (318.48); ber.: C:56.57 %, H: 9.49 %; gef.: C: 56.03 %, H: 9.30 %.

**3. Herstellung von (-)-(2S,5S)-5-((Dimethyl-thexyl-silyloxy)-methyl)-2-methoxy-2,5-dihydrofuran-4-carbaldehyd (24)**

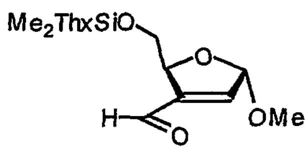

**24**

In einem 500 mL Dreihalskolben, ausgestattet mit Tropftrichter, Wasserabscheider und Rückflußkühler (mit aufgesetztem Hg-Bubbler) wurden in einer Argonatmosphäre 3.66 g (42.15 mmol) LiBr und 5.10 mL (42.46 mmol) frisch im Kugelrohr (40-60°C, ca. 2 mbar) destillierter Tetramethylharnstoff in 200 mL Toluol vorgelegt und unter magnetischem Rühren im Ölbad zum Rückfluß erhitzt. Anschließend tropfte man 3.14 g (9.86 mmol) des vorstehend beschriebenen Epoxidgemisches (22, 23), gelöst in 150 mL Toluol, innerhalb von 2.5 h zur siedenden Reaktionslösung. Man ließ noch weitere 30 min reagieren, kühlte dann auf Raumtemp. ab und versetzte mit 170 mL Ether. Zur Abtrennung eines braunen Polymerisationsproduktes filtrierte man durch 50 g Kieselgel, wusch mit 150 mL Toluol / Ether = 2+1 nach und zog im Vak. am Rotationsverdampfer das Lösungsmittel ab. Der braune, ölige Rückstand wurde durch Flash-Chromatographie an 220 g Kieselgel (*n*-Hexan / Essigester = 6+1) gereinigt. Man erhielt nach Trocknen im Ölpumpenvakuum 2.23 g (75 %) des Aldehydes 24 als analytisch reines, rot-braunes Öl. - **DC** : *n*-Hexan / Essigester = 4+1, $R_f$= 0.37. - **Drehwert** : $[\alpha]^{20}_{589}$ = - 68.7, (c = 1.0 in $CHCl_3$); - **CD** : $\Theta$ ($\lambda$) = - 19352 (233.0 nm), - 2429 (335.0 nm), - 2052 (341.0 nm), - 2832 (348.0 nm), - 1701 (357.0 nm), - 2321 (365.0 nm), - 679 (378.0 nm), - 947 (384.0 nm), (c= 0.007 in *n*-Hexan). - **UV (MeOH)** : $\lambda_{max}(\varepsilon)$ = 214.0 nm (8935). - **FT-IR (Film) :** $\tilde{\nu}$ = 2958, 2868 (m,m, ges. C-H), 2830, 2717 (m,w, C-H-Aldehyd), 1692 (s, C=O), 1465, 1354, 1252, 1190, 1136 (jeweils m), 1042 (s, C-O), 969, 903, 833, 778 (jeweils m). - **$^1$H-NMR :** (270 MHz, $CDCl_3$): $\delta$ = 0.02 + 0.06 (s+s, jeweils 3H, $(CH_3)_2Si$), 0.78 ($\psi$s, 6H, $(CH_3)_2C$), 0.82 ($\psi$d, J = 6.9 Hz, 6H, $(C\underline{H}_3)_2CH$), 1.55 (sept, J = 6.9 Hz, 1H, $(CH_3)_2C\underline{H}$), 3.43 (s, 3H, OMe), 3.90 ($\psi$d, J = 2.5 Hz, 2H, $CH_2O$), 5.12-5.16 (m, 1H, 5-H), 5.91 ($\psi$d, J = 4.3 Hz, 1H, 2-H), 6:68 ($\psi$s, 1H, 3-H), 9.88 (s, 1H,CHO). - **Analyse :** $C_{15}H_{28}O_4Si$ (300.47); ber.: C: 59.96%, H: 9.39 %; gef.: C: 59.99 %, H: 9.34 %.

**4. Herstellung von (-)-(2S,5S)-5-((Dimethyl-thexyl-silyloxy)-methyl)-2-methoxy-4-vinyl-2,5-dihydrofuran (25)**

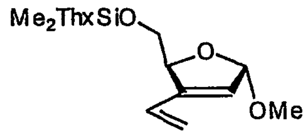

**25**

In einem 250 mL Schlenkkolben wurden in einer Argonatmosphäre 1.85 g (5.18 mmol) Methyltriphenylphosphoniumbromid in 50 mL abs. THF vorgelegt und auf - 40°C gekühlt. Unter magnetischem Rühren und Argon-Gegenstrom injizierte man mit einer Spritze 3.0 mL (4.80 mmol) *n*-Butyllithium-Lösung (1.6 M in Hexan) hinzu, ließ 30 min reagieren (Gelbfärbung), kühlte auf - 78°C ab und gab dann 1.01 g (3.36 mmol) des Aldehydes 24, gelöst in 20 mL abs. THF, zur Reaktionslösung. Man ließ langsam auf Raumtemp. kommen (ca. 4.5 h) wobei sich die Lösung orange färbte, und überführte sie mit 200 mL Ether in einen Scheidetrichter. Nach Zugabe von 200 mL Eiswasser trennte man die Phasen und wusch die organische Phase mit 200 mL ges. wäßr. NaCl-Lösung. Die wäßrigen Phasen extrahierte man mit 1 x 200 mL und 2 x 100 mL Ether und trocknete die vereinigten organischen Phasen mit $Na_2SO_4$. Nach Filtration wurde im Vak. am Rotationsverdampfer eingeengt und der Rückstand durch Flash-Chromatographie an 60 g Kieselgel (*n*-Hexan / Essigester = 10+1) gereinigt. Man erhielt nach Trocknen im Ölpumpenvakuum 0.9741 g (97 %) analysenreines **25** als farbloses Öl. - **DC** : *n*-Hexan/Essigester = 10+1, $R_f$ = 0.32. - **Drehwerte** :$[\alpha]^{20}_{589}$ = - 49.5, $[\alpha]^{20}_{578}$ = - 52.5, $[\alpha]^{20}_{546}$ = - 62.5, $[\alpha]^{20}_{436}$ = - 136.3, $[\alpha]^{20}_{365}$ = - 282.7, (c = 1.1 in n-Hexan). - **CD :** $\Theta$ ($\lambda$) = - 35370 (227.5 nm), (c =0.002 in *n*-Hexan). - **UV (n-Hexan):** $\lambda_{max}(\varepsilon)$ = 226.0 nm (15664). - **FT-IR (Film):** $\tilde{\nu}$ = 3092 (w, unges. C-H), 2946, 2866 (s,s, ges. C-H),1597 (m, C=C), 1465, 1367, 1251, 1194, 1140 (m,m,s,m,s), 1066 (s, C-O), 960, 914, 832, 778 (m,m,s,m). - **$^1$H-NMR :** (270 MHz, $C_6D_6$) : $\delta$ = 0.09 + 0.11 (s+s, jeweils 3H, $(CH_3)_2Si$), 0.89 ($\psi$s, 6H, $(CH_3)_2C$), 0.94 (d, J = 6.8 Hz, 6H, $(\underline{CH_3})_2CH$), 1.63 (sept, J = 6.9 Hz, 1H, $(CH_3)_2C\underline{H}$), 3.29 (s, 3H, OMe), 3.58 (dd, $^3J$ = 3.7 Hz, $^2J$ =11.1 Hz, 1H, $CH_2O$), 3.79 ($\psi$dd, $^3J$= 2.7 Hz, $^2J$ = 10.9 Hz, 1H, $CH_2O$), 4.93 (d, J = 11.2 Hz, 1H, 2'-H(*E*)), 4.97 - 5.02 (m, 1H, 5-H), 5.02 (d, J = 17.7 Hz, 1H, 2'-H(*Z*)), 5.57 ($\psi$s, 1H, 3-H), 5.87 ($\psi$d, J = 4.0 Hz, 1H, 2-H), 6.20 (dd, $^3J$ = 10.9 Hz, $^3J_{1',2'(Z)}$ = 18.1 Hz, 1H, 1'-H). - **$^{13}$C-NMR :** (62.90 MHz, $C_6D_6$): $\delta$ = - 3.4, - 3.3 (jeweils q, $(CH_3)_2Si$), 18.7, 18.8 (jeweils q, $(\underline{C}H_3)_2CH$), 20.5, 20.6 (jeweils q, $(\underline{C}H_3)_2C$), 25.4 (s, Si-C), 34.6 (d, $(CH_3)_2\underline{C}H$), 53.3 (q, $OCH_3$), 64.8 (t, $CH_2O$), 85.6 (d, C-5), 108.6 (d, C-2), 117.6 (t, C-2'), 126.2 (d, C-3), 129.5 (d, C-1'), 143.5 (s, C-4). - **Analyse :** $C_{16}H_{30}O_3Si$ (298.50); ber.: C: 64.38%, H:10.13 %; gef.: C: 64.35 %, H: 10.13 %.

**5. Herstellung von (-)-(2'S,5'S)-3-*E*-(5'-((Dimethyl-thexyl-silyloxy)-methyl)-2'-methoxy-2',5'-dihydrofuran-4-yl)-acrylsäureethylester (26)**

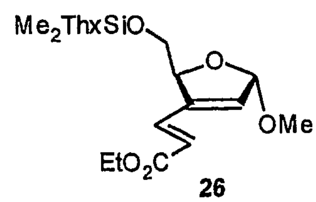

**26**

In einem 250 mL Zweihalskolben mit Rückflußkühler und aufgesetztem Hg-Bubbler wurden 1.64 g (5.46 mmol) des Aldehydes **24** und 2.85 g (8.18 mmol) Ethoxycarbonyl-methylentriphenylphosphoranin 100 mL abs. THF gelöst und unter magnetischem Rühren im Wasserbad zum Sieden erhitzt. Da nach 1 h die DC-Reaktionskontrolle (Bed. s. u.) einen vollständigen Umsatz, ließ man auf Raumtemp. abkühlen, engte im Vak. am Rotationsverdampfer ein und trocknete kurz im Ölpumpenvakuum. Den verbliebenen Rückstand zog man mit $CH_2Cl_2$ auf Kieselgel auf und reinigte ihn durch Flash-Chromatographie an 80 g Kieselgel (*n*-Hexan / Essigester = 6+1). Man erhielt 1.92g (95 %) des Esters **26** als gelbes Öl in analytisch reiner Form. **- DC :** *n*-Hexan / Essigester = 4 + 1, $R_f$= 0.39. - **Drehwerte** : $[\alpha]^{20}_{589}$ = - 16.8, $[\alpha]^{20}_{578}$ = - 18.1, $[\alpha]^{20}_{546}$ = - 23.3, $[\alpha]^{20}_{436}$ = - 66.5, $[\alpha]^{20}_{365}$ = - 171.8, (c = 1.2 in *n*-Hexan). - **CD** : $\Theta$ ($\lambda$) = + 1631 (224.0 nm),

- 16944 (253.5 nm), (c = 0.002 in *n*-Hexan). - **UV (*n*-Hexan) :** $\lambda_{max}(\varepsilon)$ = 253.5 nm (19913). - **FT-IR (Film)** : $\tilde{\nu}$ = 3080 (w, unges. C-H), 2957, 2868 (m,m, ges. C-H), 1720 (s, C=O), 1648, 1609 (m,m, C=C), 1465, 1366, 1307, 1254, 1177 (m,m,m,m,s), 1041 (s, C-O), 833, 778 (s,m). - **$^1$H-NMR :** (270 MHz, $C_6D_6$) : $\delta$ = 0.04 + 0.06 (s+s, jeweils 3H, $(CH_3)_2Si$), 0.85 ($\psi$s, 6H, $(CH_3)_2C$), 0.90 (d, J = 6.9 Hz, 6H, $(CH_3)_2CH$), 0.98 (t, J =7.1 Hz, 3H, $OCH_2CH_3$), 1.59 (sept, J = 6.9 Hz, 1H, $(CH_3)_2CH$), 3.22 (s, 3H, OMe), 3.53 (dd, $^3J$ = 3.2 Hz, $^2J$ = 11.1 Hz,1H, $CH_2O$), 3.65 (dd, $^3J$ = 3.5 Hz, $^2J$ = 11.1 Hz, 1H, $CH_2O$), 4.02 (q, J = 7.1 Hz, 2H, $OCH_2CH_3$), 4.85-4.89 (m, 1H, 5'-H), 5.67 ($\psi$s, 1H, 3'-H), 5.75 ($\psi$d, J = 4.1 Hz, 1H, 2'-H),5.99 (d, J = 16.2 Hz, 1H, 2-H), 7.46 (d, J = 16.2 Hz, 1H, 3-H). - **$^{13}$C-NMR :** (62.90 MHz, $C_6D_6$) : $\delta$ = - 3.5, - 3.4 (jeweils q, $(CH_3)_2Si$), 14.2 (q, $CH_3CH_2O$), 18.7 (q, $(CH_3)_2CH$), 20.5 (q, $(CH_3)_2C$), 25.4 (s, Si-C), 34.5 (d, $(CH_3)_2CH$), 53.5 (q, $OCH_3$), 60.4 (t, $CH_3CH_2O$), 64.5 (t,$CH_2O$), 85.4 (d, C-5'), 108.3 (d, C-2'), 122.5 (d, C-3'), 132.4 (d, C-3), 135.8 (d, C-2), 142.0 (s, C-4'), 165.9 (s, C-1). **-Analyse**: $C_{19}H_{34}O_5Si$ (370.56); ber.: C: 61.58%, H: 9.25 %; gef.: C: 61.77 %, H: 9.27 %.

6. **Herstellung von (-)-*endo*-[1',2',3,4-η-((2S,5S)-5-((Dimethyl-thexyl-silyloxy)-methyl)-2-methoxy-4-(*s-cis*-vinyl)-2,5-dihydrofuran)]-tricarbonyl-eisen (27)**

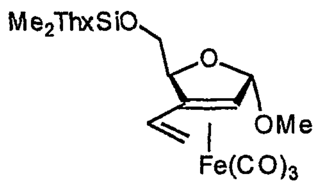

**27**

In einem 100 mL Zweihalskolben mit Rückflußkühler und aufgesetztem Hg-Bubbler wurden in einer Argonatmosphäre 0.2425 g (0.81 mmol) des Vinyldihydrofurans 25 und 0.44 g (1.21mmol) $Fe_2(CO)_9$ in 40 mL entgastem Ether (frisch durch basisches $Al_2O_3$ filtriert) vorgelegt und unter weitgehendem Lichtausschluß zum Sieden erhitzt (Grünfärbung durch entstehendes $Fe_3(CO)_{12}$). Nach 2 h versetzte man erneut unter Argon-Gegenstrom mit 0.15 g (0.41 mmol) $Fe_2(CO)_9$, spülte mit wenig Ether nach und erhitzte solange unter Rückfluß, bis laut DC (Bed. s.u.) alle eisenhaltigen Nebenprodukte (wahrscheinlich $\eta^2$-$Fe(CO)_4$-Komplexe) verschwunden waren (insgesamt 6.5 h). Zur Aufarbeitung wurde die Reaktionslösung im Vak. am Rotationsverdampfer eingeengt, kurz im Ölpumpenvakuum getrocknet, anschließend unter striktem Sauerstoffausschluß mit wenig *n*-Hexan in eine Flash-Säule überführt und mit Argondruck chromatographiert (90 g entgastes Kieselgel, erst *n*-Hexan zum Entfernen von $Fe(CO)_5$ und $Fe_3(CO)_{12}$, dann *n*-Hexan / Essigester = 10+1). Man erhielt nach Trocknen im Ölpumpenvakuum 0.3091 g (87 %) Rohprodukt in Form eines rotbraunen Öls, bei dem es sich laut $^1$H-NMR um ein Gemisch des *endo*-Komplexes **27** und seines *exo*-Diastereomeren im Verhältnis von 77:23 handelte. Das diastereomere Nebenprodukt konnte durch präparative HPLC abgetrennt werden. Man isolierte 0.1316g (37 %) des Komplexes **27** als rotbraunes Öl in analytisch reiner Form. - **DC** : *n*-Hexan / Essigester = 10+1, $R_f$ = 0.39. - **analyt. HPLC :** *n*-Hexan / Ether = 10+0.25; MN Nucleosil 50-10; 2 mL/min; Refraktom.; Retentionszeit : 5.64 min. - **s.-präp. HPLC :** *n*-Hexan/Essigester = 10+0.3;10 mL/min; RI-Detekt. - **Drehwerte** : $[\alpha]_{589}^{20}$ = - 62.2, $[\alpha]_{578}^{20}$ = - 66.7, $[\alpha]_{546}^{20}$ = - 82.9, bei 436 und 365 nm kein Lichtdurchlaß , (c =1.3 in $CHCl_3$).- **CD** : $\Theta$ ($\lambda$) = - 9215 (258.5 nm), + 1920(284.0 nm), - 7087 (320.0 nm), (c = 0.010 in $CHCl_3$). - **UV** ($CHCl_3$) : $\lambda_{max}(\varepsilon)$ = 289.0 nm (2554). - **FT-IR (Film) :** $\tilde{\nu}$ = 3046 (w, unges. C-H), 2958, 2866 (m,m, ges. C-H), 2054, 1979 (s, s und br., C≡O), 1465, 1365, 1253, 1121 (jeweils m), 1054 (m, C-O), 831, 778 (m,m). - **$^1$H-NMR** : (270 MHz, $CDCl_3$): $\delta$ = - 0.15 (dd, $^2J_{2'anti,2'syn}$ = 2.3 Hz, $^3J_{2'anti,1'}$ = 8.6 Hz,1H, 2'-$H_{anti}$), 0.12 + 0.13 (s+s, jeweils 3H, $(CH_3)_2Si$), 0.86 ($\psi$s, 6H, $(CH_3)_2C$), 0.88 (d, J = 6.9 Hz, 6H, $(CH_3)_2CH$), 1.55 - 1.71 (m, u.a. mit $^3J_{2'syn,1'}$ = 6.5 Hz, 2H, 2'-$H_{syn}$, $(CH_3)_2CH$), 1.77 ($\psi$d, J = 3.0 Hz, 1H, 3-H), 3.42 (s, 3H,OMe), 3.73 (dd, $^3J$= 5.9 Hz, $^2J$ = 10.3 Hz, 1H, $CH_2O$), 3.86 (dd, $^3J$ = 3.6 Hz, $^2J$ = 10.3 Hz, 1H, $CH_2O$), 4.81 ($\psi$t, J = 4.8 Hz, 1H, 5-H), 5.40-5.41(m, 1H, 2-H), 5.44 ($\psi$t, J = 8.1 Hz, 1H, 1'-H). - **$^{13}$C-NMR :** (100.61 MHz, $CDCl_3$) : $\delta$ = - 3.5, - 3.4 (jeweils q, $(CH_3)_2Si$), 18.6 (q, $(CH_3)_2CH$), 20.1, 20.3 (jeweils q, $(CH_3)_2C$), 25.2 (s, Si-C), 34.1 (d, $(CH_3)_2CH$), 36.3 (t, C-2'), 56.1 (q, $OCH_3$), 62.5 (d, C-3), 66.4 (t,$CH_2O$), 74.4 (d, C-1'), 82.5 (d, C-5), 107.9 (d, C-2), 111.5 (s, C-4), 210.2 (br., $Fe(CO)_3$).- **Analyse**: $C_{19}H_{30}FeO_6Si$ (438.38); ber.: C: 52.06% H: 6.90 %; gef.: C: 52.04 %, H: 6.76 %.

7. **Herstellung von (-)-*endo*-[2,3,3',4'-η-(2'S,5'S)-3-*E*-(5'-((Dimethyl-thexyl-silyloxy) methyl)-2'-methoxy-2',5'-dihydrofuran-4-yl)-acrylsäureethylester]-tricarbonyleisen (28)**

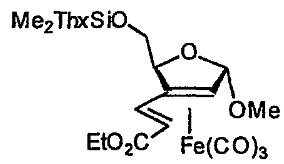

**28**

In einem 100 mL Zweihalskolben mit Rückflußkühler und aufgesetztem Hg-Bubbler wurden in einer Argonatmosphäre 0.2467 g (0.67 mmol) des Esters 26 und 0.36 g (0.99 mmol) $Fe_2(CO)_9$ in 50 mL entgastem Ether (frisch durch basisches $Al_2O_3$ filtriert) vorgelegt und unter weitgehendem Lichtausschluß zum Sieden erhitzt (Grünfärbung durch entstehendes $Fe_3(CO)_{12}$). Nach 2 h versetzte man erneut unter Argon-Gegenstrom mit 0.16 g (0.44 mmol) $Fe_2(CO)_9$, spülte mit wenig Ether nach und erhitzte solange unter Rückfluß, bis laut DC (Bed. s.u.) alle eisenhaltigen Nebenprodukte verschwunden waren (insgesamt 28 h). Zur Abtrennung unlöslicher $Fe_x(CO)_y$-Komplexe filtrierte man die Reaktionslösung unter Schutzgas durch 30 g Kieselgel, wusch mit n-Hexan / Essigester = 1+1 nach und zog im Vak. am Rotationsverdampfer das Lösungsmittel ab. Der dunkle, ölige Rückstand wurde anschließend unter striktem Sauerstoffausschluß mit wenig n-Hexan in eine Flash-Säule überführt und mit Argondruck chromatographiert (Hexan / Essigester = 8+1). Man erhielt nach Trocknen im Ölpumpenvakuum 0.3115 g (92 %) Rohprodukt in Form eines rotbraunen Öls, bei dem es sich laut $^1$H-NMR um ein Gemisch von 28 und seinem exo-Diastereomeren im Verhältnis von 79 : 21 handelte. Die Abtrennung des diastereomeren Nebenproduktes erfolgte durch semi-präparative HPLC (Bed. s.u.). Man erhielt 0.1938g (57 %) des *endo*-Komplexes **28** als rotbraunes Öl in analytisch reiner Form.
- **DC** : *n*-Hexan / Essigester = 10+1, $R_f$= 0.29. - **analyt. HPLC** : *n*-Hexan / Essigester = 10+0.5; MN Nucleosil 50-10; 2 mL/min; Refraktom.; Retentionszeit : 4.56 min. - **s.-präp. HPLC** : *n*-Hexan / Essigester = 10+0.5;10 mL/min; RI-Detekt. - **Drehwerte:** $[\alpha]^{20}_{589}$ = - 111.0, $[\alpha]^{20}_{578}$ = - 117.0, $[\alpha]^{20}_{546}$ = - 136.8, bei 436 und 365 nm kein Lichtdurchlaß , (c = 1.2 in $CHCl_3$). - **CD :** Θ (λ) = ca. - 25985 (256.0 nm),+ 11968 (314.5 nm), - 10338 (350.0 nm), + 1566 (402.5 nm), (c= 0.011 in $CHCl_3$). - **UV ($CHCl_3$)** : $\lambda_{max}(\varepsilon)$ = 301.0 nm (2883). - **FT-IR (Film):** 3070 (w, unges. C-H), 2959, 2867 (m,m, ges. C-H), 2063, 1986 (s, s und br., C≡O), 1709 (m, C=O), 1466, 1368, 1253, 1194, 1125, 1052, 833 (jeweils m). - **$^1$H-NMR** : (270 MHz, $CDCl_3$) : δ = 0.11 + 0.12 (s+s, jeweils 3H, $(CH_3)_2Si$), 0.59 (d, J = 7.5Hz, 1H, 2-H), 0.84 (ψs, 6H, $(CH_3)_2C$), 0.87 (d, J = 6.9 Hz, 6H, $(C\underline{H}_3)_2CH$), 1.25 (t, J = 7.1 Hz, 3H, $OCH_2C\underline{H}_3$), 1.62 (sept, J = 6.9 Hz, 1H, $(CH_3)_2C\underline{H}$), 2.03 - 2.04 (m, 1H, 3'-H), 3.42 (s, 3H, OMe), 3.78 (dd, $^3J$ = 5.2 Hz, $^2J$ = 10.5 Hz, 1H, $CH_2O$), 3.88 (dd, $^3J$ = 3.3 Hz, $^2J$ = 10.5 Hz, 1H, $CH_2O$), 4.02 - 4.23 (m, u.a. mit J = 7.2Hz, 2H, $OC\underline{H}_2CH_3$), 4.79 - 4.82 (m, 1H, 5'-H), 5.44 - 5.45 (m, 1H, 2'-H), 5.96 (d, J = 8.2 Hz, 1H, 3-H). - **$^{13}$C-NMR**: (100.61 MHz, $CDCl_3$): δ = - 3.5 (q, $(CH_3)_2Si$), 14.2 (q, $\underline{C}H_3CH_2O$), 18.5, 18.6 (jeweils q, $(\underline{C}H_3)_2CH$), 20.2, 20.3 (jeweils q, $(\underline{C}H_3)_2C$), 25.2 (s, Si-C), 34.1 (d, $(CH_3)_2\underline{C}H$), 43.4 (d, C-2), 56.2 (q, $OCH_3$), 60.5 (t, $CH_3\underline{C}H_2O$), 63.1 (d, C-3'), 66.2 (t, $CH_2O$), 75.4 (d, C-3), 82.6 (d, C-5'), 107.7 (d, C-2'), 110.1 (s, C-4'), 172.4 (s, C-1), 212 (br., $Fe(CO)_3$). - **Analyse** : $C_{22}H_{34}FeO_8Si$ (510.44); ber.: C: 51.77 %, H: 6.71 %; gef.: C: 51.97 %, H: 6.57 %.

**8. Herstellung von *exo*-[1",2",2',3'-η-(2',3'-Didehydro-2',3'-dideoxy-5'-O-(dimethylthexyl-silyl)-3'-*s*-*cis*-vinyl-β-*D*-uridin]-tricarbonyl-eisen (29)**

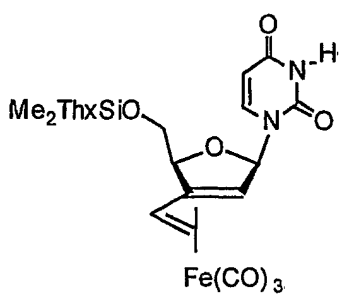

**29**

In einem ausgeheizten 100 mL Dreihalskolben mit Rückflußkühler und aufgesetztem Hg-Bubbler wurden unter striktem Sauerstoff-und Feuchtigkeitsausschluß zu 0.5715 g (2.23 mmol) 2,4-Bistrimethylsiloxy-pyrimidin 0.3260 g (0.74mmol) Komplex 27 und 100 mL $CH_2Cl_2$ (frisch durch basisches $Al_2O_3$ filtriert und entgast) gegeben und unter magnetischem Rühren zum Sieden erhitzt. Zu dieser Lösung gab man mittels einer Dosierpumpe innerhalb von 2 h 1.75 mL (1.75 mmol) $SnCl_4$- Lösung (1 M in Heptan) hinzu und ließ, nachdem die DC-Reaktionskontrolle (Bed. s.u.) einen vollständigen Umsatz anzeigte, auf Raumtemp. abkühlen. Zur Aufarbeitung wurde die Lösung mit 20 mL $CH_2Cl_2$ in einem Scheidetrichter überführt und rasch mit jeweils 100 mL ges. wäßr. $NaHCO_3$-Lösung und ges. wäßr. NaCl-Lösung gewaschen. Die wäßrigen Phasen extrahierte man mit 2 x 50 mL $CH_2Cl_2$ und trocknete die vereinigten organischen Phasen mit $Na_2SO_4$. Zur Vermeidung einer möglichen oxidativen Dekomplexierung entgaste man die Lösung sofort durch kurzzeitiges Anlegen eines leichten Ölpumpenvakuums und belüftete anschließend wieder mit Argon. Nach Filtration wurde im Vak. am Rotationsverdampfer eingeengt und kurz im Ölpumpenvakuum getrocknet. Anschließend wurde der gelbe, ölige Rückstand unter striktem Sauerstoffausschluß in eine Flash-Säule überführt und mit Argondruck chromatographiert (90 g entgastes Kieselgel, *n*-Hexan / Essigester = 3+2). Man erhielt nach Trocknen im Hochvakuum 0.3617 g (94 %) Rohprodukt in Form eines gelben Schaumes, bei dem es sich laut $^1$H-NMR um ein Gemisch des Komplexes **29** und des Diastereomeren im Verhältnis von 84 : 16 handelte.

Die Abtrennung des diastereomeren Nebenproduktes erfolgte durch semi-präparative HPLC (Bed. s.u.). Man erhielt 0.2588 g (67 %) des Komplexes **29** und 0.0451 als gelben, erstarrten Schaum, in analytisch reiner Form.
- **Schmp**. : 76 - 78°C ($CH_2Cl_2$), gelber, fester Schaum. - **DC** : *n*-Hexan / Essigester = 1+1, $R_f$= 0.40. - **analyt. HPLC** : *n*-Hexan / Essigester = 10+4.33; MN Nucleosil 50-10; 2 mL/min; Refraktom.; Retentionszeit : 4.65 min. - **s.-präp. HPLC** : *n*-Hexan / Essigester = 10+5; 10 mL/min; RI-Detekt. - **Drehwerte** : $[\alpha]^{20}_{589}$ = - 165.7, $[\alpha]^{20}_{578}$ = - 175.1, $[\alpha]^{20}_{546}$ = - 206.8, bei 436 und 365 nm kein Lichtdurchlaß , (c= 0.9 in MeOH). - **CD** : Θ (λ) = - 1079 (271.5 nm), - 21924 (306.0 nm), (c = 0.006 in MeOH). - **UV (MeOH) :** $\lambda_{max}(\varepsilon)$ = 260.0 nm (14251). - **FT-IR (KBr)** : $\tilde{\nu}$ = 3412, 3192 (w,w, jeweils br., N-H), 3061 (w, unges. C-H), 2959, 2867 (m,w, ges. C-H), 2057, 1987 (s, s und br., C≡O), 1686 (s und br., C=O), 1464, 1255, 1123, 832 (jeweils m). - **$^1$H-NMR** : (270 MHz, $CDCl_3$) : δ = - 0.03 (dd, $^2J_{2''anti,2''syn}$ = 2.4 Hz, $^3J_{2''anti,1''}$ = 8.6 Hz, 1H, 2''-$H_{anti}$), 0.15 + 0.18 (s+s, jeweils 3H, $(CH_3)_2Si$), 0.86-0.88 (m, 12H, $(CH_3)_2C$, $(C\underline{H}_3)_2CH$), 1.63 (sept, J = 7.0 Hz, 1H, $(CH_3)_2C\underline{H}$), 1.79 - 1.82 (m, 2H, 2'-H, 2''-$H_{syn}$), 3.94 - 4.05 (m, 2H, 5'-H), 4.99 (ψt, J = 3.8 Hz, 1H, 4'-H), 5.59 (ψt, J = 7.8 Hz, 1H, 1''-H), 5.67 (dd, J =2.2 Hz, $^3J_{5,6}$ = 8.2 Hz, 1H, 5-H), 6.15 (s, 1H, 1'-H), 7.85 (d, J =8.2 Hz, 1H, 6-H), 8.28 (breites s, 1H, $D_2O$ austauschbar, N-H). - **$^{13}$C-NMR** : (62.90 MHz, $CDCl_3$) : δ = - 3.4, - 3.3 (jeweils q, $(CH_3)_2Si$), 18.5, 18.6 (jeweils q, $(\underline{C}H_3)_2CH$), 20.2, 20.3 (jeweils q, $(\underline{C}H_3)_2C$), 25.5 (s, Si-C), 34.0 (d, $(CH_3)_2\underline{C}H$), 37.6 (t, C-2''), 58.2 (d, C-2'), 66.3 (t, C-5'), 75.6 (d, C-1''), 85.0 (d, C-4'), 90.4 (d, C-1'), 101.9 (d, C-5), 109.7 (s, C-3'), 140.3 (d, C-6), 150.2 (s, C-4), 163.0 (s, C-2), 209.3 (br., $Fe(CO)_3$). - **MS :** (MAT 8222, EI, 70 eV): m/z (%) = 518 (2) [$M^+$], 490 (1) [$M^+$- CO], 434 (40) [$M^+$- 3xCO],349 (48) [$M^+$- 3xCO, - Thexyl]. - **Analyse :** $C_{22}H_{30}FeN_2O_7Si$ (518.42); ber.: C: 50.97 %, H: 5.83 %, N: 5.40 %; gef.: C: 50.95 %, H: 5.67 %, N: 5.66 %.

**9. Herstellung von *exo*-[1'',2'',2',3'-η-(2',3'-Didehydro-2',3'-dideoxy-5'-O-(dimethyl-thexylsilyl)-3'-*s*-*cis*-vinyl-β-*D*-thymidin]-tricarbonyl-eisen (30)**

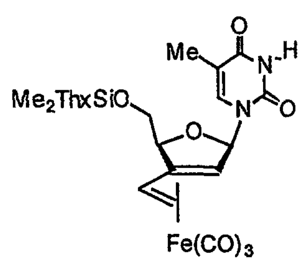

**30**

In einem ausgeheizten 100 mL Zweihalskolben mit Hg-Bubbler wurden unter striktem Sauerstoff-und Feuchtigkeitsausschluß zu 0.1842 g (0.68 mmol) 5-Methyl-2,4-bis-trimethylsiloxy-pyrimidin (0.1019 g, 0.23mmol) Komplex **28** und 50 mL $CH_2Cl_2$ (frisch durch basisches $Al_2O_3$ filtriert und entgast) gegeben und unter magnetischem Rühren im Eisbad auf 0°C gekühlt. Zu dieser Lösung gab man mittels einer Dosierpumpe innerhalb von 1.5 h 0.70 mL (0.70 mmol) $SnCl_4$-Lösung (1 M in Heptan) hinzu, kontrollierte den Umsatz per DC (Bed. s.u.) und überführte dann den Kolbeninhalt mit einer Transfer-Nadel in 100 mL einer kräftig gerührten ges. wäßr. $NaHCO_3$-Lösung. Anschließend wurde die Lösung mit 20 mL $CH_2Cl_2$ in einem Scheidetrichter überführt und rasch mit 100 mL ges. wäßr. NaCl-Lösung

gewaschen. Die übrige Aufarbeitung erfolgte analog zu der in Abschnitt 7.8.3.1. beschriebenen. Nach Filtration vom Trockenmittel und Entfernen des Lösungsmittels im Vak. am Rotationsverdampfer wurde der Rückstand unter striktem Sauerstoffausschluß in eine Flash-Säule überführt und mit Argondruck chromatographiert (80 g entgastes Kieselgel, *n*-Hexan / Essigester= 3+2). Man erhielt nach Trocknen im Ölpumpenvakuum 0.1237g (100 %) Rohprodukt in Form eines gelben Schaumes, bei dem es sich laut $^1$H-NMR um ein Gemisch des Komplexes **30** und seines Diastereomeren im Verhältnis von 59 : 41 handelte. Die Abtrennung des diastereomeren Nebenproduktes erfolgte durch semi-präparative HPLC (Bed. s.u.). Man erhielt 0.0619 g (50 %) des Komplexes **30** als gelben, erstarrten Schaum, in analytisch reiner Form.

**Komplex E14 : - Schmp. :** 74 - 77°C (subl., $CH_2Cl_2$), gelber, fester Schaum. - **DC :** *n*-Hexan / Essigester = 1+1, $R_f$ = 0.48. - analyt. **HPLC :** *n*-Hexan / Essigester = 10 + 4.33; MN Nucleosil 50-10; 2 mL/min; Refraktom.; Retentionszeit : 2.85 min. - **s.-präp.** HPLC : *n*-Hexan / Essigester = 10 + 5; 10 mL/min; RI-Detekt. - Drehwerte : $[\alpha]^{20}_{589}$ = - 176.5, $[\alpha]^{20}_{578}$ = - 186.5, $[\alpha]^{20}_{546}$ = - 219.8, $[\alpha]^{20}_{436}$ = - 468.1, bei 365 nm kein Lichtdurchlaß , (c = 0.9 in MeOH). - CD : Θ (λ) = ca. - 9964 (241.5 nm), -12588 (257.0 nm), - 8268 (278.0 nm), - 22366 (305.0 nm), (c =0.005 in MeOH). - UV **(MeOH):** $\lambda_{max}(\varepsilon)$ = 266.0 nm (13387). - **FT-IR (KBr) :** $\tilde{\nu}$ = 3413, 3185 (w,w, jeweils br., N-H), 3059 (w, unges. C-H), 2959, 2867 (m,w, ges. C-H), 2057, 1987 (s, s und br., C≡O), 1691 (s und br., C=O), 1466, 1255, 1118, 832 (jeweils m). - **$^1$H-NMR:** (250 MHz, $CDCl_3$) : δ = 0.00 (dd, $^2J_{2''anti,2''syn}$ = 2.5 Hz, $^3J_{2''anti,1''}$ = 8.7 Hz, 1H, 2"-$H_{anti}$), 0.16 + 0.17 (s+s, jeweils 3H, $(CH_3)_2Si$), 0.88 - 0.90 (m, 12H, $(CH_3)_2C$, $(\underline{CH_3})_2CH$), 1.65 (sept, J = 6.9 Hz, 1H, $(CH_3)_2\underline{CH}$), 1.79 - 1.83 (m, u.a. mit J = 2.5 Hz, 2H, 2"-$H_{syn}$, 2'-H), 1.92 (d, $^4J$ = 1.2 Hz, 3H, 5-$CH_3$), 3.84 (dd, $3J_{5'a,4'}$ = 6.2 Hz, $^2J_{5'a,5'b}$ = 10.8 Hz, 1H, 5'-Ha), 3.98 (dd, $^3J_{5'b,4'}$ = 4.8 Hz, $^2J_{5'b,5'a}$ = 10.8 Hz, 1H, 5'-Hb), 4.92 (ψt, J = 5.2 Hz, 1H, 4'-H), 5.64 (ψt, J = 7.9 Hz, 1H, 1"-H), 6.09 (s, 1H, 1'-H), 7.35 (d, $^4J$ = 1.2 Hz, 1H, 6-H), 8.40 (breites s, 1H, $D_2O$ austauschbar, N-H). - **$^{13}$C-NMR :** (62.90 MHz, $CDCl_3$): δ = - 3.3 (q, $(CH_3)_2Si$), 12.7 (q, 5-$\underline{C}H_3$), 18.5, 18.6 (jeweils q, $(\underline{C}H_3)_2CH$), 20.2, 20.4 (jeweils q, $(\underline{C}H_3)_2C$), 25.4 (s, Si-C), 34.0 (d, $(CH_3)_2\underline{C}H$), 37.6 (t, C-2"), 57.5 (d, C-2'), 66.3 (t, C-5'), 76.2 (d, C-1"), 84.5 (d, C-4'), 90.8 (d, C-1'), 109.8 (s, C-3'), 110.3 (s, C-5), 135.5 (d, C-6), 150.3 (s, C-4), 163.5 (s, C-2), 209.5 (br., $Fe(CO)_3$). - **MS** : (MAT 8222, EI, 70 eV): m/z (%) = 532 (3) [$M^+$], 504 (1) [$M^+$- CO], 448 (40) [$M^+$- 3xCO],363 (50) [$M^+$- 3xCO, - Thexyl]. - **Analyse**: $C_{23}H_{32}FeN_2O_7Si$ (532.45); ber.: C: 51.88 %, H: 6.06 %, N: 5.26 %; gef.: C: 51.96 %, H: 6.11 %, N: 5.19 %.

**10. Herstellung von *exo*-[2',2",3',3"-η-(3'-(3"(*E*)-Acrylsäureethylester-3-yl)-2',3'-didehydro-2',3'-dideoxy-5'-O-(dimethyl-thexyl-silyl)-f3b-*D*-uridin)]-tricarbonyl-eisen (31)**

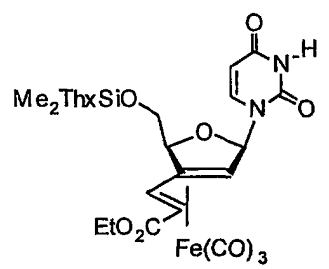

**31**

Unter striktem Sauerstoff- und Feuchtigkeitsausschluß wurden 0.2495 g (0.97 mmol) 2,4-Bistrimethylsiloxy-pyrimidin, 0.1774 g (0.35 mmol) Ester **28** und 80 mL $CH_2Cl_2$ unter magnetischem Rühren zum Sieden erhitzt. Mittels einer Dosierpumpe wurde innerhalb von 2 h mit 1.20 mL (1.20 mmol) $SnCl_4$-Lösung (1 M in Heptan) zugegeben. Nach dem die DC-Reaktionskontrolle (Bed. s.u.) vollständigen Umsatz anzeigte, wurde wie oben beschrieben aufgearbeitet. Nach Filtration vom Trockenmittel und Entfernen des Lösungsmittels im Vak. am Rotationsverdampfer wurde der Rückstand unter striktem Sauerstoffausschluß in eine Flash-Säule überführt und mit Argondruck chromatographiert (80 g entgastes Kieselgel, *n*-Hexan / Essigester = 1+1). Man erhielt nach Trocknen im Ölpumpenvakuum 0.1968 g (96 %) Rohprodukt in Form eines gelben Schaumes, bei dem es sich laut $^1$H-NMR um ein Gemisch der Komplexe **31** und seines Diastereomeren im Verhältnis von 87 : 13 handelte. Die Abtrennung des diastereomeren Nebenproduktes erfolgte durch semi-präparative HPLC (Bed. s.u.). Man erhielt 0.1497g (73 %) des Komplexes als gelben, erstarrten Schaum, in analytisch reiner Form.

- **Schmp. :** 84 - 86°C ($CH_2Cl_2$), gelber, fester Schaum. - **DC :** *n*-Hexan / Essigester = 1+1, $R_f$ = 0.40. - **analyt. HPLC:** *n*-Hexan / Essigester = 10 + 6.67; MN Nucleosil 50-10; 2 mL/min; Refraktom.; Retentionszeit : 3.75 min. - **s.-präp. HPLC :** *n*-Hexan / Essigester = 10 + 6; 10 mL/min; RI-Detekt. - **Drehwerte :** $[\alpha]^{20}_{589}$ = - 182.0, $[\alpha]^{20}_{578}$ = - 190.4, $[\alpha]^{20}_{546}$ = - 217.4, bei 436 und 365 nm kein Lichtdurchlaß , (c= 1.0 in MeOH). - **CD :** Θ (λ) = ca. - 10310 (272.5 nm),- 14553

(288.0 nm), - 2003 (316.5 nm), - 9848 (344.0 nm), + 3878 (397.0 nm), (c = 0.007 in MeOH). - UV **(MeOH) :** $\lambda_{max}(\varepsilon)$ = 300.0 (3042). - **FT-IR (KBr) :** $\tilde{\nu}$ = 3413, 3203 (w,w, jeweils br., N-H), 3062 (w, unges. C-H), 2960, 2868 (m,w, ges. C-H), 2067, 2004 (s, s und br., C≡O), 1704 (s und br., C=O), 1465, 1378, 1268, 831 (jeweils m). - **$^1$H-NMR :** (270 MHz, $CDCl_3$) : δ = 0.15 + 0.17 (s+s, jeweils 3H, $(CH_3)_2Si$), 0.73 (d, J = 7.6 Hz, 1H, 2"-H), 0.85 - 0.87 (m, 12H, $(CH_3)_2C$, $(C\underline{H}_3)_2CH$), 1.26 (t, J = 7.1 Hz, 3H, $OCH_2C\underline{H}_3$), 1.62 (sept, J = 6.8 Hz, 1H, $(CH_3)_2C\underline{H}$), 2.10 (s, 1H, 2'-H), 3.99 - 4.04 (m, 2H, 5'-H), 4.06 - 4.25 (m, u.a. mit J = 7.1 Hz, 2H, $OC\underline{H}_2CH_3$), 4.98 (ψt, J = 3.8 Hz, 1H, 4'-H), 5.69 (dd, J = 2.3 Hz, $^3J_{5,6}$ = 8.1 Hz, 1H, 5-H), 6.10 (dd, J = 0.7 Hz, $^3J_{3'',2''}$ = 8.4 Hz, 1H, 3"-H), 6.11 (s, 1H, 1'-H), 7.85 (d, J = 8.1 Hz, 1H, 6-H), 8.43 (breites s, 1H, $D_2O$ austauschbar, N-H). - **$^{13}$C-NMR :** (62.90 MHz, $CDCl_3$) : δ = - 3.4, - 3.2 (jeweils q, $(CH_3)_2Si$), 14.1 (q, $\underline{C}H_3CH_2O$), 18.4, 18.5 (jeweils q, $(\underline{C}H_3)_2CH$), 20.2, 20.3 (jeweils q, $(\underline{C}H_3)_2C$), 25.4 (s, Si-C), 33.9 (d, $(CH_3)_2\underline{C}H$), 44.9 (d, C-1"), 58.6 (d, C-2'), 60.9 (t, $CH_3\underline{C}H_2O$), 66.1 (t, C-5'), 76.5 (d, C-3"), 85.1 (d, C-4'), 90.4 (d, C-1'), 102.0 (d, C-5), 108.2 (s, C-3'), 140.1 (d, C-6), 150.1 (s, C-4), 162.6 (s, C-2), 171.5 (s, C-1"), 209.5 (br., $Fe(CO)_3$). - **MS :** (MAT 8222, EI, 70 eV): m/z (%) = 590 (<1) [$M^+$], 545 (3) [$M^+$- $OCH_2CH_3$], 506 (100) [$M^+$- 3xCO], 421 (50) [$M^+$- $Fe(CO)_3$, - $OCH_2CH_3$]. - **Analyse :** $C_{25}H_{34}FeN_2O_9Si$ (590.48); ber.: C: 50.85 %, H: 5.80 %, N: 4.74 %; gef.: C: 50.64 %, H: 5.86 %, N: 4.70 %.

**11. Herstellung von *exo*-[2',2",3',3"-η-(3'-(3"(*E*)-Acrylsäureethylester-3-yl)-2',3'-didehydro-2',3'-dideoxy-5'-O-(dimethyl-thexyl-silyl)-f3b-*D*-thymidin)]-tricarbonyl-eisen (32)**

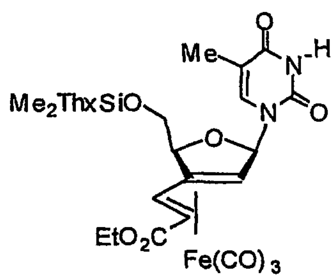

**32**

Analog zu den vorstehenden Vorschriften wurden unter striktem Sauerstoff- und Feuchtigkeitsausschluß 0.2988 g (1.10mmol) 5-Methyl-2,4-bis-trimethylsiloxy-pyrimidin, 0.1464 g (0.29 mmol) Ester **28** und 70 mL $CH_2Cl_2$ unter magnetischem Rühren zum Sieden erhitzt und mittels einer Dosierpumpe innerhalb von 2 h mit 1.30 mL (1.30 mmol) $SnCl_4$-Lösung (1 M in Heptan) versetzt. Nach dem die DC-Reaktionskontrolle (Bed. s.u.) vollständigen Umsatz anzeigte, wurde wie zuvor beschrieben aufgearbeitet. Nach Filtration vom Trockenmittel und Entfernen des Lösungsmittels im Vak. am Rotationsverdampfer wurde der Rückstand unter striktem Sauerstoffausschluß in eine Flash-Säule überführt und mit Argondruck chromatographiert (80 g entgastes Kieselgel, *n*-Hexan / Essigester = 1+1). Man erhielt nach Trocknen im Ölpumpenvakuum 0.1609 g (93 %) Rohprodukt in Form eines gelben Schaumes, bei dem es sich laut $^1$H-NMR um ein Gemisch des Komplexes **32** und eines Diastereomeren im Verhältnis von 70 : 30 handelte. Die Abtrennung des diastereomeren Nebenproduktes erfolgte durch semi-präparative HPLC (Bed. s.u.). Man erhielt 0.0983g (57 %) des Komplexes **32** als gelben, erstarrten Schaum, in analytisch reiner Form.
- **Schmp.:** 81 - 84°C ($CH_2Cl_2$), gelber, fester Schaum. - **DC:** *n*-Hexan / Essigester = 1+1, $R_f$ = 0.45. - **s.-präp. HPLC:** *n*-Hexan / Essigester = 10 + 5; 10 mL/min; RI-Detekt. - **Drehwerte:** $[\alpha]^{20}_{589}$ = - 205.5, $[\alpha]^{20}_{578}$ = - 215.1, $[\alpha]^{20}_{546}$ = - 246.4, bei 436 und 365 nm kein Lichtdurchlaß , (c= 0.9 in MeOH). - CD: Θ (λ) = - 32856 (251.5 nm), - 2643 (316.5 nm), - 10309 (343.5 nm), + 3991 (397.0 nm), (c = 0.005 in MeOH). - UV **(MeOH):** $\lambda_{max}(\varepsilon)$ = 259.5 (15053), λ (ε) = 305.0 (sh, 3032). - **FT-IR (KBr):** $\tilde{\nu}$ = 3412,3190 (w,w,jeweils br., N-H), 3065 (w, unges. C-H), 2959, 2868 (m,w, ges. C-H), 2067, 2004 (s, s und br., C≡O), 1698 (s und br., C=O), 1466, 1265, 1197, 832 (jeweils m). - **$^1$H-NMR:** (250 MHz, $CDCl_3$) : δ = 0.15 + 0.17 (s+s, jeweils 3H, $(CH_3)_2Si$), 0.76 (d, J = 7.7 Hz, 1H, 2"-H), 0.87 (s, 6H, $(CH_3)_2C$), 0.88 (d, J = 5.4 Hz, $(C\underline{H}_3)_2CH$), 1.26 (t, J = 7.1 Hz,3H, $OCH_2C\underline{H}_3$), 1.64 (ψquin, J = 6.8 Hz, 1H, $(CH_3)_2C\underline{H}$), 1.92 (d, $^4J$ = 1.2 Hz, 3H, 5-$CH_3$), 2.14 (s, 1H, 2'-H), 3.90 (dd, $^3J_{5'a,4'}$ = 5.7 Hz, $^2J_{5'a,5'b}$ = 10.9 Hz, 1H, 5'-Ha), 4.01 (dd, $^3J_{5'b,4'}$ = 4.8 Hz, $^2J_{5'b,5'a}$ = 10.9 Hz, 1H, 5'-Hb), 4.06 - 4.23 (m, u.a. mit J = 7.1 Hz, 2H, $OC\underline{H}_2CH_3$), 4.92 (ψt, J = 5.3 Hz, 1H, 4'-H), 6.04 (s, 1H, 1'-H), 6.15 (d, J = 8.3 Hz, 1H, 3"-H), 7.36 (d, $^4J$ = 1.2 Hz, 1H, 6-H), 8.41 (breites s, 1H, $D_2O$ austauschbar, N-H). - **$^{13}$C-NMR :** (62.90 MHz, $CDCl_3$): δ = - 3.3 (q, $(CH_3)_2Si$), 12.7 (q, 5-$\underline{C}H_3$), 14.1 (q, $\underline{C}H_3CH_2O$), 18.5, 18.6 (jeweils q, $(\underline{C}H_3)_2CH$), 20.2, 20.3 (jeweils q, $(\underline{C}H_3)_2C$), 25.4 (s, Si-C), 34.0 (d, $(CH_3)_2\underline{C}H$), 45.0 (d, C-2"), 58.1 (d, C-2'), 60.8 (t, $CH_3\underline{C}H_2O$), 66.1 (t, C-5'), 77.1 (d, C-3"), 84.7 (d, C-4'), 90.9 (d, C-1'), 108.3 (s, C-5), 110.4 (s, C-3'), 135.4 (d, C-6), 150.3 (s, C-4), 163.4 (s, C-2), 171.5 (s, C-1"), 209 (br., $Fe(CO)_3$). - **MS :** (MAT 8222, DCI negativ, $NH_3$): m/z (%) = 604 (20) [$M^+$],

576 (15) [$M^+$-CO], 548 (80) [$M^+$- 2xCO], 265 (100) [$M^+$- $Fe(CO)_3$, - Thymin-1-yl, - $CO_2Et$, - H]. - **Analyse :** $C_{26}H_{36}FeN_2O_9Si$ (604.51); ber.: C: 51.66 %, H: 6.00 %, N: 4.63 %; gef.: C: 51.82 %, H: 6.07 %, N: 4.54 %.

12. **Herstellung von *exo*-[2',2",3',3"-η-(3'-(3"-(*E*)-Acrylsäureethylester-3"-yl)-2',3'didehydro-2',3'-dideoxy-5'-*O*-(dimethyl-thexyl-silyl)-β-D-cytidin)]-tricarbonyl-eisen (6)**

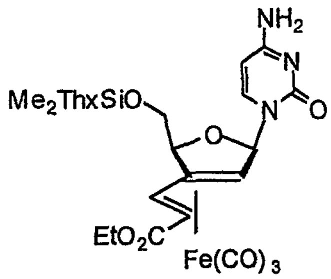

**6**

In einem 100 mL Dreihalskolben mit Rückflußkühler, Magnetrührkern und Absaughahn wurden unter einer Argonatmosphäre 0.570 g (1.12 mmol) des Komplexes 28 und 0.672 mg (2.63 mmol) N,O-Bistrimethylsilylcytosin in 40 ml abs. Dichlormethan gelöst und zum Rückfluß erhitzt. Nun wurde über einen Zeitraum von vier Stunden eine Lösung von 1.00 ml TMSOTf (1.24 g, 5.6 mmol) in 8 ml abs. Dichormethan zudosiert und die Reaktionsmischung anschließend für eine weitere Stunde unter Rückfluß erhitzt. Nach dem Abkühlen wurde der Kolbeninhalt auf Eis gegossen und mit gesättigter $NaHCO_3$-Lösung versetzt. Nach Trennung der Phasen wurde die organische Phase mit Wasser gewaschen, und die wäßrigen Phasen wurden je zweimal mit Dichlormethan reextrahiert. Die vereinigten organischen Phasen wurden über Natriumsulfat getrocknet. Nach Abfiltrieren des Trockenmittels wurde das Lösungsmittel am Rotationsverdampfer unter Argonatmosphäre entfernt. Der Rückstand wurde unter Argonatmosphäre an entgastem Kieselgel einer Chromatographie mit einem entgasten Lösungsmittelgemisch von Essigester:Methanol = 8:1 unterzogen. Das Produkt 6 wurde mit einer Ausbeute von 171 mg (0.29 mmol, 26%) als erstarrter, gelblich weißer Schaum erhalten.- **DC**: Essigester:Methanol = 30:1, $R_f$= 0.094. - **$^1$H-NMR:** (300 MHz, $CDCl_3$): δ = 0.12 (s, 3H, $Si(CH_3)$), 0.14 (s, 3H, $Si(CH_3)$), 0.68 (d, 1H, 2"-H, $^3J_{2"-H, 3"-H}$ = 7.9 Hz), 0.82 (s, 3H, $SiC(CH_3)$), 0.83 (s, 3H, $SiC(CH_3)$), 0.84 (_d, 6H, $C(C\underline{H}_3)_2H$, $^3J_{C(CH3)2H, C(CH3)2H}$ = 6.9 Hz), 1.22 (ψt, 3H, $C\underline{H}_3CH_2$, $^3J_{CH3CH2, CH3CH2}$ = 7.2 Hz), 1.59 (ψsept, 1H, $C(CH_3)_2\underline{H}$, $^{3J}C(C\underline{H}3)2\overline{H}$, $C(CH3)2\underline{H}$ = 6.9 Hz), 2.32 (s, 1H, 2'-H), 3.96 (ψd, 2H, 5'-H, $^3J_{4'-H, 5'-H}$ = 4.3 Hz), 4.06 (m, 1H, $CH_3C\underline{H}aHb$), 4.14 (m, 1H, $CH_3CHa\underline{H}b$), 4.95 (ψt, 1H, 4'-H, $^3J_{4'-H, 5'-H}$ = 4.3 Hz), 5.64 (d, 1H, 5-H, $^3J_{5-H, 6-H}$ = 7.4 Hz), 6.00 (s, sehr breit, 2H, $NH_2$), 6.04 (d, 1H, 3"-H, $^3J_{2"-H, 3"-H}$ = 7.9 Hz), 6.06 (s, 1H, 1'-H), 7.83 (d, 1H, 6-H, $^3J_{5-H, 6-H}$ = 7.4 Hz) - **$^{13}$C-NMR:** (70 MHz, $CDCl_3$): -3.4 und -3.2 ($Si(CH_3)_3$), 14.1 ($C\underline{H}_3CH_2$), 18.5 und 18.6 ($C(\underline{C}H_3)_2H$), 20.2 und 20.3 ($SiC(\underline{C}H_3)_2$), 25.4 ($Si\underline{C}(CH_3)_2$), 34.0 ($\underline{C}(CH_3)_2H$), 44.6 (2"-C), 60.4 (2'-C), 60.7 ($CH_3\underline{C}H_2$), 66.2 (5'-C), 76.3 (3"-C), 85.1 (4'-C), 91.8 (1'-C), 93.6 (5-C), 107.9 (3'-C), 141.3 (6-C), 155.8 (2-C), 156.7 (4-C), 171.8 (1"-C).

**13. Herstellung von *exo*-[1",2",2',3'-η-(2',3'-Didehydro-2',3'-dideoxy-3'-*s*-*cis*-vinyl-β-*D*uridin)]-tricarbonyl-eisen (33)**

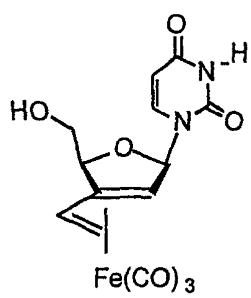

**33**

In einem 100 mL Schlenkkolben mit aufgesetztem Hg-Bubbler wurden unter Schutzgas 0.2515 g (0.48 mmol) Komplex **29** in 50 mL abs. THF vorgelegt und mit einem Eisbad auf 0°C gekühlt. Zu der magnetisch gerührten Lösung injizierte man unter Argon-Gegenstrom 2.5 mL (2.5 mmol) TBAF-Lösung (1 M in THF) und ließ noch 2.5 h reagieren, wonach DC-Reaktionskontrolle (Bed. s.u.) einen vollständigen Umsatz anzeigte. Zur Aufarbeitung wurde die Lösung mit 100 mL Essigester in einen Scheidetrichter überführt und mit 100 mL $H_2O$ versetzt. Nach der Phasentrennung wusch man die organische Phase mit 100 mL ges. wäßr. NaCl-Lösung, extrahierte die wäßrigen Phasen mit 100 mL Essigester und trocknete die vereinigten organischen Phasen mit $Na_2SO_4$. Zur Vermeidung einer möglichen oxidativen Dekomplexierung entgaste man die Lösung sofort durch kurzzeitiges Anlegen eines leichten Ölpumpenvakuums und belüftete anschließend wieder mit Argon. Nach Filtration wurde im Vak. am Rotationsverdampfer eingeengt und kurz im Ölpumpenvakuum getrocknet. Anschließend wurde der gelbe Rückstand unter striktem Sauerstoffausschluß in eine Flash-Säule überführt und mit Argondruck chromatographiert (90 g entgastes Kieselgel, *n*-Hexan / Aceton = 9+11). Man erhielt nach Trocknen im Hochvakuum 0.1812 g (99 %) des desilylierten Komplexes 33 in Form eines gelben Feststoffs, der anschließend bei - 20°C aus $CH_2Cl_2$ umkristallisiert wurde. - **Schmp. :** 118 - 120°C ($CH_2Cl_2$), gelber Feststoff. - **DC :** *n*-Hexan / Aceton = 1+1, $R_f$=0.23. - **CD :** $\Theta$ ($\lambda$) = - 5451 (274.0 nm), - 20501 (306.0 nm), (c = 0.006 in MeOH). **UV (MeOH) :** $\lambda_{max}(\varepsilon)$ = 258.5 nm (13326), $\lambda$ ($\varepsilon$) = 300.0 nm (sh, 2384). - **FT-IR (KBr) :** $\tilde{\nu}$ = 3394, 3198 (m,w, jeweils br., N-H), 3060 (w, unges. C-H), 2934 (w, ges. C-H), 2057, 1976 (s, s und br., C≡O), 1707, 1683 (jeweils s und br., C=O), 1471, 1258, 1096, 966 (jeweils m). - **$^1$H-NMR :** (270 MHz, Aceton-d$^6$) : $\delta$ = 0.32 (dd, $^2J_{2''anti,2''syn}$ = 2.0 Hz, $^3J_{2''anti,1''}$ = 8.8 Hz, 1H, 2''-$H_{anti}$), 1.91 (dd, $^2J_{2''syn,2''anti}$ = 2.0 Hz, $^3J_{2''syn,1''}$ = 6.7 Hz, 1H, 2''-$H_{syn}$), 2.32 (s, 1H, 2'-H), 3.94 ($\psi$dd, $^3J_{5'a,4'}$ = 4.3 Hz, $^2J_{5'a,5'b}$ = 11.9 Hz, 1H, 5'-Ha), 4.06 ($\psi$dd, $^3J_{5'b,4'}$ = 4.1 Hz, $^2J_{5'b,5'a}$ = 11.9 Hz, 1H, 5'-Hb), 4.57 ($\psi$t, J = 4.9 Hz, nicht integrierbar da z.T. bereits ausgetauscht, $D_2O$ austauschbar, O-H), 5.03 ($\psi$t J = 3.8 Hz, 1H, 4'-H), 5.54 (d, J = 8.1 Hz, 1H, 5-H), 6.01 ($\psi$t, J = 7.9 Hz, 1H, 1''-H), 6.31 (s, 1H, 1'-H), 8.05 (d, J = 8.1 Hz, 1H, 6-H), 10.0 (breites s, nicht integrierbar da z.T. bereits ausgetauscht, $D_2O$ austauschbar, N-H). - **MS** : (MAT 8222, EI, 70 eV): m/z (%) = 376 (1) [$M^+$], 348 (1) [$M^+$- CO], 320 (1) [$M^+$-2xCO], 292 (1) [$M^+$- 3xCO], 262 (35) [$M^+$- 3xCO, -$CH_2OH$, + H], 236 (10) [$M^+$-Ura., - CO, - H], 208 (18) [$M^+$- Ura., - 2xCO, - H], 180 (40) [$M^+$- Ura., - 3xCO, - H]. - **HRMS :** (MAT 8222, 70 eV): ber.: 375.9994; gef.: 375.9977; $C_{14}H_{12}FeN_2O_7$ (376.11).

**14. Herstellung von 2',3'-Didehydro-2',3'-dideoxy-3'-vinyl-β-*D*-uridin (34)**

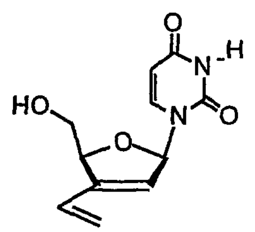

**34**

In einem 100 mL Kolben wurden 0.1719 g (0.46 mmol) Komplex 33 in 30 mL EtOH gelöst und mit 20 mL $H_2O$ versetzt. Zu der magnetisch gerührten und auf 0°C gekühlten Lösung gab man 2.10 g (3.8 mmol) $Ce(NH_4)_2(NO_3)_6$ und ließ 45 min reagieren (sofortige CO - Entwicklung). Zur Aufarbeitung überführte man die Reaktionslösung mit 80 mL Essigester in einen Scheidetrichter und versetzte mit 100 mL $H_2O$. Nach der Phasentrennung wusch man die organische Phase mit 100 mL ges. wäßr. NaCl-Lösung, extrahierte die wäßrigen Phasen mit 2 x 80 mL Essigester und trocknete die vereinigten organischen Phasen mit $Na_2SO_4$. Nach Filtration vom Trockenmittel wurde im Vak. am Rotationsverdampfer eingeengt und anschließend im Ölpumpenvakuum getrocknet. Man erhielt 0.056 g (52 %) des Produktes **34** als farblosen Feststoff in reiner Form. - **Schmp.:** 151°C (Essigester, Zersetzung). - **FT-IR (KBr)**: $\tilde{\nu}$ = 3442 (m und br., O-H, N-H), 3094 (w, unges. C-H), 2927 (w, ges. C-H), 1704 (m, C=O), 1467, 1396, 1249 (jeweilsm), 1127 (s, C-O). - **$^1$H-NMR:** (270 MHz, $CD_3OD$) : $\delta$ = 3.79 ($\psi$d, J = 12.5 Hz, 1H, 5'-Ha), 3.88 ($\psi$d, J = 12.2 Hz, 1H, 5'-Hb), 5.00 (s, 1H, 4'-H), 5.36 (d, J = 11.1 Hz, 1H, 2''-H(*E*)), 5.47 (d, J= 17.9 Hz, 1H, 2''-H(*Z*)), 5.60 (d, J = 7.8 Hz, 1H, 5-H), 5.83 (s, 1H, 2'-H), 6.55 (dd, $^3J_{1'',2''}$ = 11.3 Hz, $^3J_{1'',2''(Z)}$ = 17.9 Hz, 1H, 1''-H), 6.84 (s, 1H, 1'-H), 7.91 (d, J = 7.7 Hz, 1H, 6-H). $C_{11}H_{12}N_2O_4$ (236.23).

**15. Herstellung von (5*RS*,8*RS*)-8-Allyloxy-2-trimethylsilyl-7-oxa-bicyclo-[3.3.0]-oct-1-en-3-on (*rac*-38).**

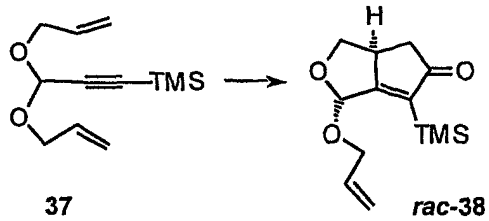

37 *rac*-**38**

Zu einer gerührten Mischung von Octacarbonyldicobalt (25 g, 69 mmol) und 4Å-Molekularsieb (108 g) in trockenem, entgasten $CH_2Cl_2$ (1.5 l) gab man unter Argon bei Raumtemp. (13.52 g, 60 mmol) 3,3-Diallyloxy-1-propinyltrimethylsilan (37). Nach 2 h Rühren bei Raumtemp. kühlte man die Reaktionsmischung auf < 0°C ab (Eis-NaCl-Bad) und versetzte sie mit azeotrop getrocknetem Trimethylamin-N-oxid (42 g, 560 mmol). Nach der Zugabe ließ man den Kolben offen und leitete für 5-10 min. Luft durch die Reaktionslösung. Anschließend ließ man auf Raumtemp. erwärmen und rührte noch 15 h an der Luft. Die durch etwas Kieselgel filtrierte Reaktionsmischung wurde im Vakuum eingeengt und der Rückstand durch Flashchromatographie (EtOAc/CyHex = 1+4) gereinigt. Man erhielt *rac*-**38** als blassgelbes Öl in einer Ausbeute von 11.56 g (76%). (Diastereoselektivität > 24:1).
**$^1$H-NMR** (250 MHz, $CDCl_3$): δ= 5.93 (tdd, $J_1$ = 5.4, $J_2$= 17.2, $J_3$ = 10.3, 1H, C<u>H</u>=$CH_2$); 5.58 (s, 1H, H-8); 5.31 (tdd, $J_1$ = 1.6, $J_2$ = 17.2, $J_3$ = 1.6, 1H, CH=CH-$\underline{H}_{trans}$); 5.22 (tdd, $J_1$ = 1.2, $J_2$ = 10.3, $J_3$ = 1.8, 1H, CH=CH-$\underline{H}_{cis}$); 4.38 (dd, $J_1$ = 6.6, $J_2$ = 7.2, 1H, H-6); 4.29 (tdd, $J_1$ = 1.4, $J_2$ = 12.2, $J_3$ = 5.6, 1H, $C\underline{H_2}$-CH=$CH_2$); 4.10 (tdd, $J_2$ = 1.3, $J_3$ = 12.5, $J_4$ = 6.4, 1H, $C\underline{H_2}$-CH=$CH_2$); 3.46 (m, 1H, H-5); 3.42 (dd, $J_1$ = 6.7, $J_2$ = 6.2, 1H, H-6); 2.66 (dd, $J_1$ = 17.6, $J_2$ = 6.4, 1H, H-4); 2.10 (dd, $J_1$ = 17.6, $J_2$ = 3.6, 1H, H-4); 0.21 (s, 9H, $SiCH_3$); **$^{13}$C-NMR** (63 MHz, $CDCl_3$): δ = 213.4 (C3), 184.9 (C1), 137.6 (C2), 133.8 ($\underline{C}H=CH_2$), 118.2 ($CH=\underline{C}H_2$), 96.7 (C8), 71.0 (C6), 68.8 ($\underline{C}H_2CH=CH_2$), 42.6 (C5), 41.8 (C4), -1.5 (SiC); **FT-IR** (ATR): 2954 (m, CH), 2896 (m, CH), 1703 (s, C=O), 1640 (s, C=C), 1410 (m), 1247 (s, C-O), 1126 (s), 1073 (s), 997 (s), 838 (s), 762 (s); **MS** (EI, 70 eV): m/z (%): 253 (1) $[M+1]^+$, 237 (2), 211 (15), 195 (20), 181 (21), 167 (8), 151 (20), 137 (7), 123 (32), 109 (9), 93 (17), 75 (44), 73 (100), 59 (11), 41 (21); **HRMS (EI)** $C_{12}H_{17}O_3Si$: ber. 237.095 $[M\text{-}15]^+$; gef. 237.095.

**16. Herstellung von (*3RS*,*5RS*,*8RS*)-8-Allyloxy-2-trimethylsilyl-7-oxa-bicyclo-[3.3.0]-oct-1-en-3-ol (*rac*-39).**

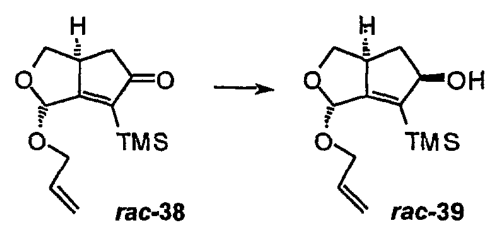

*rac*-**38** *rac*-**39**

Zu einer eisgekühlten Lösung (ca. 0°C) von *rac*-**38** (2.52 g, 10 mmol) und $CeCl_3$-$7H_2O$ (3.54 g, 9.5 mmol) in MeOH (200ml) gab man $NaBH_4$ (1.48 g, 38 mmol) in kleinen Portionen, ließ auf Raumtemp. erwärmen und rührte noch 0.5 h bevor man die Reaktion durch Zugabe von Wasser (100 ml) quenchte. Nach 1 h verteilte man zwischen MTBE (100 ml) und 10%-$NaHCO_3$ (aq.) (100 ml) und extrahierte die wässrige Phase nochmals mit MTBE (3x100 ml). Die vereinigten organischen Phasen wusch man mit 10%-$NaHCO_3$ (aq.) (100 ml) und ges. NaCl (aq.) (100 ml), trocknete mit $MgSO_4$, filtrierte und entfernte das Lösungsmittel im Vakuum. Man erhielt *rac*-**39** als farbloses Öl (2.46 g, 98%), das laut NMR keiner weiterer Reinigung bedurfte und direkt weiter umgesetzt wurde.
**$^1$H-NMR** (250 MHz, $CDCl_3$): δ= 5.90 (tdd, $J_1$ = 5.4, $J_2$ = 17.2, $J_3$ = 10.3, 1H, C<u>H</u>=$CH_2$); 5.38 (s, 1H, H-8); 5.26 (tdd, $J_1$ = 1.3, $J_2$ = 17.2, $J_3$ = 1.5, 1H, CH=CH-$\underline{H}_{trans}$); 5.17 + 5.16 (m + tdd, $J_1$ = 1.1, $J_2$ = 10.3, $J_3$ = 1.8, 2H, H-3 + CH=CH-$\underline{H}_{cis}$); 4.20 (dd, $J_1$ = $J_2$ = 8.2, 1H, H-6); 4.19 (tdd, $J_1$ = 1.4, $J_2$ = 12.4, $J_3$ = 5.3, 1H, $C\underline{H_2}$-CH=$CH_2$); 4.00 (tdd, $J_1$ = 1.3, $J_2$ = 12.4, $J_3$ = 6.4, 1H, $C\underline{H_2}$-CH=$CH_2$); 3.42 (dd, $J_1$ = $J_2$ = 7.9, 1H, H-6); 3.20 (m, 1H, H-5); 2.69 (ddd, $J_1$ = 12.1, $J_2$ = 6.5, $J_3$ = 6.0, 1H, H-4); 1.64 (bs, 1H, OH); 1.22 (ddd, $J_1$ = 12.0, $J_2$ = $J_3$ = 8.6 Hz, 1H, H-4); 0.21 (s, 9H, $SiCH_3$); **$^{13}$C-NMR** (63 MHz, $CDCl_3$): δ= 156.8 (C1), 140.3 (C2), 134.3 ($\underline{C}H=CH_2$), 117.7 ($CH=\underline{C}H_2$), 97.3 (C8), 87.61 (C3), 72.1 (C6), 68.3

(<u>C</u>$H_2$CH=$CH_2$), 46.4 (C5), 43.9 (C4), - 0.6 (SiC); **FT-R** (ATR): 3437 (m, OH), 2952 (s, CH), 2886 (m, CH), 1657 (m, C=C), 1408 (m), 1324 (s), 1245 (s, C-O), 1107 (s), 1064 (s), 989 (s), 834 (s), 753 (s); **MS** (EI, 70 eV): m/z (%) = 253 (1) [*M*-1]$^+$, 210 (3), 197 (22), 183 (20), 167 (17), 151 (19), 135 (11), 123 (20), 115 (7), 107 (18), 95 (16), 75 (46), 73 (100), 59 (10), 41 (21); **HRMS (EI)** $C_{12}H_{19}O_3Si$: ber. 239.114 [M-15]$^+$; gef. 239.110.

**17. Herstellung von (*3RS,5RS,8RS*)-8-Allyloxy-7-oxa-bicyclo-[3.3.0]-oct-1-en-3-ol (*rac-39'*)**

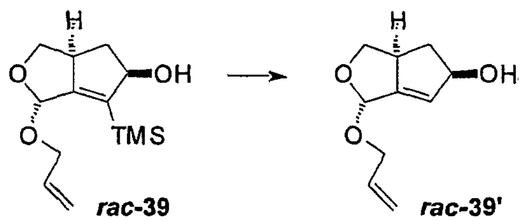

*rac*-**39** *rac*-**39'**

Zu einer gerührten Mischung von *t*-BuOK (1.14 g) in DMSO/$H_2O$ (1:19 v/v; ca. 20 ml) gab man bei Raumtemp. *rac*-**39** (2.54 g, 10 mmol). Die nun braune Reaktionsmischung erhitzte man für 2 h unter Rückfluß. Nach Abkühlen auf Raumtemp. versetzte man mit Wasser (100 ml) und extrahierte mit Essigester (4x80 ml). Die vereinigten organischen Phasen wusch man mit Wasser (2x100 ml) und ges. NaCl-Lsg. (100 ml), trocknete mit $MgSO_4$ und engte die filtrierte Lösung im Vakuum ein. Das als blassgelbes Öl erhaltene Produkt (*rac*-**39'**) (1.59 g, 87%). erwies sich als ausreichend rein, um direkt weiter umgesetzt zu werden. Eine analytische Probe wurde dennoch durch Flashchromatographie (EtOAc/CyHex = 1+9) aufgereinigt.
**$^1$H-NMR** (250 MHz, $CDCl_3$): δ= 5.85 (tdd, $J_1$ = 5.4, $J_2$ = 17.2, $J_3$ = 10.3, 1H, C<u>H</u>=$CH_2$); 5.70 (m, 1H, H-2); 5.36 (s, 1H, H-8); 5.22 (tdd, $J_1$ = 1.2, $J_2$ = 17.2, $J_3$ = 1.6, 1H, CH=CH-<u>H</u>$_{trans}$); 5.13 + 5.12 (m + tdd, $J_1$ = 1.2, $J_2$ = 10.3, $J_3$ = 1.6, 2H, H-3 + CH=CH-<u>H</u>$_{cis}$); 4.18 (dd, $J_1$ = $J_2$ = 8.2, 1H, H-6); 4.12 (tdd, $J_1$ = 1.6, $J_2$ = 12.6, $J_3$ = 5.3, 1H, C<u>H</u>$_2$-CH=$CH_2$); 3.96 (tdd, $J_1$ = 1.6, $J_2$ = 12.6, $J_3$ = 6.1, 1H, C<u>H</u>$_2$-CH=$CH_2$); 3.38 (dd, $J_1$ = $J_2$ = 7.1, 1H, H-6); 3.20 (m, 1H, H-5); 2.66 (ddd, $J_1$ = 12.4, $J_2$ = 6.3, $J_3$ = 6.1, 1H, H-4); 2.11 (bs, 1H, OH); 1.29 (ddd, $J_1$ = 12.3, $J_2$ = $J_3$ = 8.0, 1H, H-4); **$^{13}$C-NMR** (63 MHz, $CDCl_3$): δ= 147.9 (C1), 134.0 (<u>C</u>H=$CH_2$), 126.8 (C2), 117.3 (CH=<u>C</u>$H_2$), 96.8 (C8), 82.2 (C3), 72.7 (C6), 67.8 (<u>C</u>$H_2$CH=$CH_2$), 44.9 (C5), 42.9 (C4); **FT-IR** (ATR): 3396 (w, OH), 2920 (s, C-H), 2851 (m, C-H), 1646 (w, C=C), 1458 (m), 1326 (m), 1295 (m, C-O), 1149 (m), 1033 (s), 985 (s), 823 (s), 775 (m); **MS** (EI, 70 eV): m/z (%): 183 (4) [*M*+1]$^+$, 152 (20), 125 (13), 110 (40), 95 (82), 83 (64), 69 (60), 66 (98), 55 (100).

**18. Herstellung von (*3RS,5RS,8RS*)-3-Acetozy-8-allyloxy-7-oxa-bicyclo-[3.3.0]-oct-1-en (*rac*-40).**

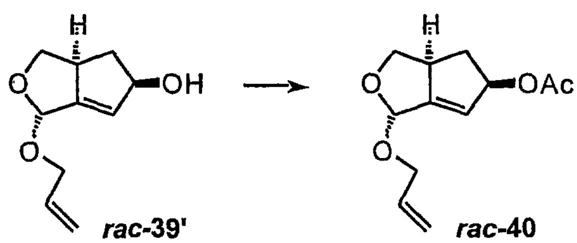

*rac*-**39'** *rac*-**40**

Zu einer unter Argon gerührten Mischung von *rac*-**39'** (2.38 g, 13 mmol), $Et_3N$ (2.3 ml, 15.6 mmol) und DMAP (196 mg, 1.56 mmol) in $CH_2Cl_2$ (50 ml) gab man bei Raumtemp. Essigsäureanhydrid (2.7 ml, 28.6 mmol). Nach 3 h Rühren versetzte man die Reaktionsmischung mit ges. $NaHCO_3$-Lsg. (aq.) (50 ml) und extrahierte die Wasserphase mit $CH_2Cl_2$ (3x80 ml). Die organischen Phasen wusch man mit ges. Lösungen von $NaHCO_3$ (aq.) (2x100 ml) und NaCl (100 ml), trocknete mit $MgSO_4$ und entfernte das Lösungsmittel im Vakuum. Den braunen Rückstand reinigte man durch Flashchromatographie (EtOAc/CyHex = 1+4). Man erhielt *rac*-**40** als farbloses Öl (2.89 g, 99%).
**$^1$H-NMR** (250 MHz, $CDCl_3$): δ = 5.95 (m, 1H, H-3); 5.87 (tdd, $J_1$ = 5.4, $J_2$ = 17.2, $J_3$ = 10.3, 1H, C<u>H</u>=$CH_2$); 5.73 (m,

1H, H-2); 5.41 (s, 1H, H-8); 5.26 (tdd, $J_1$ = 1.2, $J_2$ = 17.2, $J_3$ = 1.6, 1H, CH=CH-$\underline{H}_{trans}$); 5.17 (tdd, $J_1$ = 1.3, $J_2$ = 10.3, $J_3$ = 1.6, 1H, CH=CH-$\underline{H}_{cis}$); 4.25 (dd, $J_1$ = $J_2$ = 8.0, 1H, H-6); 4.18 (tdd, $J$1= 1.6; $J_2$ = 12.4, $J_3$ = 5.4, 1H, $C\underline{H}_2$-CH=$CH_2$); 3.96 (tdd, $J_1$ = 1.4, $J_2$ = 12.4, $J_3$ = 6.1, 1H, $C\underline{H}_2$-CH=$CH_2$); 3.44 (dd, $J_1$ = $J_2$ = 7.91, 1H, H-6); 3.32 (m, 1H, H-5); 2.77 (ddd, $J_1$ = 12.7, $J_2$ = $J_3$ = 6.8, 1H, H-4); 2.03 (s, 1H, $C\underline{H}_3$COO); 1.50 (ddd, $J_1$ = 12.7, $J_2$ = $J_3$ = 7.7, 1H, H-4); **$^{13}$C-NMR** (63 MHz, $CDCl_3$): δ = 170.0 (C=O), 150.2 (C1), 134.2 ($\underline{C}$H=$CH_2$), 122.6 (C2), 117.4 (CH=$\underline{C}H_2$), 96.8 (C8), 84.1 (C3), 72.6 (C6), 68.0 ($\underline{C}H_2$CH=$CH_2$), 45.0 (C5), 39.0 (C4), 21.1 ($\underline{C}H_3$COO); **FT-IR** (ATR): 2973 (m, C-H), 2932 (m, C-H), 2887(m, C-H), 1732 (s, C=O), 1444 (w), 1426 (w), 1362 (s), 1295 (m, C-O), 1232 (s, C-O), 1152 (s), 1065 (s), 1023 (s), 991 (s), 891 (s), 826 (m), 782 (w); **MS** (EI, 70 eV): m/z (%) = 225 (2) $[M+H]^+$, 182 (5), 167 (42), 150 (8), 142 (13), 134 (27), 124 (32), 111 (25), 95 (52), 94 (100), 93 (26), 83 (18), 79 (38), 67 (45), 66 (74), 57 (12), 55 (42), 53 (18).

**19. Allgemeine Vorschrift zur Pd-katalysierten Einführung von Pyrimidin-Nucleobasen**

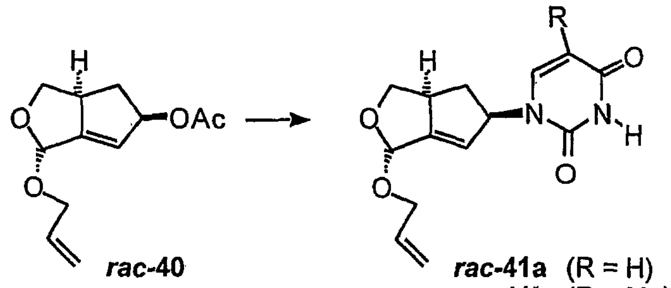

***rac*-40**

***rac*-41a** (R = H)
***rac*-41b** (R = Me)
***rac*-41c** (R = Br)

Eine gerührte Suspension einer Pyrimidin-Nucleobase (1.65 mmol) und NaH (60 mg, ~ 60% Dispersion in Mineralöl) in trockenem, entgastem DMSO (10 ml) wird unter Argon für 30 min. auf 70°C erhitzt, wobei eine annähernd klare Lösung entsteht. Nach Abkühlung auf Raumtemp. werden $Pd(PPh_3)_4$ (58 mg, ca. 5 mol%), $PPh_3$ (29 mg, 11 mol %) sowie eine Lösung des Allylacetates *rac*-**40** (224 mg, 1 mmol) in trockenem THF (2 ml) zugegeben. Anschließend wird unter Argon für 15 h auf 70°C erhitzt. Die auf Raumtemp. abgekühlte Mischung wird dann durch etwas Kieselgel filtriert und mit $CH_2Cl_2$ (20 ml) verdünnt. Nach dem Waschen mit ges. wäßrig. NaCl (4x20 ml) wird mit $MgSO_4$ getrocknet und das Lösungsmittel im Vakuum entfernt. Das Rohprodukt wird durch Flashchromatographie (EtOAc/CyHex = 1:9 bis 2:1) gereinigt.

**20. Herstellung von (*1RS,3aRS,5RS*)-1-(1-Allyloxy-3,3a,4,5-tetrahydro-1*H*cyclopenta[c]furan-5-yl)-1*H*-pyrimidine-2,4-dion (*rac*-41a).**

Ein Experiment im 1 mmol-Maßstab lieferte *rac*-**41a** als weiß-gelben Feststoff (188 mg, 66%). Die Substanz enthielt geringe Verunreinigungen (<3%) an $Ph_3P$=O.
**$^1$H-NMR** (250 MHz, $CDCl_3$): δ= 10.09 (bs, 1H, NH); 7.17 (d, $J$= 8.0, 1H, H-6'); 6.04 (dd, $J_1$ = 7.2, $J_2$ = 8.7, 1H, H-3); 5.86 (tdd, $J_1$ = 5.4, $J_2$ = 17.2, $J_3$ = 10.4, 1H, $C\underline{H}$=$CH_2$); 5.70 (d, $J$= 8.0, 1H, H-5'); 5.57 (m, 1H, H-2); 5.43 (s, 1H, H-8); 5.23 (tdd, $J_1$ = 1.2, $J_2$ = 17.2, $J_3$ = 1.6, 1H, CH=CH-$\underline{H}_{trans}$); 5.13 (tdd, $J_1$ = 1.1, $J_2$ = 10.3, $J_3$ = 1.6, 1H, CH=CH-$\underline{H}_{cis}$); 4.24 (dd, $J_1$ = $J_2$ = 7.4, 1H, H-6); 4.05 (tdd, $J_1$ = 1.6, $J_2$ = 12.7, $J_3$ = 5.4, 1H, $C\underline{H}_2$-CH=$CH_2$); 4.04 (tdd, $J_1$ = 1.3, $J_2$ = 12.6, $J_3$ = 6.1, 1H, $C\underline{H}_2$-CH=$CH_2$); 3.47 (dd, $J_1$ = $J_2$ = 7.6, 1H, H-6); 3.44 (m, 1H, H-5); 2.84 (ddd, $J_1$ = 12.5, $J_2$ = 7.0, $J_3$ = 6.8, 1H, H-4); 1.37 (ddd, $J_1$ = 12.5, $J_2$ = 8.3, $J_3$ = 7.6, 1H, H-4); **$^{13}$C-NMR** (63 MHz, $CDCl_3$): δ = 163.5 (C4'), 152.4 (C1), 150.8 (C2'), 140.4 (C6'), 133.8 ($\underline{C}$H=$CH_2$), 120.6 (C2), 117.5 (CH=$\underline{C}$H2), 103.0 (C5'), 96.2 (C8), 71.8 (C6), 67.9 ($\underline{C}H_2$CH=$CH_2$), 65.5 (C3), 45.4 (C5), 40.4 (C4); **FT-IR** (ATR): 3180 (w, N-H), 3051 (w, N-H), 2970 (w, C-H), 2886 (w, C-H), 1681 (m, C=O), 1626 (w, C=C), 1456 (m), 1375 (m), 1296 (w), 1242 (m, C-O), 1060 (m), 989 (m), 812 (w), 760 (w); **MS** (EI, 70 eV): m/z (%): 277 (8) $[M+1]^+$, 276 (5) $[M]^+$, 247 (12), 235 (9), 219 (78), 205 (100), 189 (6), 177 (7), 164 (13), 162 (38), 134 (52), 119 (37), 113 (23), 91 (13), 79 (57), 67 (15), 53 (11).

**21. Herstellung von (*1RS*,*3aRS*,*5RS*)-1-(1-Allyloxy-3,3a,4,5-tetrahydro-1*H*cyclopenta[c]furan-5-yl)-5-methyl-1*H*-pyrimidine-2,4-dion (*rac*-41b).**

Ein Experiment im 2 mmol-Maßstab lieferte *rac*-**41b** in 63 % Ausbeute.

Schmp. = 164-166°C (EtOAc/Hex); **$^1$H-NMR** (250 MHz, $CDCl_3$): δ = 8.63 (bs, 1H, NH); 6.96 (dq, *J*1= 1.3, $J_2$ = 1.0, 111, H-6'); 6.04 (dddd, $J_1$ = 2.2, $J_2$ = 1.3, $J_3$ = 6.8, $J_4$ = 8.5, 1H, H-3); 5.91 (tdd, $J_1$ = 5.4, $J_2$ = 17.2, $J_3$ = 10.3, 1H, C<u>H</u>=$CH_2$); 5.60 (m, 1H, H-2); 5.50 (s, 1H, H-8); 5.29 (tdd, $J_1$ = 1.2, $J_2$ = 17.2, $J_3$ = 1.6, 1H, CH=CH-$\underline{H}_{trans}$); 5.20 (tdd, $J_1$ = 1.3, $J_2$ = 10.3, $J_3$ = 1.7, 1H, CH=CH-$\underline{H}_{cis}$); 4.30 (dd, $J_1$ = $J_2$ = 8.1, 1H, H-6); 4.20 (tdd, $J_1$ = 1.6, $J_2$ = 12.6, $J_3$ = 5.4, 1H, $C\underline{H_2}$-CH=$CH_2$); 4.04 (tdd, $J_1$ = 1.3, $J_2$ = 12.6, $J_3$ = 6.1, 1H, $C\underline{H_2}$-CH=$CH_2$); 3.53 (dd, $J_1$ = $J_2$ = 7.8, 1H, H-6); 3.45 (m, 1H, H-5); 2.86 (ddd, $J_1$ = 12.4, $J_2$ = 7.0, $J_3$ = 6.8, 1H, H-4); 1.91 (d, *J* = 1.0, 1H, $CH_3$); 1.42 (ddd, $J_1$ = 12.4, $J_2$ = $J_3$ = 7.7, 1H, H-4); **$^{13}$C-NMR** (63 MHz, $CDCl_3$): δ = 163.4 (C4'), 152.3 (C1), 150.5 (C2'), 136.1 (C6'), 134.0 (<u>C</u>H=CH2), 120.9 (C2), 117.7 (CH=$\underline{C}H_2$), 111.7 (C5'), 96.4 (C8), 72.01 (C6), 68.1 ($\underline{C}H_2$CH=$CH_2$), 65.4 (C3), 45.5 (C5), 40.5 (C4), 12.5 ($CH_3$); **FT-IR** (ATR): 3171 (w, N-H), 3052(w, N-H), 2923 (w, C-H), 2887 (w, C-H), 1682 (s, C=O), 1467 (m), 1364 (m), 1297 (m, C-O), 1246 (m, C-O), 1061 (m), 989 (m), 825 (m), 728 (w); MS (EI, 70 eV): m/z (%): 249 (1), 221 (2), 207 (20), 191 (5), 179 (4), 165 (7), 153 (8), 141 (6), 133 (10), 126 (17), 111 (14), 105 (18), 98 (21), 97 (42), 91 (23), 85 (17), 83 (35), 71 (23), 70 (28), 69 (48), 57 (62), 55 (100).

**22. Herstellung von (*1RS,3aRS,5RS*)-1-(1-Allyloxy-3,3a,4,5-tetrahydro-1*H*cyclopenta[c]furan-5-yl)-5-brom-1*H*-pyrimidine-2,4-dion (*rac*-41c).**

Ein Experiment im 2 mmol-Maßstab lieferte *rac*-**41c** in 83 % Ausbeute.

**Schmp.** 156-158°C; **$^1$H-NMR** (250 MHz, $CDCl_3$): δ= 8.81 (bs, 1H, NH); 7.47 (s, 1H, H-6'); 6.03 (m, 1H, H-3); 5.89 (tdd, $J_1$ = 5.4, $J_2$ = 17.1, $J_3$ = 10.2, 1H, C<u>H</u>=$CH_2$); 5.60 (m, 1H, H-2); 5.51 (s, 1H, H-8); 5.28 (tdd, $J_1$ = 1.2, $J_2$ = 17.1, $J_3$ = 1.5, 1H, CH=CH-$\underline{H}_{trans}$); 5.18 (tdd, $J_1$ = 1.2, $J_2$ = 10.2, $J_3$ = 1.5, 1H, CH=CH-$\underline{H}_{cis}$); 4.29 (dd, $J_1$ = $J_2$ = 7.8, 1H, H-6); 4.21 (tdd, $J_1$ = 1.5, $J_2$ = 12.7, $J_3$ = 5.4, 1H, $C\underline{H_2}$-CH=$CH_2$); 4.18 (tdd, $J_1$ = 1.3, $J_2$ = 12.7, $J_3$ = 5.4, 1H, $C\underline{H_2}$-CH=$CH_2$); 3.54 (dd, $J_1$ = 7.8, $J_2$ = 7.6, 1H, H-6); 3.45 (m, 1H, H-5); 2.90 (ddd, $J_1$ = 12.4, $J_2$ = $J_3$ = 6.8, 1H, H-4); 1.42 (ddd, $J_1$ = 12.4, $J_2$ = 8.3, $J_3$ = 8.8, 1H, H-4); **$^{13}$C-NMR** (63 MHz, $CDCl_3$): δ= 159.0 (C4'), 152.6 (C1), 150.0 (C2'), 140.0 (C6'), 133.7 (<u>C</u>H=$CH_2$), 120.2 (C2), 117.3 (CH=$\underline{C}H_2$), 97.1 (C5'), 96.1 (C8), 71.6 (C6), 67.7 ($\underline{C}H_2$CH=$CH_2$), 65.8 (C3), 45.3 (C5), 40.4 (C4); **FT-IR** (ATR): 3167 (w, N-H), 3046 (w, N-H), 2975 (w, C-H), 2885 (w, C-H), 2829 (w, CH), 1691(s, C=O), 1615 (m, C=C), 1441 (m), 1338 (w), 1296 (w, C-O), 1242 (m, C-O), 1061 (w), 990 (m), 820 (w), 750 (m); MS (EI, 70 eV): m/z (%): 356(4) [$^{81}$Br*M*]$^+$, 354 (4) [$^{79}$Br*M*]$^+$, 327 (5), 325 (6), 299 (40), 297 (41), 285 (13), 283 (12), 277 (12), 254 (7), 242 (9), 240 (12), 228 (4), 226 (7), 214 (10), 212 (10), 193 (13), 191 (14), 165 (11), 149 (5), 147 (8), 135 (55), 119 (51), 107 (29), 95 (14), 93 (21), 91 (23), 81 (31), 79 (100), 77 (34), 67 (17), 66 (18), 65 (18), 57 (4), 55 (8), 53 (13); **HRMS (EI)** $C_{14}H_{15}BrN_2O_5$: ber. 354.022 [$^{79}$Br*M*]$^+$; gef: 354.021.

**23. Herstellung von (*1RS,3aRS,5RS*)-9-(1-Allyloxy-3,3a,4,5-tetrahydro-1*H*cyclopenta[c]furan-5-yl)-9*H*-purin-6-ylamin (*rac*-41d).**

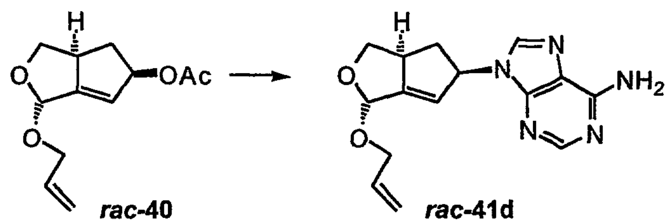

***rac*-40** ***rac*-41d**

Eine Suspension von Adenin (1.022 g, 7.5 mmol) und wasserfreiem $Cs_2CO_3$ (2.47 g, 7.5 mmol) in trockenem, entgastem DMSO (25 ml) wurde unter Argon bei 50°C für 45 min gerührt. Nach dem Abkühlen auf Raumtemp. wurde eine Lösung von *rac*-**40** (1.12 g, 5 mmol) in DMSO (5 ml) sowie $Ph_3P$ (145 mg, 11 mol%) und $Pd(PPh_3)_4$ (290 mg, 5 mol%) zugegeben, und die erhaltene Mischung bei 50°C für 15 h gerührt. Nach dem Abkühlen auf Raumtemp. wurde die Reaktionsmischung mit $CH_2Cl_2$ durch etwas Kieselgel filtriert. Das Filtrat wurde mit ges. NaCl-Lsg. (4x100 ml) gewaschen, mit $MgSO_4$ getrocknet und im Vakuum aufkonzentriert. Der Rückstand wurde durch Flashchromatographie (EtOAc/MeOH = 9+1) gereinigt. Man erhielt reines *rac*-**41d** als gelbes Wachs in 73% Ausbeute.

**$^1$H-NMR** (250 MHz, $CDCl_3$): δ= 8.29 (s, 1H, H-2'); 7.47 (s, 1H, H-8'); 6.42 (bs, 2H, N$H_2$); 6.04 (tdd, $J_1$ = 2.0, $J_2$ = 7.3, $J_3$ = 8.6, 1H, H-3); 5.90 (dddd, $J_1$ = 5.4, $J_2$ = 6.1, $J_3$ = 17.1, $J_4$ = 10.2, 1H, C<u>H</u>=$CH_2$); 5.83 (m, 1H, H-2); 5.51 (s, 1H, H-8); 5.26 (tdd, $J_1$ = 1.4, $J_2$ = 17.1, $J_3$ = 1.8, 1H, CH=CH-$\underline{H}_{trans}$); 5.17 (tdd, $J_1$ = 1.2, $J_2$ = 10.2, $J_3$ = 1.8, 1H, CH=CH-$\underline{H}_{cis}$); 4.29 (dd, $J_1$ = $J_2$ = 7.1, 1H, H-6); 4.19 (tdd, $J_1$ = 1.5, $J_2$ = 12.6, $J_3$ = 5.4, 1H, $C\underline{H_2}$-CH=$CH_2$); 4.03 (tdd, $J_1$ = 1.2, $J_2$ = 12.6, $J_3$ = 6.1, 1H, $C\underline{H_2}$-CH=$CH_2$); 3.56 (dd, $J_1$ = 7.1, $J_2$ = 7.5, 1H, H-6); 3.51 (m, 1H, H-5); 2.99 (ddd, $J_1$ = 12.4,

$J_2$ = 7.1, $J_3$ = 6.6, 1H, H-4); 1.75 (ddd, $J_1$= 12.4, $J_2$ = 8.6, $J_3$ = 9.1, 1H, H-4); **$^{13}$C-NMR** (63 MHz, $CDCl_3$): δ = 155.8 (C6'), 152.9 (C2'), 151.9 (C1), 149.5 (C4'), 138.2 (C8'), 133.9 (CH=$CH_2$), 121.3 (C2), 119.6 (C5'), 117.6 (CH=$CH_2$), 96.5 (C8), 72.0 (C6), 68.1 (CH2CH=$CH_2$), 64.1 (C3), 45.9 (C5), 41.9 (C4); **FT-IR** (ATR): 3315 (m, N-H), 3162 (m, N-H), 2970 (w, C-H), 2918 (w, C-H), 2887 (w, C-H), 1644 (s, C=C), 1596 (s), 1571 (m), 1471 (m), 1413 (m), 1327 (m), 1299 (m), 1247 (m, C-O), 1061 (m), 989 (m), 831 (w), 797 (w), 722 (m); **MS** (EI, 70 eV): m/z (%): 299 (3) [*M*]$^+$, 258 (61), 242 (57), 228 (9), 212 (16), 200 (15), 164 (63), 136 (100), 135 (63), 123 (17), 108 (21), 95 (18), 79 (39), 67 (18), 55 (18), **HRMS** (EI) $C_{15}H_{17}N_5O_2$: ber. 299.138 [*M*]$^+$, gef.: 299.139.

**25. Allgemeine Arbeitsvorschrift zur Hydrolyse und Schützung der Produkte vom Typ 41'.**

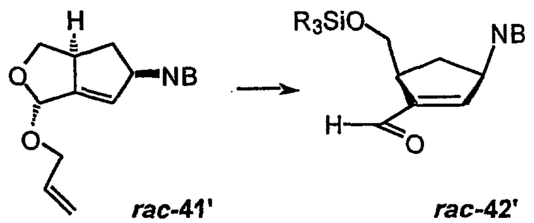

***rac*-41'** ***rac*-42'**

Eine Lösung von *rac*-**41'** (1 mmol) und $PyH^+TsO$- (76 mg, 0.3 mmol) in nassem Aceton (10 ml) wird für 3 h zum Rückfluß erhitzt. Danach wird das Lösungsmittel im Vakuum entfernt und der Kolben unter Argon gesetzt. Der Rückstand wird in trockenem Pyridin (3 ml) gelöst und mit einem Chlortrialkylsilan (1.5 mmol) versetzt. Nach 15 h Rühren bei Raumtemp. wird zwischen ges. $NaHCO_3$ (aq.) (20 ml) und EtOAc (10 ml) verteilt (30 min. Rühren bei Raumtemp.). Danach wird die Wasserphase mit EtOAc extrahiert (2x30 ml) und die vereinigten organischen Phasen mit ges. wäßrigen Lösungen von $NaHCO_3$ (40 ml) und NaCl (40 ml) gewaschen. Nach dem Trocknen ($MgSO_4$) wird im Vakuum eingeengt und das Rohprodukt (*rac*-**42'**) durch Flashchromatographie gereinigt.

**26. Herstellung von (3*RS*,5*RS*)-5-[Dimethyl-(1,1,2-trimethyl-propyl)-silanyloxymethyl]-3-(2,4-dioxo-3,4-dihydro-2*H*-pyrimidin-1-yl)-cyclopent-1-encarbaldehyd (*rac*-42a).**

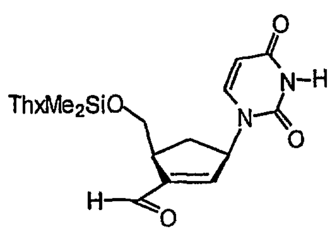

***rac*-42a**

Ein Experiment im 0.5 mmol-Maßstab lieferte *rac*-**42a** in 80 % Ausbeute als gelblich-weißen Feststoff, ausgehend von *rac*-**41a** und Thexyldimethylsilylchlorid (gemäß Vorschrift 25).
Schmp. : 191°C (dec.); **$^1$H-NMR** (250 MHz, $CDCl_3$): δ= 9.85 (s, 1H, HC=O), 8.78 (bs, 1H, NH), 7.47 (d, $J_1$ = 8.0, 1H, H-6'), 5.56 (dd, $J_1$= $J_2$= 2.2, 1H, H-2), 5.90 (dddd, $J_1$ = $J_2$ = 2.2, $J_3$ = 9.5, $J_4$ = 7.1, 1H, H-3), 5.71 (dd, $J_1$= 8.0, $J_2$ = 2.4, 1H, H-5'), 4.27 (dd, $J_1$ = 10.0, $J_2$ = 3.0, 1H, $SiOCH_a$), 3.58 (dd, $J_1$ = 10.0, $J_2$ = 2.5, 1H, $SiOCH_b$), 3.18 (m, 1H, H-5), 2.79 (ddd, $J_1$ = 14.2, $J_2$ = $J_3$ = 9.5, 1H, H-4), 1.82 (ddd, $J_1$ = 14.2, $J_2$ = $J_3$ = 7.5, 1H, H-4), 1.56 (septet, $J$ = 6.8, 1H, $Me_2CH$), 0.81 (d, $J$= 6.8 Hz, 6H, $(CH_3)_2CH$), 0.788 + 0.786 (2s, 6H, $C(CH_3)_2$), 0.042 + 0.036 (2s, 6H, $SiCH_3$); **$^{13}$C-NMR** (63 MHz, $CDCl_3$): δ = 188.9 (CH=0), 163.2 (C4'), 150.8 (C2'), 150.3 (C1), 147.8 (C2), 141.4 (C6'), 103.2 (C5'), 62.2 ($CH_2OSi$), 58.9 (C3), 44.3 (C5), 34.0 $Me_2CH$), 33.3 (C4), 25.4 ($Me_2CSi$), 20.3 and 20.2 ($(CH_3)_2CH$), 18.5 and 18.4 ($(CH_3)_2CSi$), -3.5 and -3.6 ($(CH_3)_2Si$); **FT-IR** (ATR): 3038 (w, N-H), 2955 (m, C-H), 2865 (w, C-H), 1697 (s, C=O), 1680 (s, C=O), 1649 (s, C=C), 1473 (w), 1445 (w), 1425 (w), 1383 (m), 1276 (m), 1248 (m, C-O), 1186 (m), 1111 (m), 1063 (w), 1019 (w), 998 (w), 979 (w), 849 (m), 828 (m), 804 (m), 779 (m), 761 (m); **MS** (EI, 70 eV): m/z (%): 295 (7), 294

(17), 293 (100) [*M*-85]$^+$, 275 (27), 250 (18), 201 (55), 181 (62), 169 (26), 151 (8), 137 (5), 99 (7), 91 (5), 89 (7), 85 (6), 77 (6), 75 (23), 73 (18), 69 (6), 59 (8), 57 (5); **HRMS** (EI) $C_{13}H_{17}N_2O_4Si$ [*M*-85]$^+$: ber. 293.096, gef.: 293.096.

**27. Herstellung von (3*RS*,5*RS*)-5-[Dimethyl-(1,1,2-trimethyl-propyl)-silanyloxymethyl]-3-(5-methyl-2,4-dioxo-3,4-dihydro-2*H*-pyrimidin-1-yl)-cyclopent-1-encarbaldehyd (*rac*-42b).**

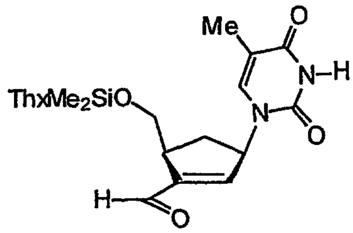

***rac*-42b**

Ein Experiment im 0.7 mmol-Maßstab lieferte *rac*-**42b** in 65 % Ausbeute als gelblichen Feststoff, ausgehend von *rac*-**41b** und Thexyldimethylsilylchlorid (gemäß Vorschrift 25).
**Schmp.:** 187°C; **$^1$H-NMR** (250 MHz, $CDCl_3$): δ = 10.06 (bs, 1H, NH), 9.81 (s, 1H, HC=O), 6.99 (q, *J*= 1.2, 1H, H-6'), 6.58 (m, 1H, H-2), 5.83 (dddd, $J_1$ = $J_2$ = 2.4, $J_3$ = $J_4$ = 8.6,1H, H-3), 4.18 (dd, $J_1$ = 10.0, $J_2$ = 3.4, 1H, SiOC$H_a$), 3.53 (dd, $J_1$ = 10.0, $J_2$ = 2.4, 1H, SiOC$H_b$), 3.10 (m, 1H, H-5), 2.65 (ddd, $J_1$ = 13.4, $J_2$ = $J_3$ = 8.8, 1H, H-4), 1.85 (d, J = 1.2, 3H, $CH_3$), 1.80 (ddd, $J_1$ = 13.4, $J_2$ = $J_3$ = 8.4, 1H, H-4), 1.51 (Sept., *J*= 7.1, 1H, $Me_2CH$), 0.83 (d, *J*= 7.1, 6H, $(CH_3)_2CH$], 0.793 + 0.790 (2s, 6H, $C(CH_3)_2$), 0.058 + 0.057 (2s, 6H, $SiCH_3$); **$^{13}$C-NMR** (63 MHz, $CDCl_3$): δ = 188.9 (CH=O), 164.1 (C4'), 151.1 (C2'), 149.8 (C1), 148.4 (C2), 136.2 (C6'), 111.8 (C5'), 61.4 ($CH_2OSi$), 59.0 (C3), 44.2 (C5), 34.0 ($Me_2CH$), 33.4 (C4), 25.1 ($Me_2CSi$), 20.2 and 20.2 (($CH_3$)2CH), 18.4 und 18.4 ($(CH_3)_2CSi$), 12.3 ($CH_3$), -3.5 und -3.7 ($SiCH_3$); **FT-IR** (ATR): 3177 (w, N-H), 3050 (w, N-H), 2954 (m, C-H), 2864 (m, C-H), 1683 (s, C=O), 1464 (m), 1376 (m), 1279 (m), 1249 (m, C-O), 1153 (w), 1113 (m), 1057 (w), 1017 (w), 982 (w), 874 (m), 830 (m), 778(m), 722 (w); **MS** (EI, 70 eV): m/z (%): 393 (4) [*M*+1]$^+$, 365 (13), 341 (5), 307 (100), 289 (27), 264 (10), 215 (52), 183 (31), 181 (73), 167 (12), 151 (7), 137 (5), 113 (4), 89 (7), 75 (15), 59 (4); **HRMS** (ESI) $C_{20}H_{33}N_2O_4Si$ [*M*+1]$^+$: ber. 393.2209, gef. 393.2209.

**28. Herstellung von (3*RS*,5*RS*)-3-(5-Brom-2,4-dioxo-3,4-dihydro-2*H*-pyrimidin-1-yl)-5-[dimethyl-(1,1,2-trimethyl-propyl)-silanyloxymethyl]-cyclopent-1-encarbaldehyd (*rac*-42c).**

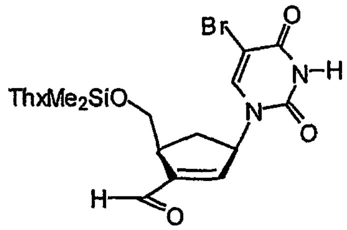

***rac*-42c**

Ein Experiment im 1 mmol-Maßstab lieferte *rac*-**42c** in 83 % Ausbeute als farblosen Feststoff, ausgehend von *rac*-**41c** und Thexyldimethylsilylchlorid (gemäß Vorschrift 25).
**Schmp.:** 163°C (dec.); **$^1$H-NMR** (250 MHz, $CDCl_3$): δ = 9.87 (s, 1H, HC=O), 8.52 (bs, 1H, NH), 7.54 (s, 1H, H-6'), 6.57 (m, 1H, H-2), 5.85 (dddd, $J_1$ = $J_2$ = 2.3, $J_3$ = 8.7, $J_4$ = 7.6, 1H, H-3), 4.25 (dd, $J_1$ = 10.0, $J_2$ = 3.2, 1H, SiOC$H_a$), 3.52 (dd, $J_1$ = 10.0, $J_2$ = 2.3, 1H, SiOC$H_b$), 3.17 (m, 1H, H-5), 2.75 (ddd, $J_1$ = 13.7, $J_2$ = $J_3$ = 8.8, 1H, H-4), 1.85 (ddd, $J_1$ = 13.7, $J_2$ = $J_3$ =7.7, 1H, H-4), 1.55 (septet, *J* = 6.8, 1H, $Me_2CH$), 0.83 (d, *J* = 6.8, 6H, $(CH_3)_2CH$), 0.810 + 0.805 (2s, 6H, $C(CH_3)_2$), 0.06 (s, 6H, $SiCH_3$), **$^{13}$C-NMR** (63 MHz, $CDCl_3$): δ = 188.9 (CH=O), 159.2 (C4'), 150.5 (C2'), 150.4 (C1), 147.3 (C2), 140.2 (C6'), 97.7 (C5'), 61.5 ($CH_2OSi$), 59.9 (C3), 44.3 (C5), 34.0 ($Me_2CH$), 33.5 (C4), 25.3 ($Me_2CSi$), 20.4 and 20.3 ($(CH_3)_2CH$), 18.5 and 18.4 ($(CH_3)_2CSi$), - 3.5 and -3.7 ($CH_3Si$); **FT-IR** (ATR): 3177 (w, N-H), 3046 (w, N-H),

2954 (m, C-H), 2862 (w, C-H), 2823 (w), 1704 (s, C=O), 1684 (s, C=O), 1617 (m, C=C), 1444 (m), 1368 (w), 1276 (m), 1249 (m, C-O), 1150 (w), 1109 (m), 1038 (w), 982 (w), 847 (m), 830 (m); **MS** (EI, 70 eV): m/z (%) = 374 (22), 373 (95), 372 (28), 371 (100) [*M*-85]$^+$, 355 (34), 353 (32), 339 (7), 328 (13), 297 (3), 281 (92), 279 (89), 251 (10), 249 (47), 226 (3), 224 (5), 184 (3), 183 (11), 182 (58), 181 (90), 167 (28), 152 (3), 151 (12), 137 (10), 116 (5), 91 (7), 89 (14), 85 (10), 75 (31), 73 (18), 59 (8); **HRMS** (EI) $C_{13}H_{16}BrN_2O_4Si$ [*M*-85]$^+$: ber. 371.006, gef. 371.005.

**29. Herstellung von (3*RS*,5*RS*)-3-(5-Brom-2,4-dioxo-3,4-dihydro-2*H*-pyrimidin-1-yl)-5-[*tert*butyl-diphenyl-silanyloxymethyl]-cyclopent-1-encarbaldehyd (*rac*-42d).**

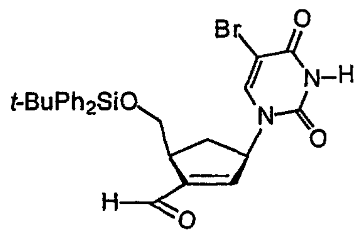

***rac*-42d**

Ein Experiment im 1 mmol-Maßstab lieferte *rac*-**42d** in 76 % Ausbeute als farblosen Feststoff, ausgehend von *rac*-**41c** und *tert*.-Butyldiphenylsilylchlorid (gemäß Vorschrift 25).
**Schmp.:** 164°C; **$^1$H-NMR** (250 MHz, $CDCl_3$): δ = 10.24 (bs, 1H, N<u>H</u>), 9.86 (s, 1H, <u>H</u>C=O), 7.75-7.32 (m, 10H, 2xPh), 7.48 (s, 1H, H-6'), 6.65 (dd, $J_1$ = $J_2$ = 2.0, 1H, H-2), 5.80 (dddd, $J_1$ = $J_2$ = 2.1, $J_3$ = $J_4$ = 8.6, 1H, H-3), 4.21 (dd, $J_1$ = 10.3, $J_2$ = 4.4, 1H, SiOC$H_a$), 3.52 (dd, $J_1$ = 10.3, J2= 2.7, 1H, SiOC$H_b$), 3.15 (m, 1H, H-5), 2.72 (ddd, $J_1$ = 13.7, $J_2$ = $J_3$ = 8.6, 1H, H-$4_a$), 1.85 (ddd, $J_1$ = 13.7, $J_2$ = $J_3$ = 8.4, 1H, H-$4_b$), 1.07 + 1.06 (2s, 9H, $(CH_3)_3C$); **$^{13}$C-NMR** (250 MHz, $CDCl_3$): δ = 188.8 (<u>C</u>H=O), 159.3 (C4'), 150.4 (C2'), 150.4 (C1), 147.1 (C2), 140.0 (C6'), (136.0+135.5) und (133.0+132.8) und (129.8+129.4) und (127.7+127.6) (alle vom Ph), 97.8 (5'), 62.5 ($CH_2OSi$), 60.1 (C3), 44.3 (C5), 34.0 (C4), 26.9 und 26.8 und 26.5 ($(CH_3)_3CSi$), 19.3 ($(CH_3)_3CSi$); **FT-IR** ($cm^{-1}$, ATR): 3177 (w, N-H), 3067 (w, N-H), 2928 (m, C-H), 2854 (m, C-H), 1707 (s, C=O), 1683 (s, C=O), 1619 (m, C=C), 1588 (w), 1469 (w), 1443 (m), 1426 (m), 1362 (w), 1276 (m), 1261 (m), 1242 (m, C-O), 1151 (w), 1110 (m), 1060 (w), 1037 (w), 997 (w), 983 (w), 839 (m), 821 (m), 741 (m), 701 (s).

**30. Herstellung von (3*RS*,5*RS*)-3-(6-Amino-purin-9-yl)-5-[dimethyl-(1,1,2-trimethyl-propyl)-silanyloxy-methyl]-cyclopent-1-encarbaldehyd (*rac*-42e).**

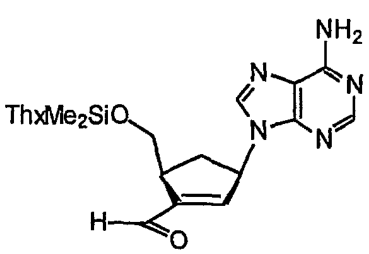

***rac*-42e**

Ein Experiment im 1 mmol-Maßstab lieferte *rac*-**42e** in 45 % Ausbeute als gelb-orangen Feststoff, ausgehend von *rac*-**41d** und Thexyldimethylsilylchlorid (gemäß Vorschrift 25, jedoch unter Verwendung von 2 Äquiv. $PyH^+TsO^-$ (1. Schritt) sowie 3.3 Äquiv. $ThexMe_2SiCl$, 3.3 Äquiv. $Et_3N$, 0.3 Äquiv. DMAP in 5 ml $CH_2Cl_2$ (2. Schritt).
**Schmp.:** 144°C (dec.); **$^1$H-NMR** (250 MHz, $CDCl_3$): δ = 9.86 (s, 1H, <u>H</u>C=O), 8.30 (s, 1H, H-2'), 7.93 (s, 1H, H-8'), 6.75 (dd, $J_1$ = $J_2$ = 2.2, 1H, H-2), 6.40 (s, 2H, N$H_2$), 5.84 (dddd, $J_1$ = $J_2$ = 2.2, $J_3$ = 9.1, $J_4$ = 6.8, 1H, H-3), 4.14 (dd, $J_1$ = 10.0, $J_2$ = 3.8, 1H, SiOC$H_a$), 3.66 (dd, $J_1$ = 10.0, $J_2$ = 2.4, 1H, SiOC$H_b$), 3.24 (m, 1H, H-5), 2.89 (ddd, $J_1$ = 14.0, $J_2$ =

$J_3$ = 9.1, 1H, H-4), 2.10 (ddd, $J_1$ = 14.0, $J_2$ = $J_3$ = 6.8, 1H, H-4), 1.53 (septet, $J$ = 6.8, 1H, $Me_2C\underline{H}$), 0.78 (d, $J$ = 6.8, 6H, $(C\underline{H}_3)_2CH$), 0.77 (s, 6H, $C(C\underline{H}_3)_2$), 0.02 (s, 6H, $C\underline{H}_3Si$), **$^{13}$C-NMR** (63 MHz, $CDCl_3$): δ = 189.2 ($\underline{C}H$=O), 155.7 (C4'), 152.8 (C2'), 149.5 (C1), 149.5 (C6'), 147.4 (C2), 138.5 (C8'), 119.2 (C5'), 62.2 ($\underline{C}H_2OSi$), 57.5 (C3), 44.7 (C5), 35.1 (C4), 34.0 ($Me_2\underline{C}H$), 25.3 ($Me_2\underline{C}Si$), 20.3 and 20.2 ($(\underline{C}H_3)_2CH$), 18.4 and 18.4 ($(\underline{C}H_3)_2CSi$), -3.6 ($\underline{C}H_3Si$); **FT-IR** (ATR): 3318 (w, N-H), 3168 (w, N-H), 2953 (m, C-H), 2864 (w, C-H), 1683 (s, C=O), 1645 (s, C=C), 1598 (s), 1575 (m), 1468 (m), 1413 (m), 1327 (m), 1297 (m), 1250 (s, C-O), 1149 (w), 1109 (m), 1085 (m), 1085 (m), 1015 (w), 979 (w), 830 (m), 777 (m), 649 (m); **MS** (EI, 70 eV): m/z (%): 401(2) $[M]^+$, 318 (8), 317 (24), 316 (100), 302 (7), 242 (4), 224 (5), 212 (4), 192 (5), 182 (11), 181 (30), 167 (8), 151 (7), 137 (4), 136 (12), 135 (10), 108 (3), 85 (4), 75 (17), 59 (4); **HRMS** (ESI) $C_{20}H_{31}N_5O_2Si$: ber. 402.2325, gef. 402.2327.

**31. Allgemeine Arbeitsvorschrift für die Überführung der Aldehyde vom Typ 42' in Ester vom Typ 43' durch Wittig-Reaktion.**

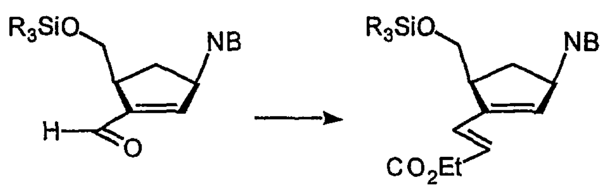

***rac*-42'** ***rac*-43'**

Zu einer Lösung des benötigten Wittig-Ylides (202 mg, 1.1 eq.) in trockenem THF (2 ml) gibt man bei 50°C eine Lösung des Aldehydes ***rac*-42'** (500 µmol, 1 eq.) in trockenem THF (4 ml). Nach 16 h Rühren bei 50°C wird die Reaktionsmischung auf Raumtemp. abgekühlt, im Vakuum etwas eingeengt und das Produkt ***rac*-42'** durch Chromatographie an Kieselgel (EtOAc/CyHex = 1+2) gereinigt.

**32. Herstellung von (3*RS*,5*RS*)-3-[5-[Dimethyl-(1,1,2-trimethyl-propyl)-silanyloxymethyl]-3-(2,4-dioao-3,4-dihydro-2*H*-pyrimidin-1-yl)-cyclopent-1-enyl]-acrylsäure-ethylester (*rac*-43a).**

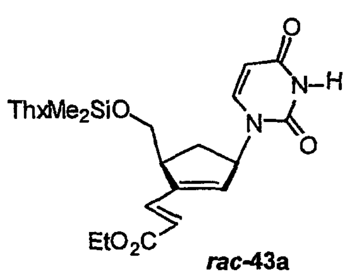

***rac*-43a**

Ein Experiment im 0.25 mmol-Maßstab lieferte *rac*-**43a** in 96 % Ausbeute als blassgelben Feststoff, ausgehend von *rac*-**42a** (gemäß Vorschrift 31).

**Schmp.** 92-96°C (EtOAc/Hex); **$^1$H-NMR** (250 MHz, $CDCl_3$): δ = 8.45 (s, 1H, NH), 7.47 (d, $J_{cis}$ = 8.0, 1H, H-5"), 7.38 (d, $J_{trans}$ = 16.2, 1H, H-2), 6.06 (d, $J_{trans}$ = 16.2, 1H, H-3), 5.91 (s, 1H, H-2'), 5.71 (m, 1H, H-3'), 5.66 (d, $J_{cis}$ = 8.0, 1H, H-6"), 4.22 (q, $J$ = 7.2, 2H, $OC\underline{H}_2CH_3$), 3.96 (dd, $J_1$ = 10.4, $J_2$ = 3.8, 1H, $SiOC\underline{H}_a$), 3.63 (dd, $J_1$ = 10.5, $J_2$ = 2.7, 1H, $SiOC\underline{H}_b$), 3.08 (m, 1H, H-5'), 2.79 (ddd, $J_1$ = 14.3, $J_2$ = $J_3$ = 9.5, 1H, H-4'), 1.73 (ddd, $J_1$ = 14.5, $J_2$ = $J_3$ = 5.5, 1H, H-4'), 1.55 (septet, $J$ = 6.8, 1H, $Me_2C\underline{H}$), 1.29 (t, $J$ = 7.1, 3H, $OCH_2C\underline{H}_3$), 0.81 (d, $J$ = 6.8, 6H, $(C\underline{H}_3)_2CH$), 0.79 (s, 6H, $C(C\underline{H}_3)_2$), 0.04 (s, 6H, $C\underline{H}_3Si$); **$^{13}$C-NMR** (63 MHz, $CDCl_3$): δ = 166.4 (C1), 163.0 (C4"), 150.8 (C2"), 146.7 (C1'), 141.7 (C6"), 137.9 (C3), 134.7 (C2'), 121.7 (C2), 102.5 (C5"), 63.2 ($\underline{C}H_2OSi$), 60.7 $O\underline{C}H_2CH_3$), 59.4 (C3'), 45.8 (C5'), 34.0 ($Me_2\underline{C}H$), 33.7 (C4'), 25.4 ($Me_2\underline{C}Si$), 20.4 and 20.2 ($(\underline{C}H_3)_2CH$), 18.5 and 18.3 ($(\underline{C}H_3)_2CSi$), 14.3 ($OCH_2\underline{C}H_3$), -3.5 ($\underline{C}H_3Si$); **FT-IR** (ATR): 3184 (w, N-H), 3051(w, N-H), 2955 (m, C-H), 2865 (w, C-H), 1703 (s, C=O), 1687 (s, C=O), 1638 (m, C=C), 1605 (w), 1461 (m), 1378 (m), 1305 (m), 1265 (s, C-O), 1249 (s, C-O), 1174 (s), 1111 (m), 1094 (m),

1064 (w), 1034 (w), 986 (w), 873 (w), 830 (s), 778 (m);
**MS** (EI, 70 eV): m/z (%): 449 (1) $[M+1]^+$, 403 (45), 379 (13), 363 (15) $[M-85]^+$, 253 (8), 252 (25), 251 (100), 205 (37), 188 (7), 187 (41), 177 (33), 169 (58), 149 (9), 133 (5), 132 (8), 131 (48), 104 (13), 103 (44), 99 (18), 89 (17), 77 (7), 75 (35), 74 (9), 73 (38), 59(10), 58 (21);
**HRMS** (EI) $C_{17}H_{23}N_2O_5Si$ ($M^+$-85): ber. 363.138, gef. 363.138.

**33. Herstellung von (3*RS*,5*RS*)-3-[5-[Dimethyl-(1,1,2-trimethyl-propyl)-silanyloxymethyl]-3-(5-methyl-2,4-dioxo-3,4-dihydro-2*H*-pyrimidin-1-yl)-cyclopent-1-enyl]-acrylsäureethylester (*rac*-43b).**

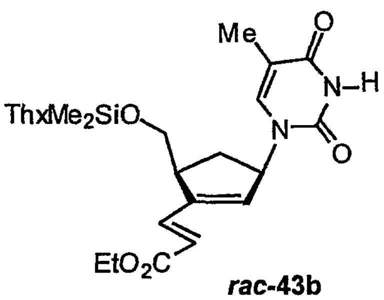

***rac*-43b**

Ein Experiment im 0.25 mmol-Maßstab lieferte *rac*-**43b** in 95 % Ausbeute als blassgelben Feststoff, ausgehend von *rac*-**42b** (gemäß Vorschrift 31).
**Schmp.:** 132-134 (EtOAc/Hex); **$^1$H-NMR** (250 MHz, $CDCl_3$): δ = 8.50 (s, 1H, NH), 7.38 (d, $J_{trans}$ = 16.2, 1H, H-2), 7.05 (s, 1H, H-6"), 6.08 (d, $J_{trans}$ = 16.2, 1H, H-3), 5.93 (s, 1H, H-2'), 5.65 (m, 1H, H-3'), 4.22 (q, J= 7.1, 2H, $OC\underline{H_2}CH_3$), 3.87 (dd, $J_1$ = 10.4, $J_2$ = 4.4, 1H, $SiOC\underline{H_a}$), 3.63 (dd, $J_1$ = 10.4, $J_2$ = 3.3, 1H, $SiOC\underline{H_b}$), 3.06 (m, 1H, H-5'), 2.72 (ddd, $J_1$ = 13.9, $J_2$ = $J_3$ = 8.9, 1H, H-4'), 1.88 (s, 3H, $C\underline{H_3}$-5"), 1.72 (ddd, $J_1$ = 13.6, $J_2$ = $J_3$ = 6.7, 1H, H-4'), 1.56 (septet, *J*= 6.8, 1H, $Me_2C\underline{H}$), 1.29 (t, *J* = 7.1, 3H, $OCH_2C\underline{H_3}$), 0.82 (d, J= 6.9, 6H, $(C\underline{H_3})_2CH$), 0.79 (s, 6H, $C(C\underline{H_3})_2$), 0.04 (s, 6H, $C\underline{H_3}Si$); **$^{13}$C-NMR** (63 MHz, $CDCl_3$): δ = 166.4 (C1), 163.6 (C4"), 150.8 (C2"), 146.7 (C1'), 138.1 (C6"), 136.7 (C3), 134.6 (C2'), 121.5 (C2), 111.0 (C5"), 63.0 ($\underline{C}H_2OSi$), 60.7 $O\underline{C}H_2CH_3$), 59.5 (C3'), 45.9 (C5'), 34.0 ($Me_2\underline{C}H$), 33.9 (C4'), 25.3 ($Me_2\underline{C}Si$), 20.34 and 20.27 ($(\underline{C}H_3)_2CH$), 18.48 and 18.44 ($(\underline{C}H_3)_2CSi$), 14.2 ($OCH_2\underline{C}H_3$), 12.5 (5"-$\underline{C}H_3$), - 3.5 ($\underline{C}H_3Si$); **FT-IR** (ATR): 3176 (w, N-H), 3043 (w, N-H), 2954 (m, C-H), 2864 (w, C-H), 1684 (s, C=O), 1639 (s, C=C), 1603 (w), 1464 (m), 1387 (m), 1377 (w), 1364(m), 1305 (m), 1266 (s, C-O), 1249 (s, C-O), 1173 (s), 1159(s), 1112 (m), 1094 (m), 1065 (w), 1034 (w), 985 (w), 873 (w), 830 (s); **MS** (EI, 70 eV): m/z (%): 449 (1) $[M+1]^+$, 403 (45), 379 (13), 363 (15) $[M-85]^+$, 253 (8), 252 (25), 251 (100), 205 (37), 188 (7), 187 (41), 177 (33), 169 (58), 149 (9), 133 (5), 132 (8), 131 (48), 104 (13), 103 (44), 99 (18), 89 (17), 77 (7), 75 (35), 74 (9), 73 (38), 59(10), 58 (21); **HRMS** (EI) $C_{18}H_{25}N_2O_5Si$ ($M^+$-85): ber. 377.153, gef. 377.153.

**34. Herstellung von (3*RS*,5*RS*)-3-[3-(5-Brom-2,4-dioxo-3,4-dibydro-2*H*-pyrimidin-1-yl)-5-[dimethyl-(1,1,2-trimethyl-propyl)-silanyloxymethyl]-cyclopent-1-enyl]-acrylsäure-ethylester (*rac*-43c).**

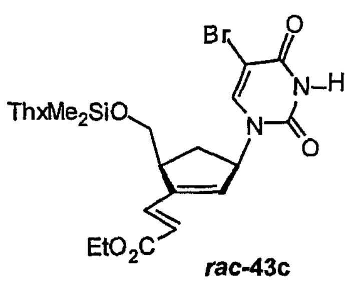

***rac*-43c**

Ein Experiment im 0.5 mmol-Maßstab lieferte *rac*-**43c** in 82 % Ausbeute als farblosen Feststoff, ausgehend von *rac*-**42c** (gemäß Vorschrift 31).

**Schmp**.: 164-165°C (EtOAc/CyHex); **$^1$H-NMR** (250 MHz, $CDCl_3$): δ = 10.17 (s, 1H, NH), 7.51 (s, 1H, H-6"), 7.35 (d, $J_{trans}$ = 16.4, 1H, H-2), 6.04 (d, $J_{trans}$ = 16.0,1H, H-3), 5.92 (s, 1H, H-2'), 5.62 (m, 1H, H-3'), 4.17 (q, $J$ = 7.1, 2H, $OC\underline{H}_2CH_3$), 3.84 (dd, $J_1$ = 10.4, $J_2$ = 4.2, 1H, $SiOC\underline{H}_a$), 3.57 (dd, $J_1$ = 10.2, $J_2$ = 3.2, 1H, $SiOC\underline{H}_b$), 3.03 (m, 1H, H-5'), 2.72 (ddd, $J_1$ = 14.1, $J_2$ = $J_3$ = 9.0, 1H, H-4'), 1.68 (ddd, $J_1$ = 13.9, $J_2$ = $J_3$ = 6.4, 1H, H-4'), 1.51 (Sept., J = 6.8, 1H, $Me_2C\underline{H}$), 1.24 (t, J= 7.0, 3H, $OCH_2C\underline{H}_3$), 0.77 (d, $J$ = 6.8, 6H, $(C\underline{H}_3)_2CH$), 0.74 (s, 6H, $C(C\underline{H}_3)_2$), 0.01 + - 0.01 (2xs, 6H, $C\underline{H}_3Si$); **$^{13}$C NMR** (125 MHz, $CDCl_3$): δ = 166.2 (C1), 159.3 (C4"), 150.6 (C2"), 147.3 (C1'), 140.3 (C6"), 137.8 (C3), 133.7 (C2'), 121.7 (C2), 96.9 (C5"), 62.8 ($C\underline{H}_2OSi$), 60.5 $O\underline{C}H_2CH_3$), 60.3 (C3'), 45.7 (C5'), 33.9 ($Me_2\underline{C}H$), 33.8 (C4'), 25.2 ($Me_2\underline{C}Si$), 20.28 und 20.13 ($(\underline{C}H_3)_2CH$), 18.35 und 18.26 ($(\underline{C}H_3)_2CSi$), 14.1 ($OCH_2\underline{C}H_3$), -3.56 und -3.64 ($(\underline{C}H_3)_2Si$); **FT-R**(ATR): 3178 (w, N-H), 3053 (w, N-H), 2954 (m, C-H), 2864 (w, C-H), 1705 (s, C=O), 1637 (m, C=C), 1617 (m), 1442 (m), 1389 (w), 1366 (w), 1337(w), 1306 (m), 1265 (m, C-O), 1249 (m, C-O), 1174 (m), 1112 (m), 1066 (w), 1034 (m), 985 (w), 874 (w), 830 (m), 777 (m).

**35. Herstellung von (3*RS*,5*RS*)-3-[3-(5-Bromo-2,4-dioxo-3,4-dihydro-2*H*-pyrimidin-1-yl)-5-(*tert*-butyl-diphenyl-silanyloxymethyl)-cyclopent-1-enyl]-acrylsäureethylester (*rac*-43d).**

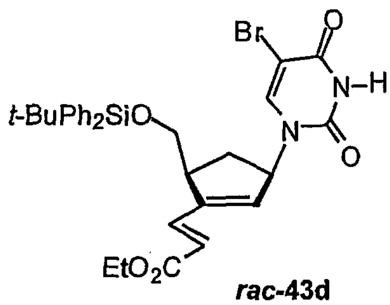

***rac*-43d**

Ein Experiment im 0.2 mmol-Maßstab lieferte *rac*-**43d** in 72 % Ausbeute als farbloses Öl, ausgehend von *rac*-**42c** (gemäß Vorschrift 31).

**$^1$H-NMR** (250 MHz, $CDCl_3$): δ = 9.61 (s, 1H, NH), 7.44-7.32 (m, 12H, H-6" , H-3, 2xPh), 5.94 (d, $J_{trans}$ = 16.1, 1H, H-3), 5.93 (s, 1H, H-2'), 5.61 (m, 1H, H-3'), 4.22 (q, $J$ = 7.1, 2H, $OC\underline{H}_2CH_3$), 3.74 (m, 2H, $SiOC\underline{H}_2$), 3.08 (m, 1H, H-5'), 2.77 (ddd, $J_1$ = 14.2, $J_2$ = $J_3$ = 8.8, 1H, H-4'), 1.79 (ddd, $J_1$ = 14.1, $J_2$ = $J_3$ = 6.3, 1H, H-4'), 1.30 (t, $J$ = 7.0, 3H, $OCH_2C\underline{H}_3$), 1.04 (s, 9H, $C(C\underline{H}_3)_3$); **$^{13}$C-NMR** (63 MHz, $CDCl_3$): δ = 166.2 (C1), 159.1 (C4"), 150.3 (C2"), 147.7 (C1'), 140.0 (C6"), 137.6 (C3), (135.5 + 135.4) and (132.9 + 132.7) and (129.9 + 129.5) and (127.8 + 127.7) (all of Ph), 133.4 (C2'), 122.2 (C2), 97.1 (C5"), 64.2 ($\underline{C}H_2OSi$), 60.7 ($O\underline{C}H_2CH_3$), 60.4 (C3'), 46.0 (C5'), 34.3 (C4'), 26.9 ($(\underline{C}H_3)_3C$), 19.2 ($(CH_3)_3\underline{C}$), 14.2 ($OCH_2\underline{C}H_3$); **FT-IR** (ATR): 3472 (w), 3178 (w, N-H), 3066 (w, N-H), 2953 (w, C-H), 2854 (w, C-H), 1703 (s, C=O), 1638 (m, C=C), 1617 (m), 1442 (m), 1426 (m), 1367 (w), 1306 (w), 1266 (m, C-O), 1240 (m, C-O), 1175 (m), 1110 (s), 1034 (w), 985 (w), 821 (m), 741 (m), 702 (s).

**36. Allgemeine Arbeitsvorschrift für die $Fe(CO)_3$-Komplexierung von Dienen des Typs 43'.**

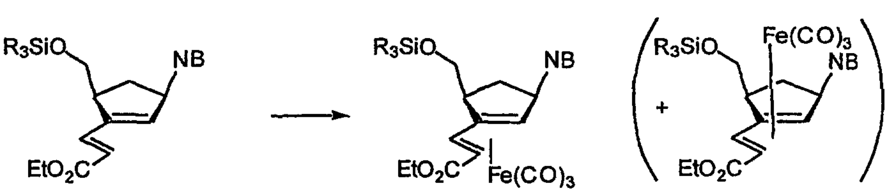

*rac*-**43'** *rac*-**44'**

Zu einer gerührten Suspension von $Fe_2(CO)_9$ (91 mg, 0.25 mmol) in abs. $Et_2O$ (5 ml) gibt man unter Argon eine Lösung des Diens *rac*-**43'** (0.1 mmol) in abs. $Et_2O$ (1 ml) und erhitzt die Mischung für 24 h zum Rückfluß. Nach

Abkühlung auf Raumtemp. entfernt man das Lösungsmittel im Vakuum und reinigt den Rückstand durch Flashchromatographie (EtOAc/CyHex = 1 : 4 bis = 1 : 2). Neben dem Hauptprodukt *rac*-**44'** erhält man stets noch (separiert) gewisse Mengen des diastereomeren Komplexierungsproduktes.

**37. Herstellung von (3S*R*,5*RS*)-3-[5-[Dimethyl-(1,1,2-trimethyl-propyl)-silanyloxy-methyl]-3-(2,4-dioxo-3,4-dihydro-2*H*-pyrimidin-1-yl)-cyclopent-1-enyl]-acrylsäureethylestertricarbonyleisen (*rac*-44a).**

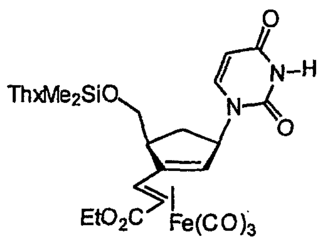

***rac*-44a**

Ein Experiment im 0.1 mmol-Maßstab lieferte *rac*-**44a** in 37 % Ausbeute als orange-rotes Öl ausgehend von *rac*-**43a** (gemäß Vorschrift 36).

**$^1$H-NMR** (250 MHz, $CDCl_3$): δ = 8.99 (s, 1H, NH), 7.59 (d, $J_{cis}$ = 7.7, 1H, H-5"), 6.06 (d, $J$ = 7.9, 1H, H-3), 5.68 (d, $J_{cis}$ = 8.0, 1H, H-6"), 5.16 (dd, $J_1$ = 10.0, $J_2$ = 3.4, 1H, H-3'), 4.10 (m, 2H, OC$\underline{H}_2$CH$_3$), 3.98 (dd, $J_1$ = 10.1, $J_2$ = 4.9, 1H, SiOC$\underline{H}_a$), 3.78 (dd, $J_1$ = 10.2, $J_2$ = 5.9, 1H, SiOC$\underline{H}_b$), 3.14 (m, 1H, H-5'), 2.66 (ddd, $J_1$ = 15.5, $J_2$ = $J_3$ = 9.5, 1H, H-4$_a$'), 1.87 (s, 1H, H-2'), 1.76 (ddd, $J_1$ = 15.3, $J_2$ = $J_3$ = 3.5, 1H, H-4$_b$'), 1.62 (Sept., $J$= 6.8, 1H, Me$_2$C$\underline{H}$), 1.22 (t, $J$= 7.1, 3H, OCH$_2$C$\underline{H}_3$), 0.86 (d, $J$ = 6.8, 6H, (C$\underline{H}_3$)$_2$CH), 0.85 (s, 6H, C(C$\underline{H}_3$)$_2$), 0.70 (d, $J$= 7.6, 1H, H-2), 0.14 + 0.12 (2xs, 6H, C$\underline{H}_3$Si); **$^{13}$C-NMR** (63 MHz, $CDCl_3$): δ = 171.8 (C1), 163.0 (C4"), 150.5 (C2"), 141.2 (C6"), 113.1 (C1'), 102.7 (C5"), 78.2 (C3), 67.3 ($\underline{C}$H$_2$OSi), 64.2 (C2'), 60.7 (O$\underline{C}$H$_2$CH$_3$), 59.4 (C3'), 49.6 (C2), 45.4 (C5'), 34.0 (Me$_2\underline{C}$H), 33.9 (C4'), 25.5 (Me$_2\underline{C}$Si), 20.5 and 20.3 (($\underline{C}$H$_3$)$_2$CH), 18.5 and 18.4 (($\underline{C}$H$_3$)$_2$CSi), 14.1 (OCH$_2\underline{C}$H$_3$), -3.2 and -3.3 ($\underline{C}$H$_3$Si); **FT- IR** (ATR): 3189 (w, N-H), 3053 (w, N-H), 2956 (m, C-H), 2865 (w, C-H), 2055 (s, C≡O), 1992 (s, C≡O), 1974 (s, C≡O), 1701 and 1697 and 1692 and 1681 (s, C=O), 1630 (m, C=C), 1495 (w), 1461 (m), 1427 (m), 1375 (m), 1275 (m, C-O), 1250 (m, C-O), 1177 (m), 1098 (m), 1033 (m), 873 (w), 830 (m), 778 (m), 615 (m), 607 (m);

**38. Herstellung von (3*RS*,5*RS*)-3-[5-[Dimethyl-(1,1,2-trimethyl-propyl)-silanyloxy-methyl]-3-(5-methyl-2,4-dioxo-3,4-dihydro-2*H*-pyrimidin-1-yl)-cyclopent-1-enyl]-acrylsäureethylestertricarbonyleisen (*rac*-44b).**

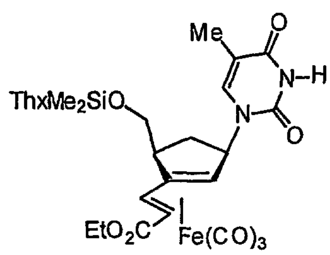

***rac*-44b**

Ein Experiment im 0.1 mmol-Maßstab lieferte *rac*-**44b** in 40 % Ausbeute als orange-rotes Öl ausgehend von *rac*-**43b** (gemäß Vorschrift 36).

**$^1$H-NMR** (250 MHz, $CDCl_3$): δ = 8.56 (s, 1H, NH), 7.10 (s, 1H, H-6"), 6.12 (d, $J$ = 7.9, 1H, H-3), 5.07 (dd, $J_1$ = 9.4, $J_2$ = 3.6, 1H, H-3'), 4.11 (m, 2H, OC$\underline{H}_2$CH$_3$), 3.84 (dd, $J_1$ = 10.2, $J_2$ = 6.3, 1H, SiOC$\underline{H}_a$), 3.73 (dd, $J_1$ = 10.1, $J_2$ = 7.6, 1H, SiOC$\underline{H}_b$), 3.12 (m, 1H, H-5'), 2.60 (ddd, $J_1$ = 15.5, $J_2$ = $J_3$ = 9.1, 1H, H-4$_a$'), 1.89 (s, 4H, H-2' + CH$_3$), 1.73 - 1.56 (m, 2H, H-4$_b$' + Me$_2$C$\underline{H}$), 1.23 (t, $J$ = 7.2, 3H, OCH$_2$C$\underline{H}_3$), 0.88 (d, $J$ = 6.5, 6H, (C$\underline{H}_3$)$_2$CH), 0.87 (s, 6H, C(C$\underline{H}_3$)$_2$), 0.72 (d, $J$ = 7.8, 1H, H-2), 0.13 (s, 6H, C$\underline{H}_3$Si); **$^{13}$C-NMR** (63 MHz, $CDCl_3$): δ = 171.7 (C1), 163.3 (C4"), 150.4 (C2"), 136.3 (C6"), 112.9 (C1'), 111.2 (C5"), 78.9 (C3), 66.9 ($\underline{C}$H$_2$OSi), 63.4 (C2'), 60.6 (O$\underline{C}$H$_2$CH$_3$), 60.0 (C3'), 46.6 (C2), 45.5 (C5'),

34.1 ($Me_2\underline{C}H$), 33.8 (C4'), 25.4 ($Me_2\underline{C}Si$), 20.4 und 20.3 ($(\underline{C}H_3)_2CH$), 18.6 und 18.5 ($(\underline{C}H_3)_2CSi$), 14.1 ($OCH_2\underline{C}H_3$), 12.6 (5"-$\underline{C}H_3$), -3.3 ($\underline{C}H_3Si$); **FT-IR** (ATR): 3178 (w, N-H), 3047 (w, N-H), 2955 (m, C-H), 2864 (w, C-H), 2094 (w, C≡O), 2056 (s, C≡O), 2023 (s, C≡O), 1991 (s, C≡O), 1701 und 1698 und 1692 (s, C=O), 1465 (m), 1450 (w), 1426 (w), 1389 (w), 1376 (w), 1366 (w), 1278 (m, C-O), 1250 (m, C-O), 1220 (w), 1175 (m), 1111 (m), 1094 (m), 1035 (w), 873 (w), 830 (m), 777 (m), 621 (m), 610 (m).

**39. Herstellung von (3*RS*,5*RS*)-3-[3-(5-Brom-2,4-dioxo-3,4-dihydro-2*H*-pyrimidin-1-yl)-5-[dimethyl-(1,1,2-trimethyl-propyl)-silanyloxymethyl]-cyclopent-1-enyl]-acrylsäureethylester-tricarbonyleisen (*rac*-35 = *rac*-44c).**

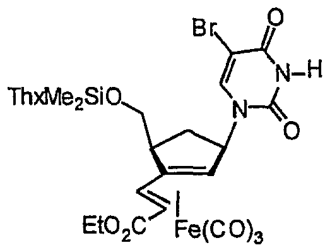

***rac*-44c (= *rac*-35)**

Ein Experiment im 0.1 mmol-Maßstab lieferte, ausgehend von *rac*-**43c**, *rac*-**44c** in 73 % Ausbeute als gelbes Öl, das aus Cyclohexan kristallisierte (gemäß Vorschrift 36).
**Schmp.:** 100 - 104°C (CyHex); **$^1$H-NMR** (250 MHz, $CDCl_3$): δ = 8.79 (s, 1H, NH), 7.64 (s, 1H, H-6"), 6.13 (d, $J$ = 7.9, 1H, H-3), 5.09 (dd, $J_1$ = 9.6, $J_2$ = 3.6, 1H, H-3'), 4.12 (m, 2H, $OC\underline{H}_2CH_3$), 3.87 (dd, $J_1$ = 10.1, $J_2$ = 6.2, 1H, $SiOC\underline{H}_a$), 3.73 (dd, $J_1$ = 10.1, $J_2$ = 7.2, 1H, $SiOC\underline{H}_b$), 3.14 (m, 1H, H-5'), 2.63 (ddd, $J_1$ = 15.3, $J_2$ = $J_3$ = 9.4, 1H, H-4$_a$'), 1.85 (s, 1H, H-2'), 1.72 (ddd, $J_1$ = 15.6, $J_2$ = $J_3$ = 3.9, 1H, H-4$_b$'), 1.65 (Sept., $J$= 6.9, 1H, $Me_2C\underline{H}$), 1.23 (t, J= 7.1, 3H, $OCH_2C\underline{H}_3$), 0.88 (d, $J$ = 6.6, 6H, $(C\underline{H}_3)_2CH$), 0.88 (s, 6H, $C(C\underline{H}_3)_2$), 0.76 (d, J= 7.7, 1H, H-2), 0.16 + 0.15 (2xs, 6H, $C\underline{H}_3Si$); **$^{13}$C-NMR** (63 MHz, $CDCl_3$): δ = 171.6 (C1), 158.6 (C4"), 149.7 (C2"), 140.0 (C6"), 112.9 (C1'), 97.0 (C5"), 79.0 (C3), 67.0 ($\underline{C}H_2OSi$), 62.4 (C2'), 60.73 ($O\underline{C}H_2CH_3$), 60.69 (C3'), 46.5 (C2), 45.7 (C5'), 34.1 ($Me_2\underline{C}H$), 33.9 (C4'), 25.5 ($Me_2\underline{C}Si$), 20.5 und 20.4 ($(\underline{C}H_3)_2CH$), 18.6 und 18.5 ($(\underline{C}H_3)_2CSi$), 14.1 ($OCH_2\underline{C}H_3$), -3.22 und -3.24 ($(\underline{C}H_3)_2Si$); **FT-R** (ATR): 3179 (w, N-H), 3055 (w, N-H), 2954 (m, C-H), 2926 (m, C-H), 2863 (w, C-H), 2058 (s, C≡O), 1996 (s, C≡O), 1978 (s, C≡O), 1701 (s, C=O), 1617 (w), 1445 (w), 1427 (w), 1376 (w), 1271 (m, C-O), 1252 (m, C-O), 1177 (m), 1104 (m), 1035 (w), 871 (w), 831 (m), 778 (w), 613 (m);

**40. Herstellung von (3*RS*,5*RS*)-3-[3-(5-Brom-2,4-dioxo-3,4-dihydro-2*H*-pyrimidin-1-yl)-5-(*tert*-butyl-diphenyl-silanyloxymethyl)-cyclopent-1-enyl]-acrylsäure-ethylester tricarbonyleisen (*rac*-44d).**

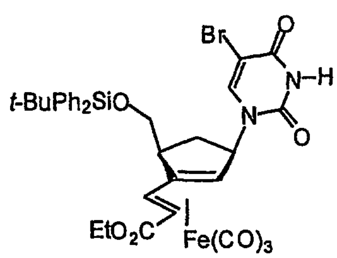

***rac*-44d**

Ein Experiment im 0.91 mmol-Maßstab lieferte *rac*-**44d** in 49 % Ausbeute als gelb-oranges Öl ausgehend von *rac*-**43d** (gemäß Vorschrift 36).
**$^1$H-NMR** (250 MHz, $CDCl_3$): δ= 8.58 (s, 1H, NH), 7.67-7.63 (m, 5H, H-6" + Ph), 7.44-7.35 (m, 6H, Ph), 6.10 (d, $J$ = 8.0, 1H, H-3), 5.02 (dd, $J_1$ = 9.4, $J_2$ = 3.2, 1H, H-3'), 4.12 (m, 2H, $OC\underline{H}_2CH_3$), 3.81 (d, $J$ = 7.0, 2H, $SiOC\underline{H}_2$), 3.14 (m, 1H, H-5'), 2.60 (ddd, $J_1$ = 15.7, $J_2$ = $J_3$ = 9.1, 1H, H-4$_a$'), 1.76 (s, 1H, H-2'), 1.63 (ddd, $J_1$ = 15.9, $J_2$ = $J_3$ = 3.6, 1H, H-4$_b$'),

1.26 (t, $J$ = 7.1, 3H, $OCH_2C\underline{H}_3$), 1.11 (s, 9H, $(C\underline{H}_3)_3C$), 0.67 (d, $J$ = 7.8, 1H, H-2); **FT-R** (ATR): 3184 (w, N-H), 3068 (w, N-H), 2928 (w, C-H), 2855 (w, C-H), 2058 (s, C≡O), 1997 (s, C≡O), 1979 (s, C≡O), 1698 (s, C=O), 1617 (w), 1444 (w), 1426 (m), 1375 (w), 1270 (m, C-O), 1238 (m, C-O), 1178 (m), 1111 (m), 1034 (w), 822 (w), 740 (w), 701 (m), 625 (w), 613 (m), 609 (m);

**41. Herstellung von 5-O-(Trityl)-D-ribono-1,4-lacton (45')**

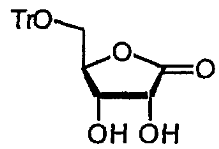

**45'**

20.1 g (+)-D-Ribonsäure-γ-lacton (0.136 mol) wurden in 350 ml Pyridin gelöst. Zur weitgehenden Entfernung von Wasser wurden etwa 50 ml des Lösungsmittels am Rotationsverdampfer abdestilliert. Anschließend wurde mit 41.7 g Chlortriphenylmethan (0.149 mmol) versetzt und ein Trockenrohr ($CaCl_2$) aufgesetzt. Bei 55°C wurde über Nacht gerührt. Das Lösungsmittel wurde am Rotationsverdampfer weitgehend entfernt. Der Rückstand wurde mit EtOAc und 0.1%iger HCl in einen Scheidetrichter überführt. Die organische Phase wurde mit ges. NaCl-Lösung gewaschen und die wäßrigen Phasen zweimal mit EtOAc reextrahiert. Die vereinigten organischen Phasen wurden über $Na_2SO_4$ getrocknet, das Lösungsmittel am Rotationsverdampfer entfernt und das Produkt aus Chloroform umkristallisiert. Das Produkt 45' kristallisierte zusammen mit einem Äquivalent Chloroform in feinen, weißen Nadeln aus (44.0 g, 63%). Einengen der Mutterlauge ergab weitere 490 mg ( 4%).

$[\alpha]_D^{20}$ = +33.5 ($c$ = 1.0 in Benzol); **$^1$H-NMR** (250 MHz, $C_6D_6$): δ = 2.40 (d, $J$= 1.5 Hz, 1H, HO-C4, austauschbar gegen $D_2O$), 2.58 (d, $J$= 4.4 Hz, 1H, HO-C3, austauschbar gegen $D_2O$), 2.68 (dd, $J$= 2.6, 10.9 Hz, 1H, C*H*HO), 3.23 (dd, $J$= 3.2, 10.9 Hz, 1H, CH*H*O), 3.73 (dd, $J$= 1.5, 5.4 Hz, 1H, H-C4), 4.10 (ψt, 1H, H-C5), 4.63 (dd, $J$= 4.4, 5.4 Hz, 1H, H-C3), 6.95-7.07 und 7.30-7.34 (m, 15H, ArH); **$^{13}$C-NMR** (62.5 MHz, $CDCl_3$): δ = 63.3 ($CH_2O$), 69.8 (C-4), 70.6 (C-3), 84.5 (C-5), 88.0 ($CAr_3$), 127.6, 128.3, 128.4, 143.6 (Ar), 177.2 (C-2); **FT-IR:** 3416, 3055, 2931, 2872, 1777, 1595, 1488, 1447, 1220, 1181, 1139, 1090, 1031, 1022, 993, 947, 766, 745, 703; **MS** (EI, 70 eV): 390 (18) $[M]^+$, 313 (20), 243 (100), 183 (19), 165 (47); **HR-MS** (EI, 70 eV): berechnet für $C_{24}H_{22}O_5$: 390.147; gefunden: 390.146; **Schmp.:** 104.6°C.

**42. Herstellung von Trifluormethansulfonsäure-2-oxo-5-trityloxymethyl-2,5-dihydrofuran-3-ylester (46)**

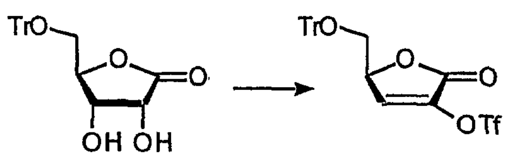

**45'** **46**

10.45 g des Lactons **45'** (20.5 mmol) wurden unter Schutzgasatmosphäre in 100 ml trockenem Dichlormethan und 8.6 ml trockenem Pyridin (0.107 mol) gelöst und auf -78°C gekühlt. Dem Gemisch wurde eine Lösung aus 9.5 ml Trifluormethansulfonsäureanhydrid (56.3 mmol) in 35 ml trockenem Dichlormethan zugetropft. Nach einer Stunde wurde auf -15°C erwärmt und eine weitere Stunde gerührt. Nach Abkühlen auf -78°C wurde mit 100 ml Diethylether versetzt und mit weiteren 500 ml Diethylether durch Kieselgel filtriert. Die Lösungsmittel wurden am Rotationsverdampfer entfernt, das Rohprodukt wurde durch Säulenchromatographie an Kieselgel mit CyHex/EtOAC (8/1) gereinigt. Das Produkt **46** (8.84 g, 85%) wurde als weißer Feststoff erhalten, der aus Hexan/MTBE umkristallisiert wurde.

$[\alpha]_D^{20}$ = -43.9 ($c$ = 1.0 in $CHCl_3$), **$^1$H-NMR** (250 MHz, $CDCl_3$): δ = 3.44 (dd, $J_{AB}$ = 10.5 Hz, $J$ = 4.3 Hz, 1H, C*H*HO), 3.55 (dd, $J_{AB}$ = 10.5 Hz, $J$ = 4.7 Hz, 1H, CH*H*O), 5.06 (m, $J$ = 4.5, 1.9 Hz, 1H, H-C5), 7.11 (d, $J$ = 1.9, 1H, H-C4), 7.24-7.40 (m, 15H, ArH); **$^{13}$C-NMR** (62.5 MHz, $CDCl_3$): δ = 62.7 ($CH_2O$), 77.7 (C5), 87.4 ($Ar_3CO$), 118.5 (q, $J_{CF}$ = 319

Hz, $CF_3$), 127.5, 128.1, 128.5 (Ar), 136.1 (C4), 137.8 (C3), 142.8 (Ar), 163.6 (C2); **Schmp**.: 140.1°C (unter Verfärbung nach gelb).

**43. Herstellung von 5-Trityloxymethyl-3-vinyl-5*H*-furan-2-on (47a)**

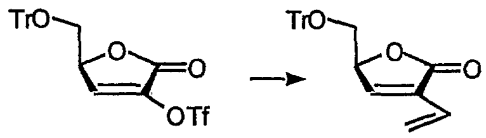

**46** **47a**

37 mg $Pd_2(dba)_3.CHCl_3$ (0.040 mmol) und 86 mg Triphenylarsin (0.28 mmol) wurden unter Argonatmosphäre in 10 ml trochenem THF gelöst. Nach Bildung des orange-roten Tetrakis(triphenylarsin)palladiumkomplexes wurden 1.01 g Lithiumchlorid (24.1 mmol) und 2.55 g Tributylvinylstannan (8.04 mmol) zugesetzt. Zu dieser Mischung wurde eine Lösung von 4.05 g des Triflates 46 (8.04 mmol) in 15 ml abs. THF während eines Zeitraumes von 15 min. getropft. Nach 10 min. wurden 2.43 g Caesiumfluorid (16 mmol) zugesetzt. Das Gemisch wurde für 2 h gerührt, anschließend mit Essigsäureethylester in einen Scheidetrichter überführt und mit 50 ml halbkonzentrierter Natriumchloridlösung gewaschen. Nach Trennung der Phasen wurde die organische Phase mit gesättigter Natriumchloridlösung gewaschen. Die wäßrigen Phasen wurden zweimal mit Essigsäureethylester reextrahiert und die vereinigten organischen Phasen über Natriumsulfat getrocknet. Das Produkt wurde durch Säulenchromatographie an Kieselgel mit Cyclohexan/Essigsäureethylester (12/1) gereinigt. Laut NMR enthielt das Produkt (2.99g) noch 14% Tributylchlorstannan (Ausbeute an **47a:** 87%), wurde aber wie nachfolgend beschrieben ohne weitere Reinigung mit Dieisennonacarbonyl komplexiert. Zur Charakterisierung wurde eine analytische Probe des Produktes **47a** aus Cyclohexan/Essigsäureethylester (15/1) umkristallisiert. $[\alpha]_D^{20}$ = -41.0 (c= 1.0 in $CHCl_3$); **$^1$H-NMR** (250 MHz, $CDCl_3$): $\delta$ = 3.35 (dd, $J_{AB}$ = 5.3 Hz, *J*= 5.0 Hz, 1H, $CH_2O$), 4.98 (m, *J*= 5.0, 1.7 Hz, 1H, H-C5), 5.48 (dd, *J*= 2.0, 10.8 Hz, 1H, C*H*H=CH), 6.27 (dd, *J*= 2.0, 17.7 Hz, 1H, CH*H*=CH), 6.42 (dd, *J*= 10.8, 17.7 Hz, 1H, $CH_2$=C*H*), 7.09 (d, *J*= 1.5 Hz, 1H, H-C4), 7.22-7.32 und 7.38-7.43 (m, 15H, ArH); **$^{13}$C-NMR** (62.5 MHz, $CDCl_3$): $\delta$ = 63.9 ($CH_2O$), 79.6 (C5), 87.0 ($Ar_3CO$), 121.6 (*C*$H_2$CH), 125.3 ($CH_2$CH), 127.3, 128.0, 128.6 (Ar), 130.5 (C3), 143.3 (Ar), 146.0 (C4), 171.3 (C2); **FT-IR** 3056, 3020, 2918, 2868, 1963, 1757, 1594, 1488, 1447, 1407, 1345, 1272, 1218, 1152, 1103, 1074, 1031, 1013, 990, 933, 910, 870, 796, 767, 746, 731, 705; **MS** (EI, 70 eV): 382 (5) $[M]^+$, 243 (100), 183 (9), 165 (57), 105 (31), 77 (25), 67 (12), 53 (17); **HR-MS** (EI, 70 eV): berechnet für $C_{26}H_{22}O_3$: 382.157; gefunden: 382.157; **Schmp.:** 94.2°C.

**44. Herstellung von [(1",2",3,4-$\eta$)-5-Trityloxymethyl-3-vinyl-5*H*-furan-2-on]-tricarbonyleisen (48a)**

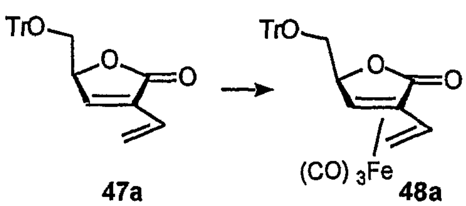

**47a** **48a**

Zu einer Lösung von 102 mg **47a** (0.267 mmol) in 5 ml trockenem THF wurden unter Argonatmosphäre 215 mg $Fe_2(CO)_9$ (0.590 mmol) gegeben. Es wurde für zwei Stunden bei Raumtemperatur gerührt und dann für eine Stunde zum Rückfluß erhitzt. Anschließend wurden erneut 215 mg $Fe_2(CO)_9$ zugesetzt, es wurde für eine Stunde bei Raumtemperatur gerührt und für eine weitere zum Rückfluß erhitzt. Dann wurde an der Luft durch Kieselgel filtriert, das Lösungsmittel am Rotationsverdampfer entfernt und der Rückstand säulenchromatographisch gereinigt. Dazu wurde zunächst mit Cyclohexan (CyHex) das als Nebenprodukt entstandene $Fe_3(CO)_{12}$ eluiert, anschließend das Produkt mit CyHex/EtOAc (20:1). Nach Entfernen des Lösungsmittels am Rotationsverdampfer wurden 50 mg **48a** (0.096 mmol, 36%) als gelber Feststoff erhalten.

**$^1$H-NMR** (250 MHz, $CDCl_3$): δ = 0.11 (dd, *J*= 2.2, 9.5 Hz, 1H, C*H*H=CH), 1.64 (dd, *J*= 2.2, 6.8 Hz, 1H, CH*H*=CH), 2.05 (d, *J*= 0.8 Hz, 1H, H-C4), 3.26 (dd, 4.1, 10.3 Hz, 1H, CH*H*O), 3.44 (dd, *J*= 4.1 Hz, 10.3 Hz, 1H, C*H*HO), 4.30 (ψt, 1H, *J* = 4.1 Hz, 1H, H-C5), 6.24 (dd, *J*= 6.8, 9.5 Hz, 1H, CHH=C*H*), 7.23 bis 4.41 (m, 15H, ArH).

**45. Herstellung von [(1",2",3,4-η)-5-Trityloxymethyl-3-vinyl-2,5-dihydrofuran-2-ol]-tricarbonyleisen (49a)**

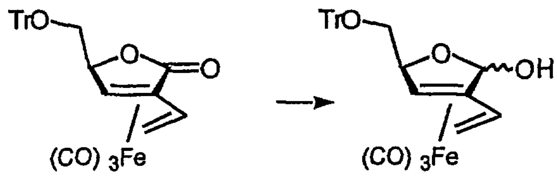

**48a** **49a**

Unter Argonatmosphäre wurden bei -78°C zu einer Lösung von 332 mg **48a** (0.636 mmol) in 20 ml absolutem Toluol 770 µl einer 1 M DIBAL-H-Lösung in Toluol (0.77 mmol) getropft. Die gelbe Lösung wurde für zweieinhalb Stunden bei dieser Temperatur gerührt. Anschließend wurde mit einigen Tropfen Aceton versetzt, im Scheidetrichter auf 0.5 M Weinsäurelösung gegossen und mit EtOAc extrahiert. Die organischen Phasen wurden mit ges. NaCl-Lösung gewaschen und über $Na_2SO_4$ getrocknet. Säulenchromatographie an Kieselgel mit CyHex/EtOAc (15:1) lieferte das Produkt **49a** in Form eines gelben Öles als Diastereomerengemisch (NMR) in einer Ausbeute von 235 mg (0.449 mmol, 71%). Dieses Gemisch wurde direkt für die nachfolgend beschriebene Transformation eingesetzt.

**46. Herstellung von [1''',2''',3',4'-η-(5-Methoxy-4-vinyl-2,5-dihydrofuran-2-yl)-methanol]-tricarbonyleisen (50a)**

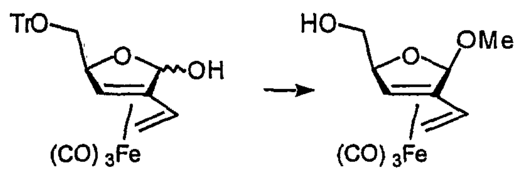

**49a** **50a**

Unter Argonatmosphäre wurde eine Lösung von 90 mg **49a** (0.305 mmol) in 15 ml absolutem MeOH mit 12 mg *p*-Toluolsulfonsäuremonohydrat (0.061 mmol) und 130 µl *o*-Ameisensäuretrimethylester (130 mg, 1.22 mmol) versetzt und für fünf Stunden bei Raumtemperatur gerührt. Anschließend wurde mit zwei Tropfen Pyridin versetzt und das Lösungsmittel am Rotationsverdampfer entfernt. Der Rückstand wurde in CyHex:EtOAc (3.5:1) aufgenommen und durch Kieselgel filtriert. Nach Entfernen des Lösungsmittels wurde das Produkt **50a** als gelber Feststoff (89 mg, 99%) erhalten.
**$^1$H-NMR** (250 MHz, $CDCl_3$): δ = -0.01 (dd, *J*= 2.2, 8.8 Hz, 1H, C*H*H=CH), 1.68 (dd, *J*= 2.2, 6.6 Hz, 1H, CH*H*=CH), 1.73 (s, 1H, H-C4'), 2.83 (dd, J 2.5, 10.2 Hz, 1H, OH), 3.36 bis 3.59 (m, 4H, C*H*HO und $CH_3O$), 3.75 (dt, 2.6, 12.1 Hz, 1H, CH*H*O), 4.33 (ψt, 1H, *J*= 2.9, 8.1 Hz, 1H, H-C5'), 5.53 (s, 1H, H-C2'), 5.59 (dd, *J*= 6.6, 8.8 Hz, 1H, CHH=C*H*).

**47. Hersetllung von [1''',2''',3,4-η-5-(Dimethyl-(1,1,2-trimethylpropyl)-silanyloxymethyl)-2-methoxy-3-vinyl-2,5-dihydrofuran]-tricarbonyleisen (51a)**

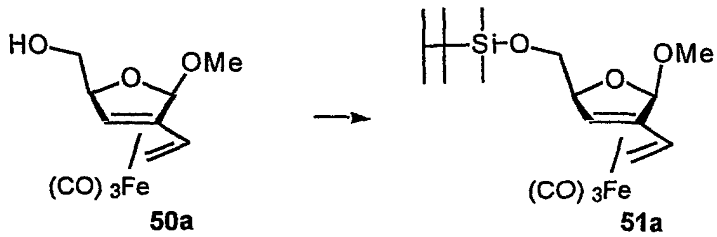

50a 51a

Unter Argonatmosphäre wurden zu einer Lösung von 87 mg **50a** (0.294 mmol) und 100 mg Imidazol (1.47 mmol) in 3 ml absolutem Dichlormethan 90 µl Thexyldimethylsilylchlorid (80 mg, 0.44 mmol) getropft. Die gelbe Lösung wurde über Nacht bei Raumtemperatur gerührt. Anschließend wurde mit Dichlormethan verdünnt, mit Wasser versetzt und eine weitere Stunde gerührt. Nach Phasentrennung wurde die wäßrige Phase zweimal mit Dichlormethan reextrahiert. Nach Trocknung der vereinigten organischen Phasen über $Na_2SO_4$ wurde das Produkt **51a** durch Chromatogtaphie an Kieselgel mit CyHex:EtOAc (15:1) mit einer Ausbeute von 124 mg (0.283 mmol, 96%) als gelbes Öl erhalten.

**$^1$H-NMR** (250 MHz, $CDCl_3$): δ= -0.08 (dd, *J* = 2.2, 8.8 Hz, 1H, C*H*H=CH), 0.08 und 0.09 (je s, 6H, $Si(CH_3)_2$), 0.81 (s, 6H, $SiC(CH_3)_2$), 0.82 (d, *J* = 6.8 Hz, 6H, CH(C$H_3$)$_2$), 1.57 (sept, *J* = 6.8 Hz, 1H, C*H*($CH_3$)$_2$), 1.64 (dd, *J* = 2.2, 6.6 Hz, 1H, CH*H*=CH), 1.99 (s, 1H, H-C4), 3.44 (dd, *J* = 8.1 Hz, 9.7 Hz, 1H, C*H*HO), 3.74 (dd, 5.9, 9.7 Hz, 1H, CH*H*O), 4.09 (dd, 1H, J = 5.9, 8.1 Hz, 1H, H-C5), 5.52 (s, 1H, H-C2), 5.59 (dd, *J* = 6.6, 8.8 Hz, 1H, CHH=C*H*).

**48. Herstellung von [4-Amino-1-{η$^4$-5-[dimethyl-(1,1,2-trimethylpropyl)-silanyloxymethyl]-3-vinyl-2,5-dihydrofuran-2-yl}-3,4-dihydro-1*H*-pyrimidin-2-on]-tricarbonyleisen (52a)**

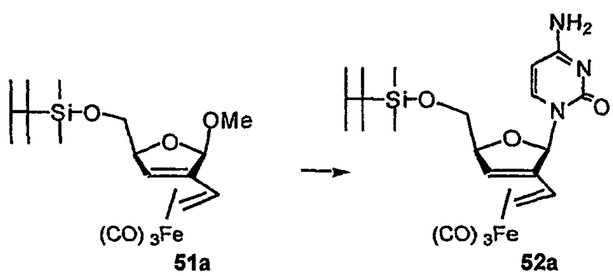

51a 52a

Unter Argonatmosphäre wurden zu einer Lösung von 117mg **51a** (0.267 mmol) und 275 mg 2,4-Bis-trimethylsiloxycytosin (1.07 mmol) in 6 ml trockenem Dichlormethan 290 µl Trimethylsilyltriflat (356 mg, 1.60 mmol) getropft. Die gelbe Lösung wurde für vier Stunden bei Raumtemperatur gerührt und anschließend zu 20 ml ges. $NaHCO_3$-Lösung getropft. Nach Trennung der Phasen wurde die wäßrige Phase zweimal mit Dichlormethan extrahiert, die vereinigten organischen Phasen wurden mit ges. NaCl-Lösung gewaschen und über $Na_2SO_4$ getrocknet. Das Produkt (52a) wurde nach Säulenchromatographie an Kieselgel mit EtOAc/MeOH (30:1) in einer Ausbeute von 116 mg (0.224 mmol, 84%) als hellgelber Feststoff erhalten.

**$^1$H-NMR** (250 MHz, $CDCl_3$): δ = -0.16 (dd, *J*= 2.2, 8.9 Hz, 1H, C*H*H=CH), 0.08 und 0.09 (je s, 6H, $Si(CH_3)_2$), 0.81 (s, 6H, $SiC(CH_3)_2$), 0.82 (d, *J*= 6.8 Hz, 6H, CH(C$H_3$)$_2$), 1.57 (sept, *J* = 6.8 Hz, 1H, C*H*($CH_3$)$_2$), 1.68 (dd, *J*= 2.2, 6.7 Hz, 1H, CH*H*=CH), 1.85 (s, 1H, H-C4'), 3.72 (dd, $J_{AB}$ = 14.1 Hz, *J* = 4.5 Hz, 1H, C*H*HO), 3.78 (dd, $J_{AB}$ = 14.1 Hz, *J* = 4.1 Hz, 1H, CH*H*O), 4.30 (ψt, 1H, H-C5'), 5.73 (d, *J* = 7.4 Hz, 1H, H-C5), 6.06 (ψt, 1H, CHH=*H*), 7.00 (s, 1H, H-C2'), 7.98 (d, *J* = 7.4 Hz, 1H, H-C6); **FT-IR** 3343, 3104, 2955, 2046, 1968, 1660, 1632, 1484, 1400, 1276, 1250, 1170, 1108, 1079, 1033, 830, 775, 730; MS (ESI, 70 eV): 540 (100) [*M*-Na]$^+$, 518 (41) [*M*-H]$^+$, 407 (76), 276 (39); **HR-MS** (ESI, 70 eV): berechnet für $C_{22}H_{31}FeN_3O_6Si$-H: 518.141; gefunden: 518.140.

Literatur:

Y. A. Hannun (1997), Apoptosis and the dilemma of cancer therapy, Blood 89, 1845-1853.

J. A. Hickman (1996), Apoptosis and chemotherapy resistance, Eur. J. Cancer 32A, 921-926.

M. Raisova, M. Bektas, T. Wieder, P. T. Daniel, J. Ebene, C. E. Orfanos, C. C. Geilen (2000), Resistance to CD95/Fas-induced and ceramide-mediated apoptosis of human melanoma cells is caused by a defective mitochondrial cytochrome c release, FEBS Lett. 473, 27-32.

G. M. Cohen (1997), Caspases: the executioners of apoptosis, Biochem. J. 326, 1-16.

F. Eßmann, T. Wieder, A. Otto, E.-C. Müller, B. Dörken, P. T. Daniel (2000), The GDP dissociation inhibitor, D4-GDI (Rho-GDI 2), but not the homologous Rho-GDI 1, is cleaved by caspase-3 during drug-induced apoptosis, Biochem. J. 346, 777-783.

E. Heßler, H.-G. Schmalz, G. Dürner (1994), Chiral Butadiene-$Fe(CO)_3$ Complexes for Organic Synthesis. Reactions of ($\eta^4$-2-Alkoxy-4-vinyl-2,5-dihydrofuran)-$Fe(CO)_3$ Derivatives, Tetrahedron Lett. 35, 4547-4550.

N. Rehnberg, G. Magnusson (1990), Chiral Aldehydes by Ring Contraction of Pento- and Hexapyranoside Epoxides, J. Org. Chem. 55, 5467 - 5476.

T. Wieder, C. Perlitz, M. Wieprecht, R. T. C. Huang, C. C. Geilen, C. E. Orfanos (1995), Two new sphingomyelin analogues inhibit phosphatidylcholine biosynthesis by decreasing membranebound CTP: phosphocholine cytidylyltransferase levels in HaCaT cells, Biochem. J. 311, 873-879.

T. Wieder, F. Eßmann, A. Prokop, K. Schmelz, K. Schulze-Osthoff, R. Beyaert, B. Dörken, P. T. Daniel (2001), Activation of caspase-8 in drug-induced apoptosis of B-lymphoid cells is independent of CD95/Fas receptor-ligand interaction and occurs downstream of caspase-3, Blood 97, 1378-1387.


Figuren:

Figur 1:
Proapoptotische Wirkung der Nukleosidanaloga N76 und N69 in malignen lymphoblastischen Zellen. Die Präparation von N69 enthielt Verunreinigungen durch ein Isomer.

Figur 2:
N76-induzierte Aktivierung der Caspase-3 in malignen lymphoblastischen Zellen.

Figur 3 :
Proapoptotische Wirkung des Nukleosidanalogons N69 in primären Zellen von Patienten mit verschiedenen bösartigen Erkrankungen des blutbildenden Systems. Die Präparation von N69 enthielt Verunreinigungen durch ein Isomer.

Figur 4:
Proapoptotische Wirkung der Nukleosidanaloga N69 und N76 in primären Patientenzellen im Vergleich mit etablierten Zytostatika.

Figur 5 :
Einfluß des komplexierten Eisens auf die antiproliferative und Zelltodeinleitende Wirkung von N69.

Figur 6:
Das carbozyklische Nukleosidanalogon JV-206-1 (Strukturformel 35) induziert Zelltod in BJAB Zellen über den mitochondrialen Apoptosesignalweg.

## Patentansprüche

1. Arzneimittel, enthaltend mindestens eine Verbindung der Strukturformel **1a** oder **1b**:

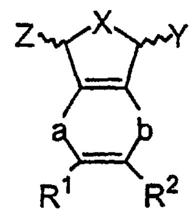

**1a**

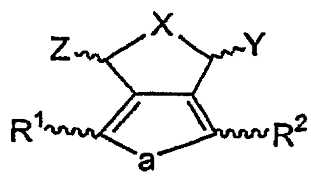

**1b**

mit

X = O, S, $CH_2$, NR, keine Bindung,

mit der Maßgabe, dass wenn X = O ist, die Verbindung der Formel 1a mit einem Metall-Ligandenkomplex assoziiert ist gemäß Formel 1'a;

Y = Adenin, Cytosin, Guanin, Uracil, Thymin, Bromuracil, Purine oder Pyrimidine sowie deren Derivate, Nucleobasen, Heterozyklen, Aminoalkyl, Aminoaryl oder sonstige zur Ausbildung von H-Brücken befähigte Reste;

Z = Alkyl, Fluoralkyl, Aryl, Fluoraryl, $-CH_2-OR'$, $-(CH_2)_nOR'$ mit R' = H, $SiR_3$, Alkyl; Fluoralkyl, Aryl, Fluoraryl, Acyl und n = 0 bis 5;

a = keine Bindung, Einfachbindung, $CH_2$, bei Strukturformel **1b** auch $CH_2CH_2$ ;

b = keine Bindung, Einfachbindung oder $CH_2$; wobei a und b so ausgewählt werden, dass die a und b umfassende Struktureinheit ein 1,3-Dien beinhaltet,

$R^1$ = H, F, Alkyl, Fluoralkyl, Aryl, Fluoraryl, OR, $-CO_2R$, $-SO_2OR$, $-CONR_2$,

$R^2$ = H, F, Alkyl, Fluoralkyl, Aryl, Fluoraryl, OR, $-CO_2R$, $-SO_2OR$, $-CONR_2$,

R= H, Aryl, verzweigte und geradkettige Alkyl, insbesondere Methyl, Ethyl, Propyl, Thexyl, tert-Butyl

und/oder deren Salze.

2. Arzneimittel nach Anspruch 1, **dadurch gekennzeichnet, dass** es die Strukturformel **1'a** oder **1'b**

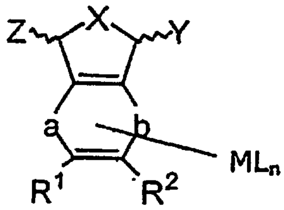

**1'a**

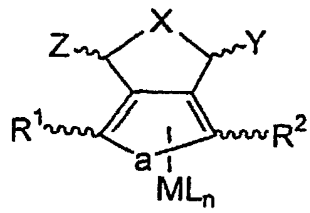

**1'b**

aufweist, wobei n eine ganze Zahl von 2 bis 4 ist,

M = Mn, Fe, Co, Ru und
L = CO, CN-R, CN, $CR_2$, COR, Halogen, Cylopentadienyl (Cp) oder ein substituiertes Cp-Derivat ist,

und an ein M gleiche oder verschiedene L gebunden sein können.

**3.** Arzneimittel nach Anspruch 2, **dadurch gekennzeichnet, dass** die mindestens eine Verbindung der Strukturformel **1'a** oder **1'b** die Strukturformel **2, 3, 4a, 4b** oder **4c**

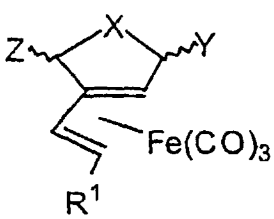

**2**

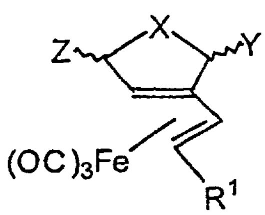

**3**

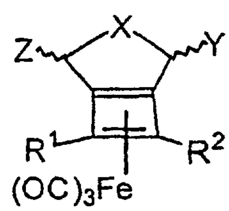

**4a**

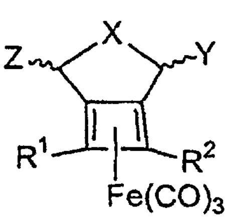

**4b**

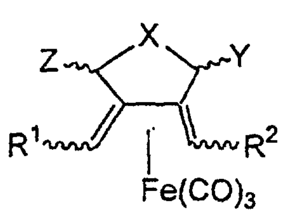

**4c**

aufweist, wobei die $Fe(CO)_3$-Einheit $\eta^4$-gebunden ist,
X = O, $CH_2$ oder keine Bindung ist
und Y, Z, $R^1$ und $R^2$ wie in Anspruch 1 ausgewählt werden.

**4.** Arzneimittel nach Anspruch 3, **dadurch gekennzeichnet, dass** die mindestens eine Verbindung der Strukturformel **2** die Strukturformel **5**

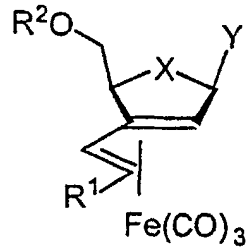

**5**

aufweist, wobei X = O, $CH_2$,

Y = Adenin, Cytosin, Guanin, Uracil, Thymin, Bromuracil, Purine oder Pyrimidine sowie deren Derivate, Nucleobasen, Heterozyklen,
$R^1$ = -H, $-CO_2R$,
$R^2$ = H, $SiR_3$, Alkyl, Fluoralkyl, Aryl, Fluoraryl, Acyl ist,

wobei R wie in Anspruch 1 ausgewählt wird.

**5.** Verbindungen der Strukturformel 5 nach Anspruch 4, **dadurch gekennzeichnet, dass**

$R^2$ = $SiR_3$, X = O,

Y und $R^1$ wie in Anspruch 4 und R wie in Anspruch 1 ausgewählt sind, mit der Maßgabe, dass

wenn Y Bromuracil, Uracil oder Methyluracil ist, $R^2$ nicht $Thexyl(CH_3)_2Si$ ist.

**6.** Verbindungen nach Anspruch 5, ausgewählt aus der Gruppe bestehend aus:

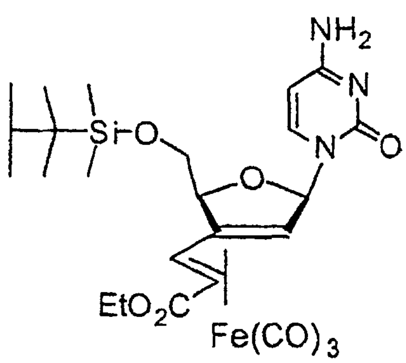

**6**

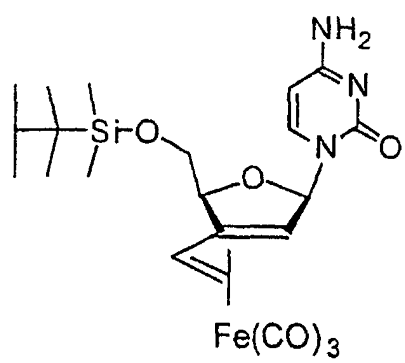

**7**

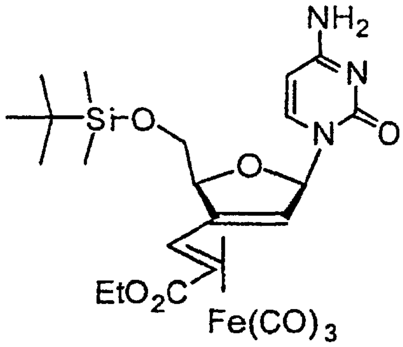
8

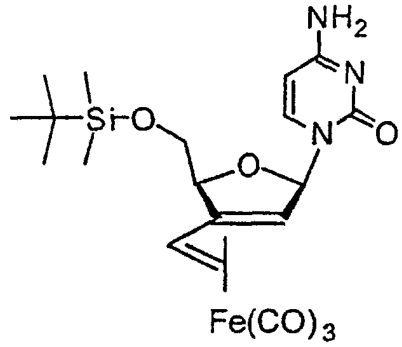
9

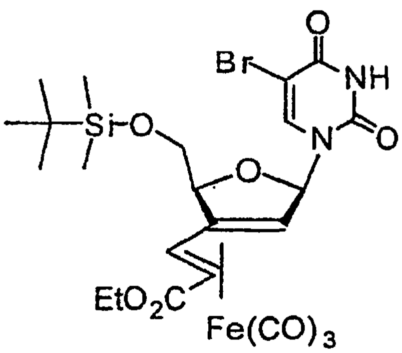
10

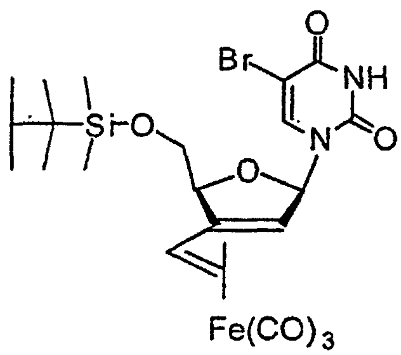
11

7. Verbindung der Strukturformel 5 nach Anspruch 4, ausgewählt aus der Gruppe bestehend aus den Verbindungen **44a, 44b, 44c** und **44d;** und Verbindungen der Strukturformel **1a** nach Anspruch 1, ausgewählt aus der Gruppe bestehend aus den Verbindungen **43a, 43b, 43c** und **43d,** mit den Strukturformeln

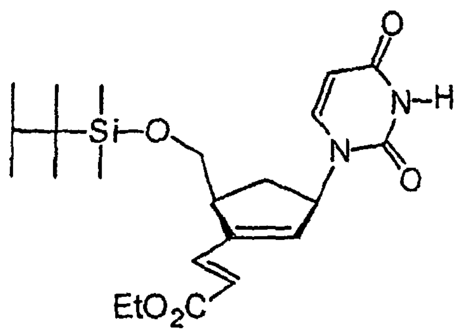
43a

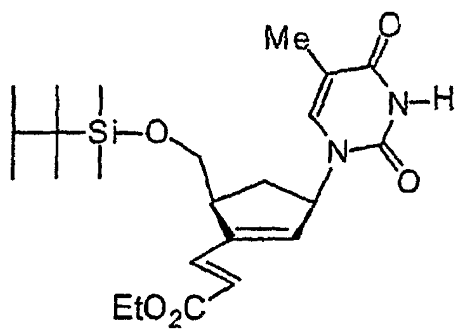
43b.

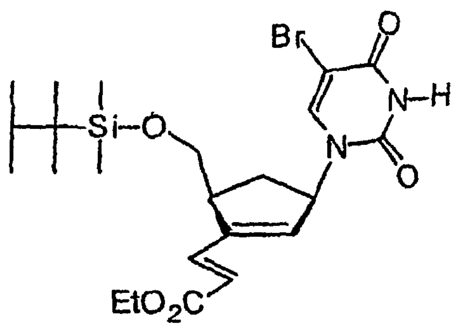

**43c**

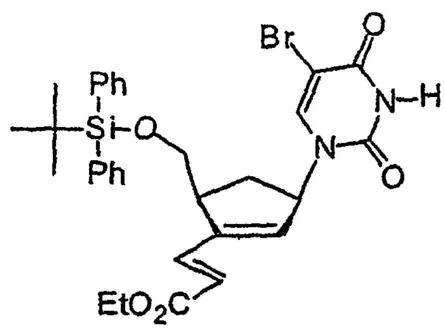

**43d**

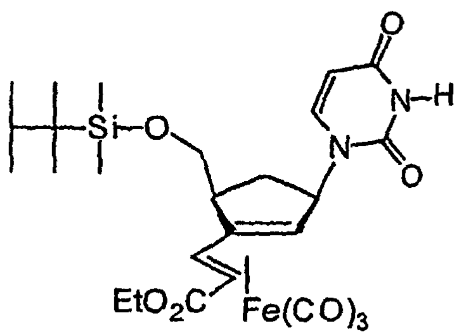

**44a**

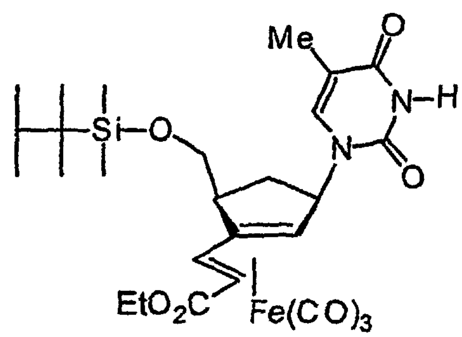

**44b**

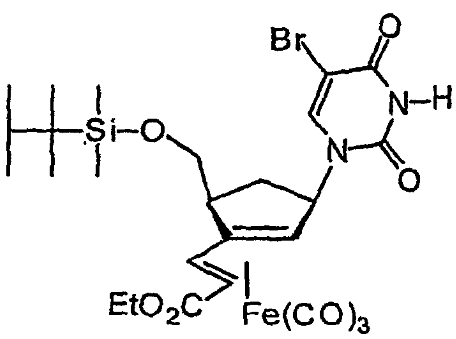

**44c = 35**

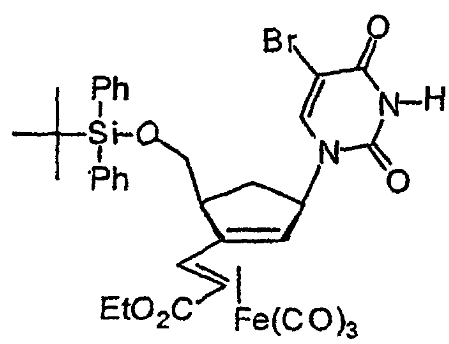

**44d**

**8.** Verwendung von Verbindungen nach einem der Ansprüche 1 bis 7 und/oder Verbindungen der Strukturformel **1a**

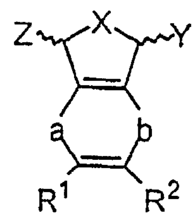

**1a**

wobei

X = O, S, $CH_2$, NR oder keine Bindung ist und

Y, Z, a, b, $R^1$ und $R^2$ wie in Anspruch 1 ausgewählt werden,

zur Herstellung eines Arzneimittels zur Behandlung maligner Erkrankungen des Knochenmarks oder anderer blutbildender Organe, solider Tumoren, epithelialer Tumoren, gutartiger oder semimaligner schnell proliferierender Tumore oder Hauterkrankungen, insbesondere Psoriasis vulgaris, Keloide und Basaliome, Lymphome, insbesonder Hodgkin- und Non-Hodgkin-Lymphome, entzündlicher, chronisch entzündlicher, bakterieller und autoimmuner Erkrankungen, zur antibakteriellen, antimykotischen, anti-Protozoen, anti-Plasmodien anti-helminthischen oder immunsuppressiven Therapie und/oder zur Auslösung der Apoptose.

**9.** Verfahren zur Synthese von Verbindungen nach Anspruch 5, 6 und/oder Anspruch 7, **dadurch gekennzeichnet, dass** es den Reaktionsschritt einer diaastereoselektiven, Eisen-unterstützten nucleophilen Substitution, vorzugsweise unter Verwendung silylierter Nucleobasen in Gegenwart einer Lewissäure umfaßt, und dass bei diesem Reaktionsschritt eine Ausgangssubstanz, die an der wie in Strukturformel 5 lokalisierten Y-Position eine veretherte oder veresterte Hydroxygruppe aufweist, umgesetzt wird, so dass an Stelle dieser Hydroxygruppe eine Nucleobase eingeführt wird.

## Claims

**1.** A medicament containing at least one compound of structural formula 1a or 1b:

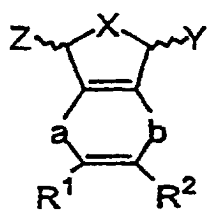

1a

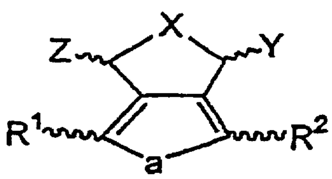

1b

wherein

X = O, S, $CH_2$, NR, no bond;

with the proviso that, if X = O, then the compound of formula 1a is associated with a metal-ligand complex according to formula 1'a;

Y = adenine, cytosine, guanine, uracil, thymine, bromouracil, purines or pyrimidines and their derivatives, nucleobases, heterocycles, aminoalkyl, aminoaryl or other residues capable of forming hydrogen bonds;

Z = alkyl, fluoroalkyl, aryl, fluoroaryl, $-CH_2-OR'$, $-(CH_2)_nOR'$ with R' = H, $SiR_3$, alkyl, fluoroalkyl, aryl, fluoroaryl, acyl, and n = 0 to 5;

a = no bond, single bond, $CH_2$, for structural formula 1b also $CH_2CH_2$;

b = no bond, single bond or $CH_2$, a and b being selected such that the moiety comprising a and b includes a 1,3-diene;

$R^1$ = H, F, alkyl, fluoroalkyl, aryl, fluoroaryl, OR, $-CO_2R$, $-SO_2OR$, $-CONR_2$;

$R^2$ = H, F, alkyl, fluoroalkyl, aryl, fluoroaryl, OR, $-CO_2R$, $-SO_2OR$, $-CONR_2$;

R = H, aryl, branched and linear alkyl, especially methyl, ethyl, propyl, thexyl, tert-butyl;
and/or their salts.

2. The medicament according to claim 1, **characterized by** having the structural formula 1'a or 1'b:

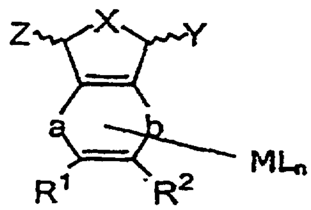

**1´a**

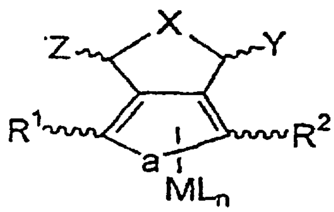

**1´b**

wherein n is an integer of from 2 to 4;
M = Mn, Fe, Co, Ru; and
L = CO, CN-R, CN, $CR_2$, COR, halogen, cyclopentadienyl (Cp), or a substituted Cp derivative; and the same or different Ls may be bound to one M.

3. The medicament according to claim 2, **characterized in that** said at least one compound of structural formula 1'a or 1'b has the structural formula 2, 3, 4a, 4b or 4c:

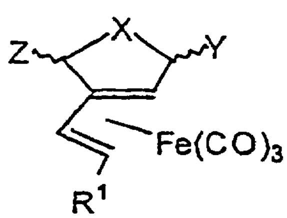

**2**

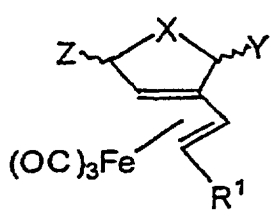

**3**

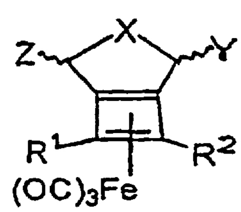

**4a**

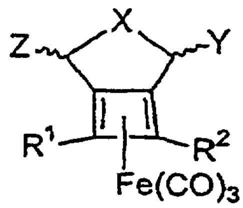

4b

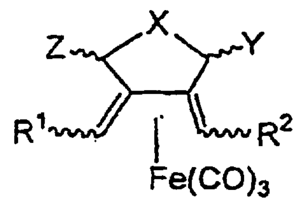

4c

wherein the $Fe(CO)_3$ unit is $\eta^4$-bound;
X = O, $CH_2$ or no bond; and
Y, Z, $R^1$ and $R^2$ are selected as in claim 1.

**4.** The medicament according to claim 2, **characterized in that** said at least one compound of structural formula 2 has the structural formula 5:

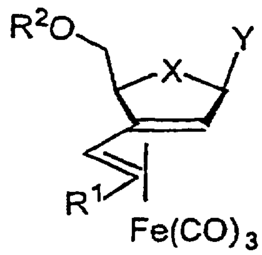

5

wherein X = O, $CH_2$;
Y = adenine, cytosine, guanine, uracil, thymine, bromouracil, purines or pyrimidines and their derivatives, nucleobases, heterocycles;
$R^1$ = -H, $-CO_2R$;
$R^2$ = H, $SiR_3$, alkyl, fluoroalkyl, aryl, fluoroaryl, acyl;
wherein R is selected as in claim 1.

**5.** The compounds of structural formula 5 according to claim 4, **characterized in that**
$R^2$ = $SiR_3$;
X = O;
Y and $R^1$ are selected as in claim 4, and R is selected as in claim 1, with the proviso that if Y is bromouracil, uracil or methyluracil, then $R^2$ is not thexyl$(CH_3)_2$Si.

**6.** The compounds according to claim 5, selected from the group consisting of:

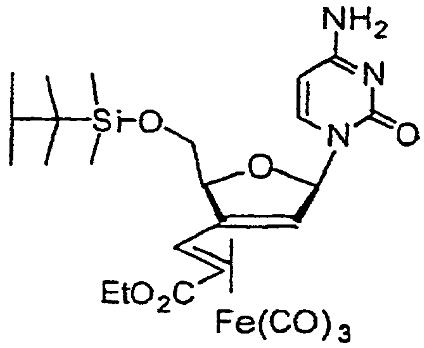

6

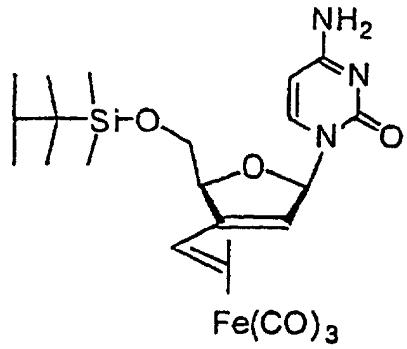

7

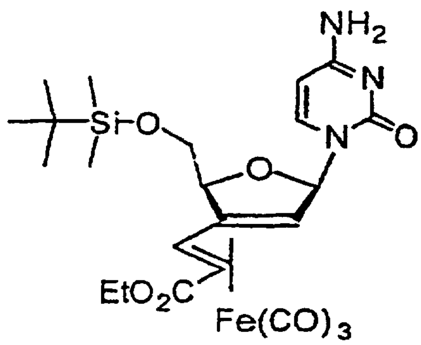

8

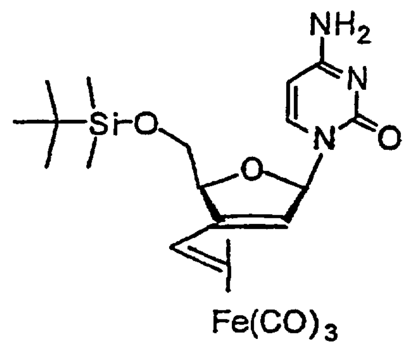

9

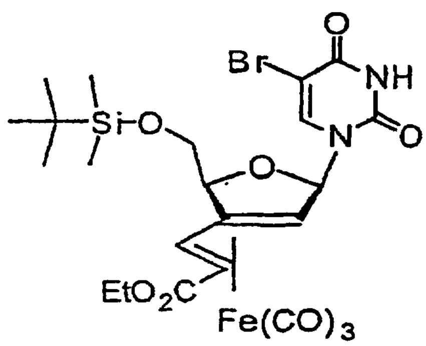

10

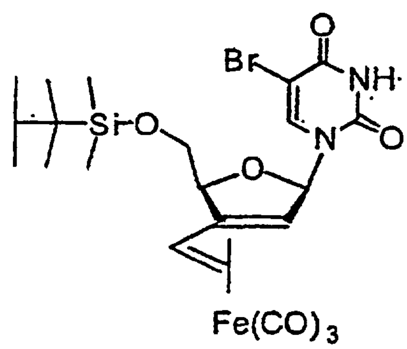

11

7. The compound of structural formula 5 according to claim 4, selected from the group consisting of compounds 44a, 44b, 44c and 44d; and compounds of structural formula 1a according to claim 1, selected from the group consisting of compounds 43a, 43b, 43c and 43d, having the structural formulas:

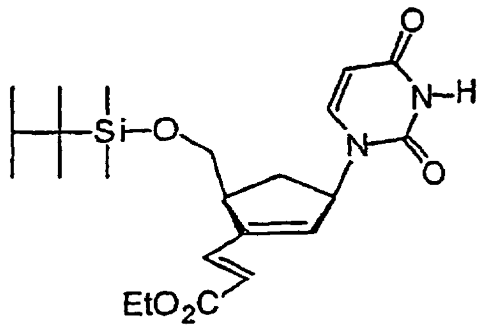

43a

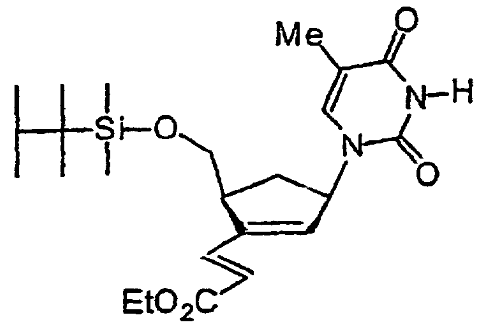

43b.

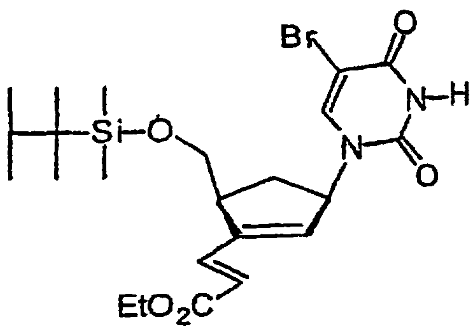

43c

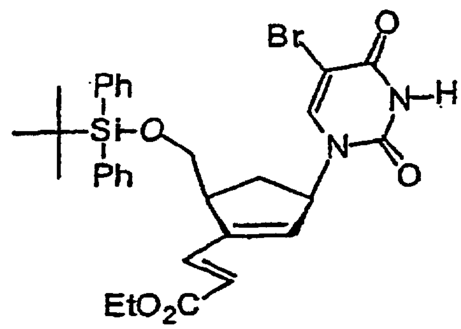

43d

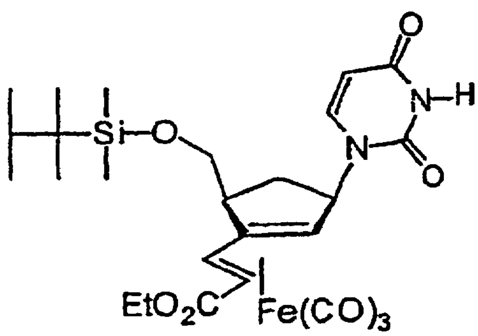

44a

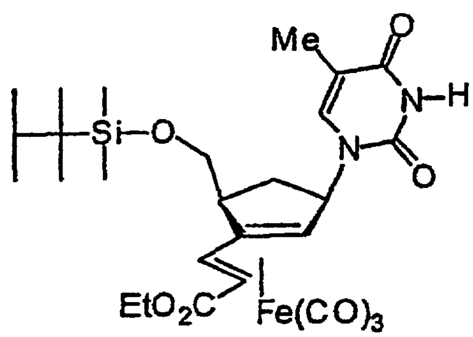

44b

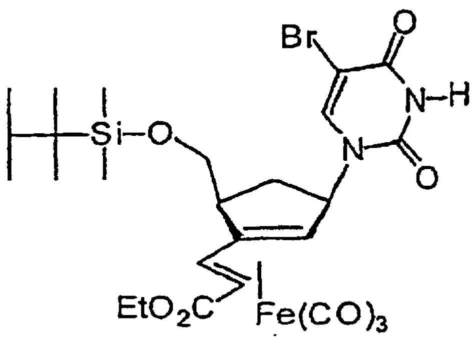

44c = 35

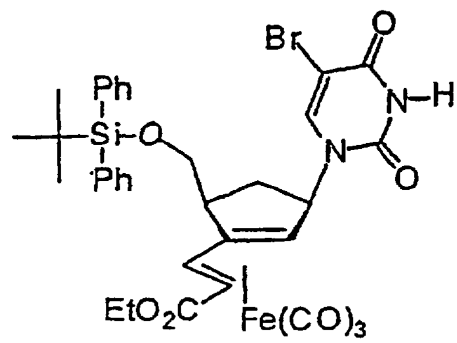

44d

**8.** Use of compounds according to any of claims 1 to 7 and/or compounds of structural formula 1a:

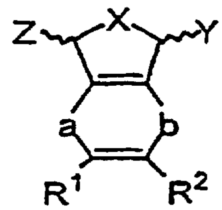

**1a**.

wherein
X = O, S, $CH_2$, NR or no bond; and
Y, Z, a, b, $R^1$ and $R^2$ are selected as in claim 1;
for the preparation of a medicament for treating malignant diseases of the bone marrow or other hematopoietic organs, solid tumors, epithelial tumors, benign or semimalignant fast proliferating tumors or skin diseases, especially psoriasis vulgaris, keloids and basaliomas, lymphomas, especially Hodgkin and non-Hodgkin lymphomas, inflammatory, chronic inflammatory, bacterial and autoimmune diseases, for antibacterial, antimycotic, anti-protozoan, anti-plasmodia, anti-helminthic or immunosuppressant therapies and/or for initiating apoptosis.

**9.** A process for the synthesis of compounds according to claims 5, 6 and/or claim 7, **characterized by** comprising the reaction step of a diastereoselective iron-supported nucleophilic substitution, preferably using silylated nucleobases in the presence of a Lewis acid, and in that a starting substance which has an etherified or esterified hydroxy group in the Y position localized as in structural formula 5 is reacted in this reaction step, so that a nucleobase is substituted for this hydroxy group.

## Revendications

**1.** Médicament contenant au moins un composé de formule développée **1a** ou **1b :**

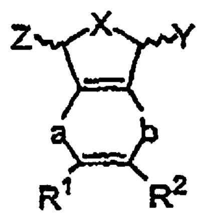

**1a**

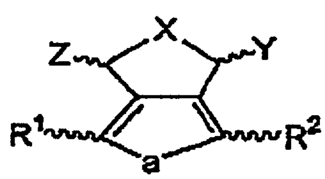

**1b**

dans lesquelles

X = O, S, $CH_2$, NR, aucune liaison,
sous réserve que si X = O, le composé de formule la est associé à un complexe métal-ligands selon la formule

**1'a**,
Y = adénine, cytosine, guanine, uracile, thymine, bromouracile, purine ou pyrimidine ainsi que leurs dérivés, nucléobases, hétérocycles,
aminoalkyle, aminoaryle ou d'autres résidus aptes à former des ponts H,
Z = alkyle, fluoroalkyle, aryle, fluoroaryle, $-CH_2-OR'$, $-(CH_2)_n-OR'$ dans lesquels R' = H, $SiR_3$, alkyle, fluoroalkyle, aryle, fluoroaryle, acyle et n= 0 à 5,
a = aucune liaison, simple liaison, $CH_2$, et aussi, pour la formule développée **1b,** $CH_2CH_2$,
b = aucune liaison, simple liaison, $CH_2$, et ce en sélectionnant a et b de telle sorte que l'unité structurale comprenant a et b contienne un 1,3-diène,
$R^1$ = H, F, alkyle, fluoroalkyle, aryle, fluoroaryle, OR, $-CO_2R$, $-SO_2OR$, $-CONR_2$,
$R^2$ = H, F, alkyle, fluoroalkyle, aryle, fluoroaryle, OR, $-CO_2R$, $-SO_2OR$, $-CONR_2$
R = H, aryle, alkyle ramifié et linéaire, notamment méthyle, éthyle, propyle, thexyle, tert-butyle,

et/ou leurs sels.

2. Médicament selon la revendication 1, **caractérisé en ce qu'**il présente la formule développée **1'a** ou **1'b**

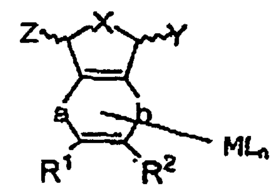

**1'a**

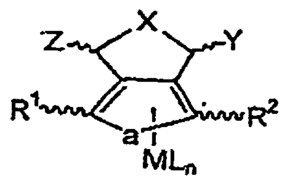

**1'b**

dans lesquelles n est un nombre entier de 2 à 4,

M = Mn, Fe, Co, Ru et
L = CO, CN-R, CN, $CR_2$, COR, halogène, cyclopentadiényle (Cp) ou est un dérivé de Cp substitué,

et des L semblables ou différents peuvent être liés à un M.

3. Médicament selon la revendication 2, **caractérisé en ce que** le au moins un composé de formule développée **1'a** ou **1'b** a la formule développée **2, 3, 4a, 4b ou 4c,**

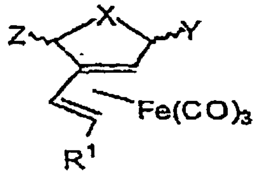

**2**.

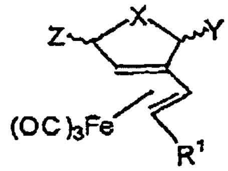

**3**

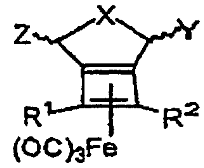

**4a**

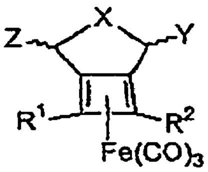

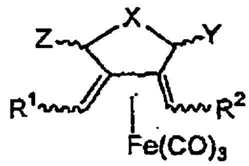

4b

4c

dans lesquelles l'unité $Fe(CO)_3$ est liée en $\eta^4$,

X = 0, $CH_2$ ou aucune liaison,
et Y, Z, $R^1$ et $R^2$ sont sélectionnés tel qu'il est indiqué dans la revendication 1.

**4.** Médicament selon la revendication 3, **caractérisé en ce que** le au moins un composé de formule développée **2** a la formule développée **5**

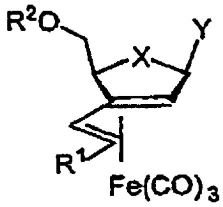

**5**

dans laquelle

X = O, $CH_2$,
Y = adénine, cytosine, guanine, uracile, thymine, bromouracile, purine ou pyrimidine ainsi que leurs dérivés, nucléobases, hétérocycles,
$R^1$ = -H, $-CO_2R$,
$R^2$ = H, $SiR_3$, alkyle, fluoroalkyle, aryle, fluoroaryle, acyle
R étant sélectionné comme il est indiqué dans la revendication 1.

**5.** Composés de formule développée 5 selon la revendication 4, **caractérisés en ce que**

$R^2$ = $SiR_3$, X = O,
Y et $R^1$ sont sélectionnés comme il est indiqué dans la revendication 4 et R tel qu'il est indiqué dans la revendication 1,

sous réserve que si Y est une bromouracile, uracile ou méthyluracile, $R^2$ n'est pas un thexyl$(CH_3)_2$Si.

**6.** Composés selon la revendication 5, sélectionnés parmi le groupe formé par les suivants :

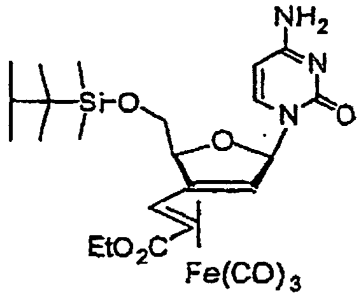

6

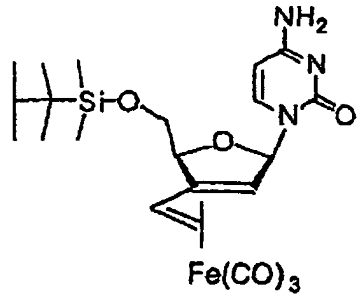

7

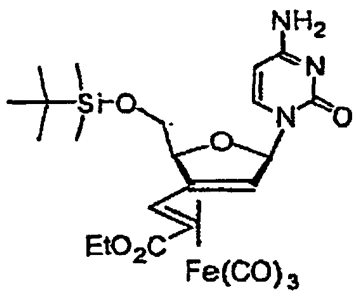

8

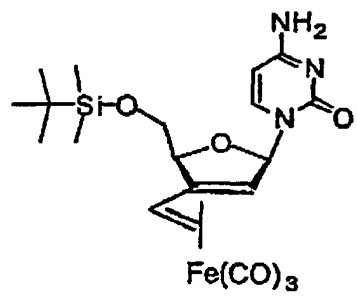

9

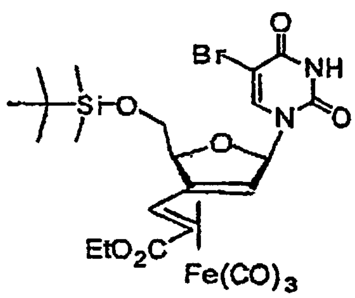

10

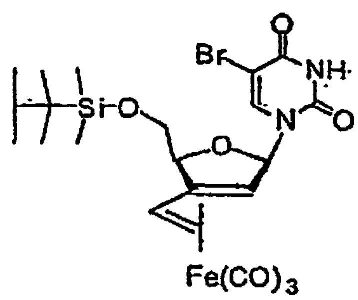

11

**7.** Composé de formule développée 5 selon la revendication 4, sélectionné parmi le groupe comprenant les composés **44a, 44b, 44c** et **44d** ; et composés de formule développée **1a** selon la revendication 1, sélectionnés parmi le groupe formé par les composés **43a, 43b, 43c et 43d,**

ayant les formules développées suivantes

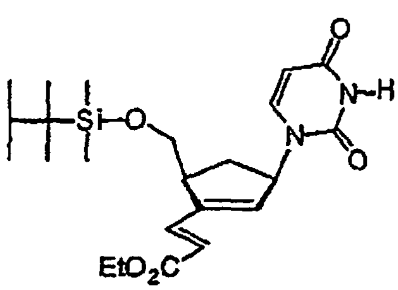

**43a**

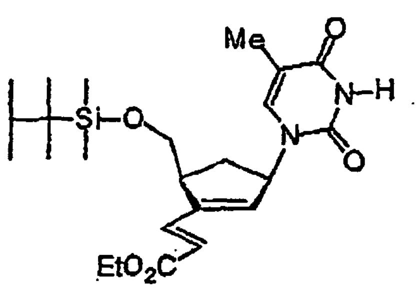

**43b.**

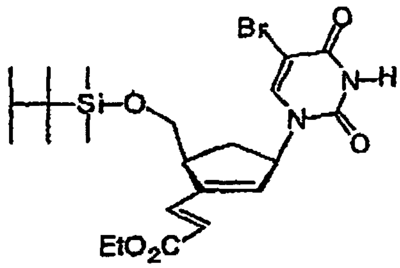
43c

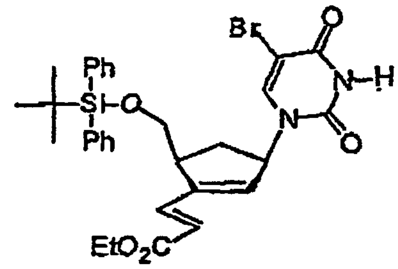
43d

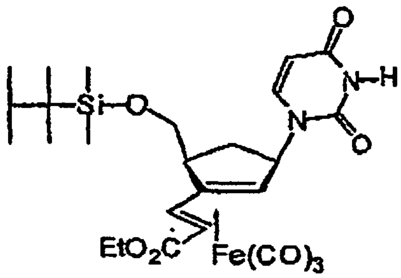
44a

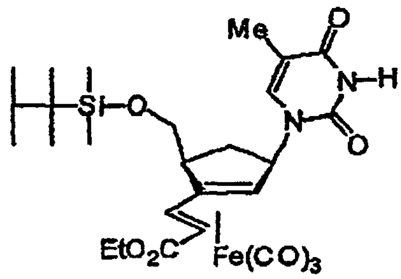
44b

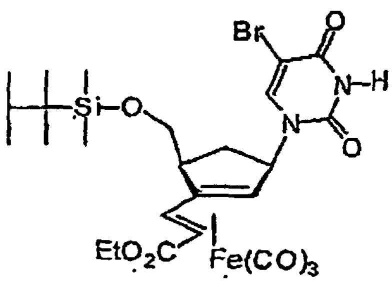
44c = 35

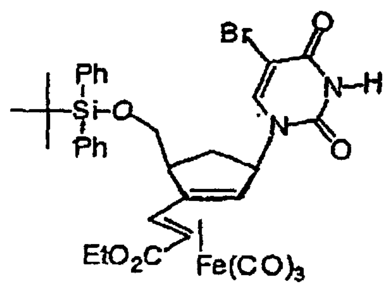
44d

**8.** Utilisation de composés selon une des revendications 1 à 7 et/ou de composés de formule développée **1a**

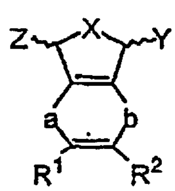
**1a**

dans laquelle

X = O, S, $CH_2$, NR ou ne représente aucune liaison et
Y, Z, a, b, $R^1$ et $R^2$ sont sélectionnés comme il est indiqué dans la revendication 1,

pour préparer des médicaments destinés au traitement des affections malignes de la moelle osseuse ou

d'autres organes hématopoïétiques, des tumeurs solides, des tumeurs épithéliales, des tumeurs ou des dermatoses bénignes ou semi-malignes à prolifération rapide, notamment du Psoriasis Vulgaris, de la chéloïde et de l'Ulcus Rodens, des lymphomes, notamment des lymphomes malins hodgkiniens ou non hodgkiniens, des maladies inflammatoires, bactériennes et auto-immunes chroniques, pour une thérapie antibactérienne, antifongique, anti-protozoaires, anti-plasmodies, antihelminthique ou immunodépressive et/ou pour déclencher l'apoptose.

**9.** Procédé de synthèse des composés selon la revendication 5, 6 et/ou 7, **caractérisé en ce qu'**il comprend une étape de réaction consistant en une substitution nucléophile diastéréosélective soutenue par du fer, de préférence en utilisant des nucléobases silylées en présence d'un acide de Lewis, et qu'au cours de cette étape de réaction, une substance de départ, comportant un groupe hydroxy éthérifié ou estérifié en position Y localisée comme dans la formule développée **5**, est transformée de manière à introduire à la place de ce groupe hydroxy une nucléobase.

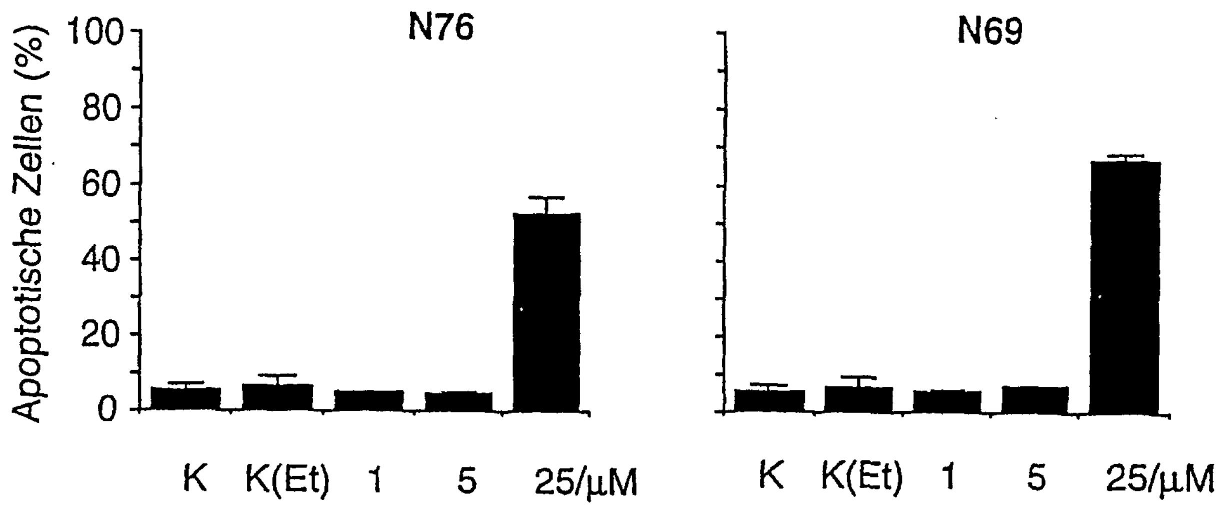


## B) Patientenzellen (Kinder mit ALL)

**Patient 1**

**Patient 2**

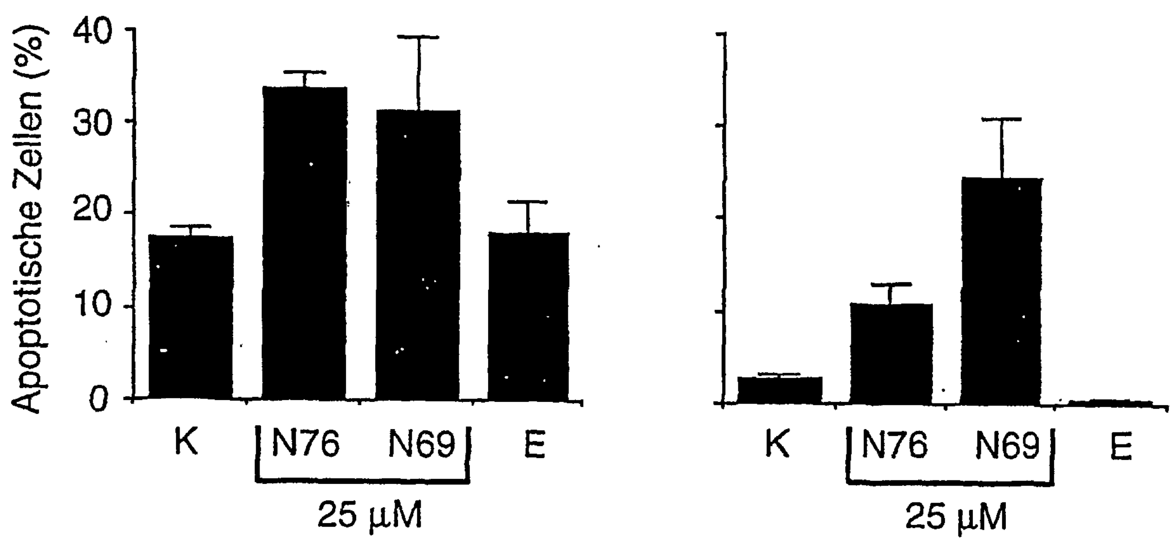


Fig.1

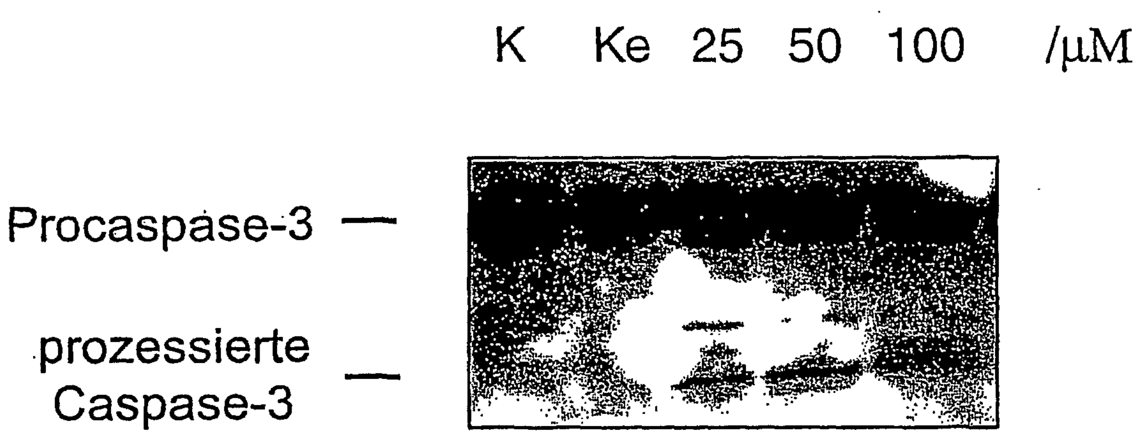


Fig.2

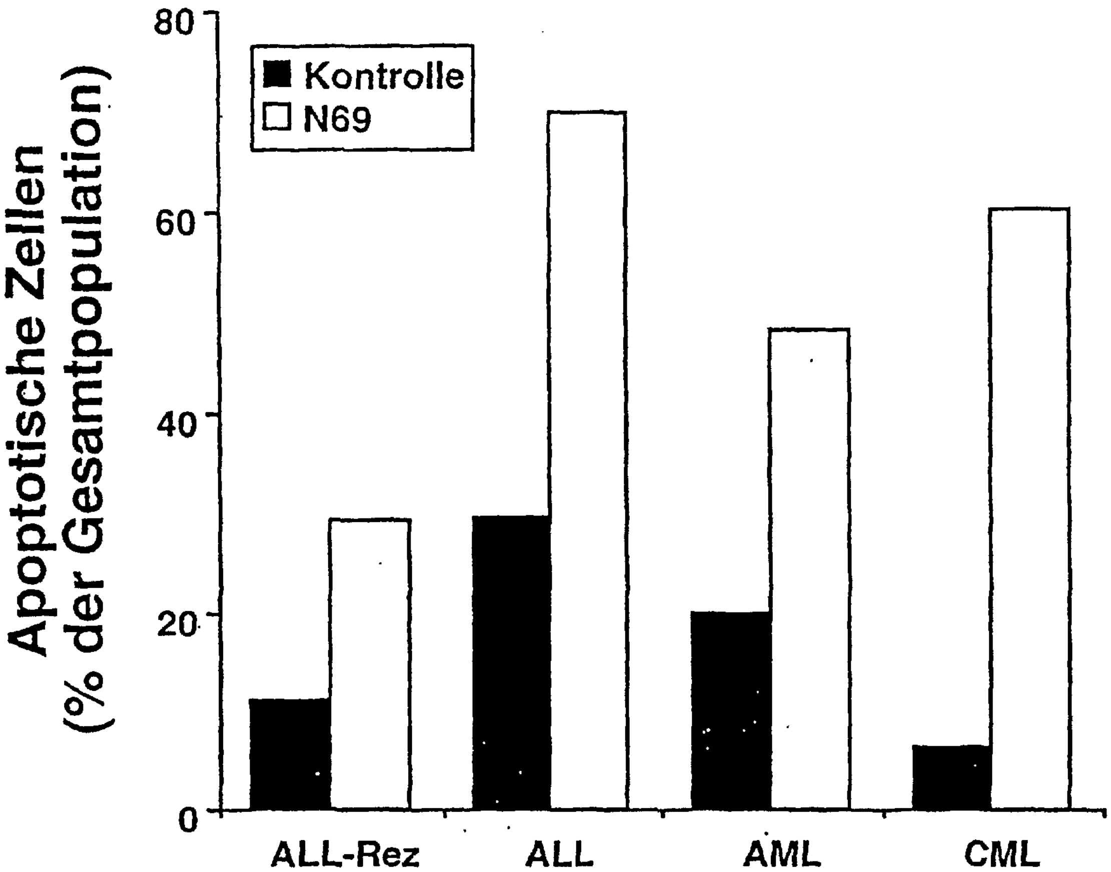
80
60
40
20
0
Apoptotische Zellen
(% der Gesamtpopulation)
Kontrolle
N69
ALL-Rez
ALL
AML
CML

Fig.3

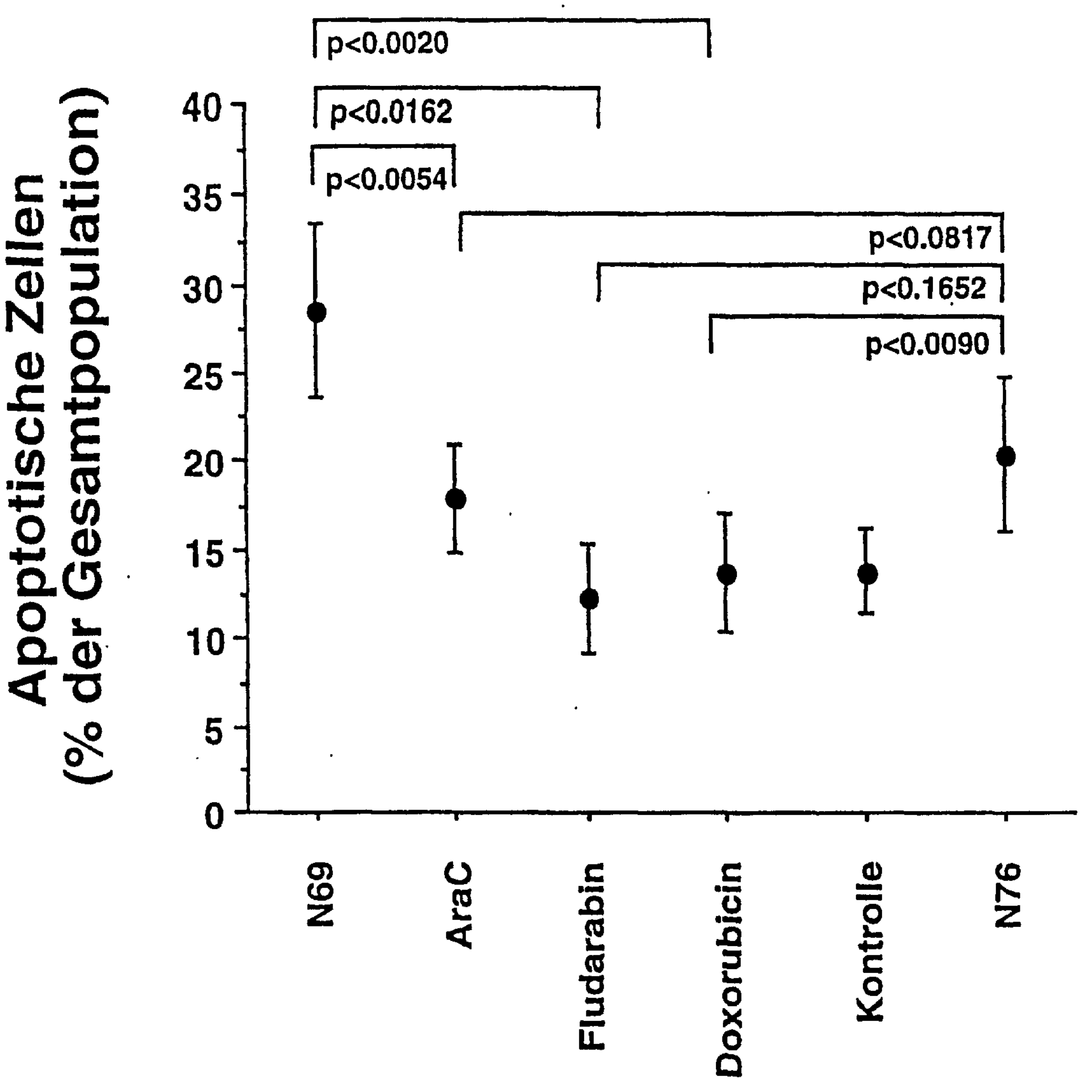

p<0.0020
p<0.0162
p<0.0054
p<0.0817
p<0.1652
p<0.0090
Apoptotische Zellen
(% der Gesamtpopulation)
40
35
30
25
20
15
10
5
0
N69
AraC
Fludarabin
Doxorubicin
Kontrolle
N76


Fig.4

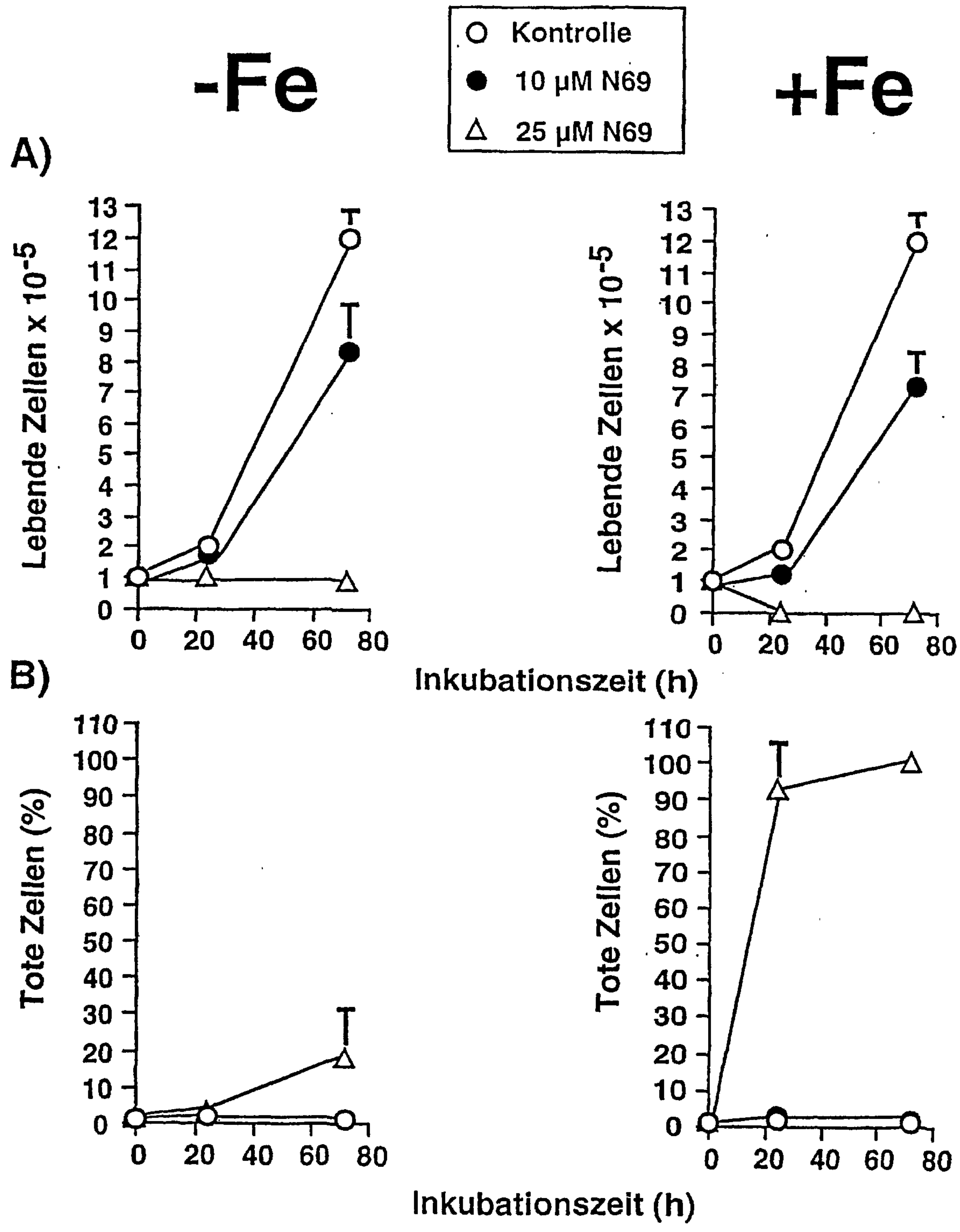
-Fe
+Fe
Kontrolle
10 µM N69
25 µM N69
A)
Lebende Zellen x 10-5
13
12
11
10
9
8
7
6
5
4
3
2
1
0
0
20
40
60
80
Inkubationszeit (h)
B)
Tote Zellen (%)
110
100
90
80
70
60
50
40
30
20
10
0
0
20
40
60
80
Inkubationszeit (h)

Fig.5

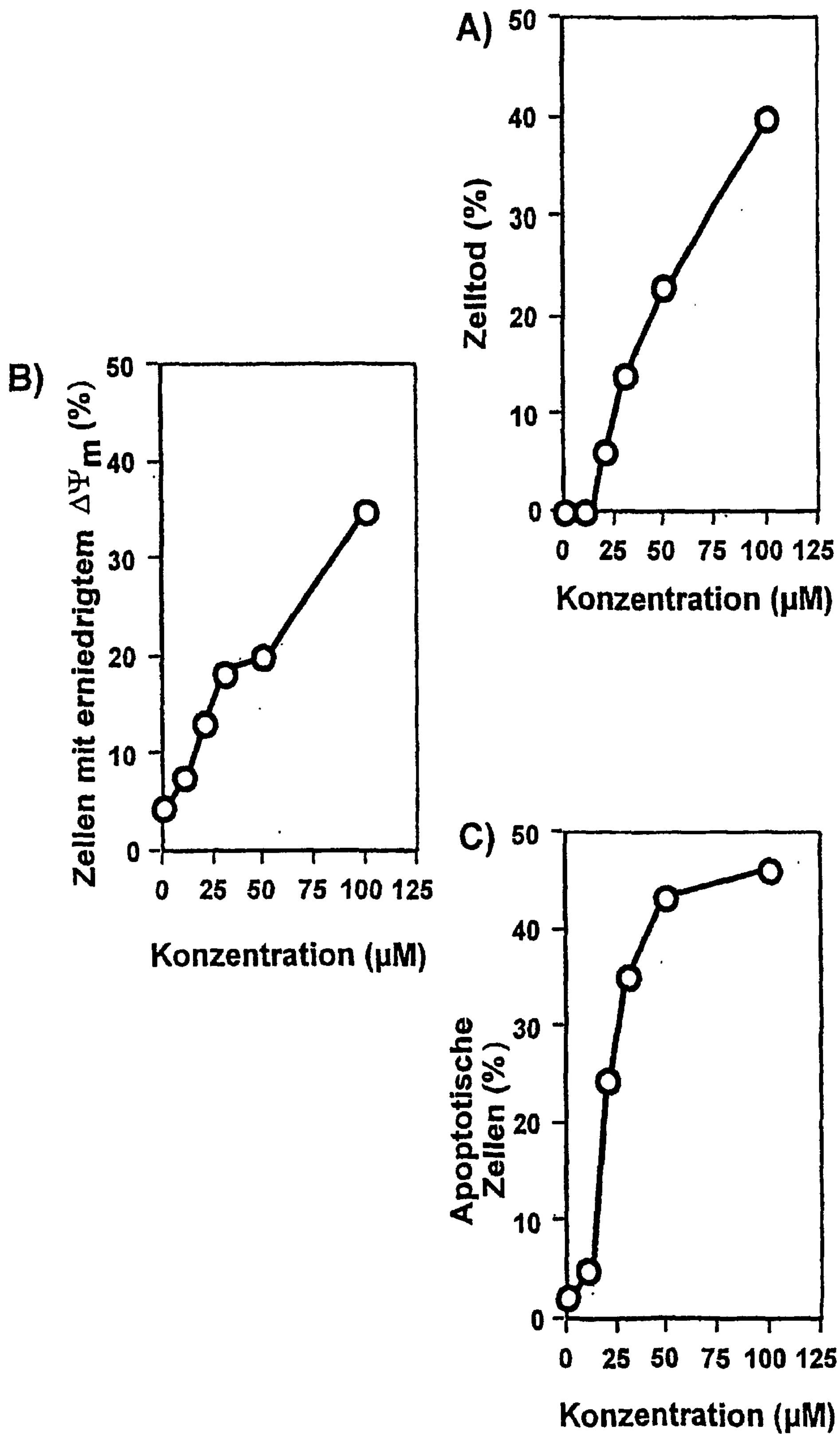
A)
Zelltod (%)
Konzentration (µM)
B)
Zellen mit erniedrigtem $\Delta\Psi_m$ (%)
Konzentration (µM)
C)
Apoptotische Zellen (%)
Konzentration (µM)

Fig.6